(19) Europäisches Patentamt / European Patent Office / Office européen des brevets

(11) EP 4 487 684 A1

(12) **EUROPEAN PATENT APPLICATION**
published in accordance with Art. 153(4) EPC

(43) Date of publication:
**08.01.2025 Bulletin 2025/02**

(21) Application number: **23763536.2**

(22) Date of filing: **02.03.2023**

(51) International Patent Classification (IPC):
***A01N 43/90*** *(2006.01)* ***A01P 5/00*** *(2006.01)*
***A01P 7/04*** *(2006.01)* ***C07D 471/04*** *(2006.01)*
***C07D 498/18*** *(2006.01)* ***C07D 498/22*** *(2006.01)*

(52) Cooperative Patent Classification (CPC):
**A01N 43/90; A01P 5/00; A01P 7/04; C07D 471/04; C07D 498/18; C07D 498/22**

(86) International application number:
**PCT/JP2023/007767**

(87) International publication number:
**WO 2023/167279 (07.09.2023 Gazette 2023/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.03.2022 JP 2022032312**
**25.08.2022 JP 2022133984**

(71) Applicant: **Sumitomo Chemical Company, Limited**
**Tokyo 103-6020 (JP)**

(72) Inventors:
- **IWATA, Chiemi**
  **Takarazuka-shi, Hyogo 665-8555 (JP)**
- **AZUMA, Shuhei**
  **Osaka-shi, Osaka 555-0021 (JP)**
- **OBATA, Hiroto**
  **Takarazuka-shi, Hyogo 665-8555 (JP)**

(74) Representative: **Vossius & Partner Patentanwälte Rechtsanwälte mbB**
**Siebertstrasse 3**
**81675 München (DE)**

# (54) METHOD FOR CONTROLLING HARMFUL ARTHROPODS OR HARMFUL NEMATODES USING ZOANTHAMINE ANALOG

(57) The present invention provides a method for controlling harmful arthropods or harmful nematodes. A compound represented by formula (I) [wherein $R^1$ represents a hydrogen atom, etc., $R^2$ represents $OR^{A2}$, $R^3$, $R^7$, $R^{14}$, $R^{17}$, $R^{19}$, and $R^{25}$ represent a hydrogen atom, $R^4$ represents a methyl group, etc., $R^5$ represents a methyl group, etc., $R^6$ represents a hydrogen atom, etc., $R^8$ represents a hydrogen atom, etc., $R^9$ represents a methyl group, etc., $R^{10}$ represents a hydrogen atom, etc., $R^{11}$ represents $OR^{A9}$, R12 represents a hydrogen atom, etc., $R^{13}$ represents a hydrogen atom, etc., $R^{15}$ represents a hydrogen atom, etc., $R^{16}$ represents a hydrogen atom, etc., $R^{18}$ represents a methyl group, etc., $R^{20}$ represents a hydrogen atom, etc., $R^{21}$ represents a methyl group, etc., $R^{22}$ represents a hydrogen atom, etc., $R^{23}$ represents a hydrogen atom, etc., $R^{24}$ represents a hydrogen atom, etc., $R^{26}$ represents a hydrogen atom, etc., $R^{27}$ represents a C1-C3 alkoxy group, etc., and $R^{A2}$ and $R^{A9}$ represent a hydrogen atom, etc.]
or an N-oxide thereof or a salt thereof can be used for controlling harmful arthropods or harmful nematodes.

## Description

TECHNICAL FIELD

This application claims priority to and the benefit of Japanese Patent Application Nos. 2022-032312 filed March 3, 2022, and 2022-133984 filed August 25, 2022, and the entire contents of which are incorporated herein by reference.

The present invention relates to a method for controlling harmful arthropods or harmful nematodes using zoanthamine analogues.

BACKGROUND ART

Hereto, some compounds have been studied so as to control harmful arthropods or harmful nematodes (for example, see Non-Patent Literature 1).

On the other hand, zoanthamines and salts thereof suppress an IL-6 production by an osteoblast, and also suppress a reduction in bone mass and bone strength associated with osteoporosis (see Non-Patent Literature 2).

CITATION LIST

NON-PATENT LITERATURE

Non-Patent Literature 1: "The Pesticide Manual 18th Edition", published by British Crop Protection Council, 2018, ISBN 978-1999896614

Non-Patent Literature 2: "Bulletin of the Chemical Society of Japan", 1998, vol.71, p.771-779

SUMMARY OF THE INVENTION

(PROBLEMS TO BE SOLVED BY INVENTION)

An object of the present invention is to provide a method for controlling harmful arthropods or harmful nematodes.

(MEANS TO SOLVE PROBLEMS)

The present invention encompasses the following aspects, but are not limited thereto.

[1] A method for controlling a harmful arthropod or a harmful nematode, which comprises applying an effective amount of a compound represented by formula (I):

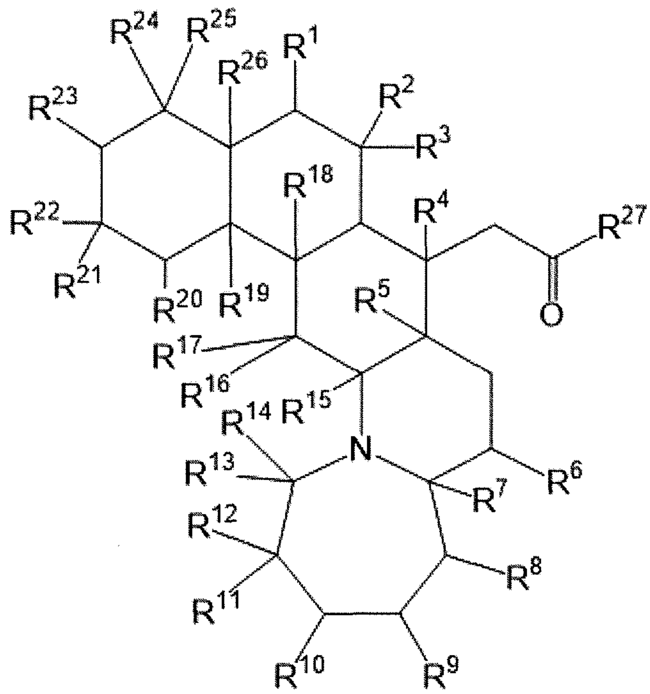

(I)

[wherein

$R^1$ represents a hydrogen atom, a C1-C3 alkyl group, or $CH_2OR^{A1}$,
$R^2$ represents $OR^{A2}$,
$R^3$ represents a hydrogen atom,
$R^4$ represents a methyl group or $CH_2OR^{A3}$,
$R^5$ represents a methyl group or $CH_2OR^{A4}$,
$R^6$ represents a hydrogen atom or $OR^{A5}$,
$R^7$ represents a hydrogen atom,
$R^8$ represents a hydrogen atom, a halogen atom, a methoxy group, or $OR^{A6}$,
$R^9$ represents a methyl group or $CH_2OR^{A7}$,
$R^{10}$ represents a hydrogen atom or $OR^{A8}$,
$R^{11}$ represents $OR^{A9}$,
$R^{12}$ represents a hydrogen atom or $OR^{A10}$,
$R^{13}$ represents a hydrogen atom, a dichloromethyl group, a hydroxy(methoxy)methyl group, a hydroxy(ethoxy) methyl group, or $OR^{A11}$,
$R^{14}$ represents a hydrogen atom,
$R^{15}$ represents a hydrogen atom or $OR^{A12}$,
$R^{16}$ represents a hydrogen atom, a halogen atom, or $OR^{A13}$,
$R^{17}$ represents a hydrogen atom,
$R^{18}$ represents a methyl group, or $CH_2OR^{A14}$,
$R^{19}$ represents a hydrogen atom,
$R^{20}$ represents a hydrogen atom or $OR^{A15}$,
$R^{21}$ represents a methyl group, $CH_2OR^{A16}$, or $CH_2NHCH_2C(O)OCH_3$,
$R^{22}$ represents a hydrogen atom or $OR^{A17}$,
$R^{23}$ represents a hydrogen atom or $OR^{A18}$,
$R^{24}$ represents a hydrogen atom, a C1-C3 alkoxy group, or $OR^{A19}$,
$R^{25}$ represents a hydrogen atom,
$R^{26}$ represents a hydrogen atom or $OR^{A20}$,
$R^{27}$ represents a C1-C3 alkoxy group or a hydroxy group, $R^{A1}$, $R^{A2}$, $R^{A3}$, $R^{A4}$, $R^{A5}$, $R^{A6}$, $R^{A7}$, $R^{A8}$, $R^{A9}$, $R^{A10}$, $R^{A11}$, $R^{A12}$, $R^{A13}$, $R^{A14}$, $R^{A15}$, $R^{A16}$, $R^{A17}$, $R^{A18}$, $R^{A19}$, and $R^{A20}$ are identical to or different from each other, and each represents a hydrogen atom, a (C1-C6 alkyl)carbonyl group, or a (C3-C6 cycloalkyl)carbonyl group,
$R^1$ and $R^{27}$ may combine with each other to form $\#^1$-$CH_2$-O-, and $\#^1$ represents a binding position of a carbon atom to which $R^1$ is attached,
$R^2$ and $R^3$ may combine with each other to form an oxo group,

$R^3$ and $R^{18}$ may combine with each other to form $\#^3$-O-$CHR^{B1}$-, and $\#^3$ represents a binding position of a carbon atom to which $R^3$ is attached,
$R^4$ and $R^{27}$ may combine with each other to form $\#^4$-$CH_2$-O-, and $\#^4$ represents a binding position of a carbon atom to which $R^4$ is attached,
$R^6$ and $R^{27}$ may combine with each other to form a bond,
$R^7$ and $R^{11}$ may combine with each other to form -O-,
$R^{13}$ and $R^{14}$ may combine with each other to form an oxo group,
$R^{15}$ and $R^{16}$ may combine together with carbon atoms to which they are attached to form a double bond,
$R^{15}$ and $R^{18}$ may combine with each other to form $\#^{15}$-O-$CHR^{B2}$-, and $\#^{15}$ represents a binding position of a carbon atom to which $R^{15}$ is attached,
$R^{15}$ and $R^{27}$ may combine with each other to form -O-,
$R^{16}$ and $R^{17}$ may combine with each other to form an oxo group,
$R^{18}$ and $R^{22}$ may combine with each other to form $\#^{18}$-$CH_2$-O-, and $\#^{18}$ represents a binding position of a carbon atom to which $R^{18}$ is attached,
$R^{18}$ and $R^{23}$ may combine with each other to form -$CH_2$-,
$R^{19}$, $R^{20}$, $R^{22}$, $R^{23}$, $R^{25}$, and $R^{26}$ may combine together with carbon atoms to which they are attached to form a benzene ring,
$R^{21}$ and $R^{22}$ may combine with each other to form =$CH_2$,
$R^{22}$ and $R^{23}$ may combine together with carbon atoms to which they are attached to form a double bond,
$R^{24}$ and $R^{25}$ may combine with each other to form an oxo group,
$R^{B1}$ and $R^{B2}$ are identical to or different from each other, and each represents a hydrogen atom, a C1-C3 alkoxy group, or a hydroxy group,
with the proviso that zoanthamine, norzoanthamine, zoanthaminone, and norzoanthaminone are excluded.]

or an N-oxide thereof or a salt thereof (hereinafter, the compound represented by formula (I), N-oxide thereof or salt thereof are referred to as "Present compound") to a harmful arthropod or a harmful nematode or a habitat for harmful arthropod or harmful nematode.

[2] The method described in [1] wherein in the formula (I), a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of a to g;

a: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{21}$ and $R^{22}$ may combine with each other to form =$CH_2$, $R^{23}$ represents a hydrogen atom, or $OR^{A18}$, $R^{18}$ and $R^{23}$ may combine with each other to form -$CH_2$-, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, and $R^{26}$ represents a hydrogen atom or $OR^{A20}$;

b: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ and $R^{27}$ combine with each other to form -O-, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, $CH_2OR^{A16}$, or $CH_2NHCH_2C(O)OCH_3$, and $R^{24}$ represents a C1-C3 alkoxy group, or $OR^{A19}$;

c: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^3$ and $R^{18}$ may combine with each other to form -O-$CHR^{B1}$- , $R^4$ and $R^{27}$ combine with each other to form $\#^4$-$CH_2$-O-, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a

halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, or $CH_2OR^{A14}$, $R^{15}$ and $R^{18}$ may combine with each other to form $\#^{15}$-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)\ OCH_3$, $R^{22}$ represents a hydrogen atom or $OR^{A17}$, $R^{18}$ and $R^{22}$ may combine with each other to form $\#^{18}$-$CH_2$-O-, $R^{23}$ represents a hydrogen atom, or $OR^{A18}$, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

d: a combination wherein $R^1$ and $R^{27}$ combine with each other to form $\#^1$-$CH_2$-O-, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom or $OR^{A20}$;

e: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)\ OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ represents a hydrogen atom, a C1-C3 alkoxy group, or $OR^{A19}$ , $R^{25}$ represents a hydrogen atom, $R^{24}$ and $R^{25}$ may combine with each other to form an oxo group, $R^{26}$ represents a hydrogen atom or $OR^{A20}$, and $R^{27}$ represents a C1-C3 alkoxy group or a hydroxy group;

f: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ and $R^{27}$ combine with each other to form a bond, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)\ OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, and $R^{26}$ represents a hydrogen atom or $OR^{A20}$; and

g: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ represents a hydrogen atom, $R^{11}$ represents $OR^{A9}$, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom or $OR^{A20}$, and $R^{27}$ represents a C1-C3 alkoxy group or a hydroxy group.

[3] The method described in [1] or [2] wherein in formula (I), $R^5$ represents a methyl group, and a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$ , $R^{22}$, $R^{23}$, $R^{24}$, $R^{25}$, $R^{26}$, and $R^{27}$ represents any combinations of $a^1$ to $g^1$:

$a^1$: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OR^{A16}$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or $OR^{A19}$, $R^{25}$ represents a hydrogen atom, $R^{24}$ and $R^{25}$ may combine with each other to form an oxo group, and $R^{26}$ represents a hydrogen atom;

$b^1$: a combination wherein $R^1$ represents a hydrogen atom or a methyl group, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{15}$ and $R^{27}$ combine with each other to form -O-, $R^{16}$ represents a hydrogen atom or a halogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a C1-C3 alkoxy group or $OR^{A19}$;

$c^1$: a combination wherein $R^1$ represents a methyl group, $R^2$ represents $OR^{A2}$, $R^3$ represents a hydrogen atom, $R^2$ and $R^3$ may combine with each other to form an oxo group, $R^3$ and $R^{18}$ may combine with each other to form $-O-CHR^{B1}-$, $R^4$ and $R^{27}$ combine with each other to form $\#^4-CH_2-O-$, $R^6$ represents a hydrogen atom or $OR^{A5}$, $R^7$ and $R^{11}$ combine with each other to form -O-, $R^{13}$ represents a hydrogen atom or $OR^{A11}$, $R^{14}$ represents a hydrogen atom, $R^{15}$ represents a hydrogen atom, $R^{16}$ represents a hydrogen atom, a halogen atom, or $OR^{A13}$ , $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form $\#^{15}-O-CHR^{B2}-$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)\ OCH_3$, $R^{22}$ represents a hydrogen atom, $R^{18}$ and $R^{22}$ may combine with each other to form $\#^{18}-CH_2-O-$, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

$d^1$: a combination wherein $R^1$ and $R^{27}$ combine with each other to form $\#^1-CH_2-O-$, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^1$: a combination wherein $R^1$ represents a hydrogen atom or a C1-C3 alkyl group, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, a dichloromethyl group, a hydroxy(methoxy)methyl group, or a hydroxy(ethoxy)methyl group, $R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, $R^{26}$ represents a hydrogen atom, and $R^{27}$ represents a C1-C3 alkoxy group or a hydroxy group;

$f^1$: a combination wherein $R^1$ represents a hydrogen atom or a C1-C3 alkyl group, $R^2$ and $R^3$ combine with each other to form an oxo group, $R^4$ represents a methyl group, $R^6$ and $R^{27}$ combine with each other to form a bond, $R^7$ and $R^{11}$ combine with each other to form -O-, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$ , $R^{25}$ represents a hydrogen atom, $R^{24}$ and $R^{25}$ may combine with each other to form an oxo group, and $R^{26}$

represents a hydrogen atom; and

$g^1$: a combination wherein $R^1$ represents a hydrogen atom or a C1-C3 alkyl group, $R^2$ and $R^3$ combine with each other to form an oxo group, $R^4$ represents a methyl group, $R^6$ represents a hydrogen atom, $R^7$ represents a hydrogen atom, $R^{11}$ represents $OR^{A9}$, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a C1-C3 alkoxy group or a hydroxy group.

[4] The method described in any one of [1] to [3] wherein $R^5$ represents a methyl group, $R^8$ represents a hydrogen atom, a halogen atom, a methoxy group, or a hydroxy group, $R^9$ represents a methyl group or $CH_2OH$, $R^{10}$ represents a hydrogen atom, an acetyloxy group, or a hydroxy group, and $R^{12}$ represents a hydrogen atom or a hydroxy group.
[5] The method described in [1] or [2] wherein in the formula (I), $R^{A2}$ represents a hydrogen atom, $R^{A5}$ represents a hydrogen atom, $R^{A8}$ represents a hydrogen atom or an acetyl group, $R^{A9}$ represents a hydrogen atom, $R^{A11}$ represents a hydrogen atom, $R^{A12}$ represents a hydrogen atom, $R^{A13}$ represents a hydrogen atom, $R^{A15}$ represents a hydrogen atom, $R^{A16}$ represents a hydrogen atom, $R^{A17}$ represents a hydrogen atom, $R^{A18}$ represents a hydrogen atom, $R^{A19}$ represents a hydrogen atom or an acetyl group, $R^{A20}$ represents a hydrogen atom, $R^{B1}$ represents a hydrogen atom or a methoxy group, $R^{B2}$ represents a hydrogen atom or a methoxy group, and
a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^2$ to $g^2$:

$a^2$: a combination wherein $R^1$ represents a hydrogen atom, a C1-C3 alkyl group, or $CH_2OH$, $R^2$ represents $OR^{A2}$, $R^3$ represents a hydrogen atom, $R^2$ and $R^3$ may combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group or $CH_2OH$, $R^{10}$ represents a hydrogen atom or $OR^{A8}$, $R^{12}$ represents a hydrogen atom or a hydroxy group, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OR^{A16}$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or a hydroxy group, $R^{25}$ represents a hydrogen atom, $R^{24}$ and $R^{25}$ may combine with each other to form an oxo group, and $R^{26}$ represents a hydrogen atom;

$b^2$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ and $R^{27}$ combine with each other to form - O-, $R^{16}$ represents a hydrogen atom or a halogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a C1-C3 alkoxy group or $OR^{A19}$;

$c^2$: a combination wherein $R^1$ represents a methyl group, $R^2$ represents $OR^{A2}$, $R^3$ represents a hydrogen atom, $R^2$ and $R^3$ may combine with each other to form an oxo group, $R^3$ and $R^{18}$ may combine with each other to form -O-$CHR^{B1}$-, $R^4$ and $R^{27}$ combine with each other to form #[4]-$CH_2$-O-, $R^5$ represents a methyl group, $R^6$ represents a hydrogen atom or $OR^{A5}$, $R^7$ and $R^{11}$ combine with each other to form -O-, $R^8$ represents a hydrogen atom, a halogen atom, a methoxy group, or a hydroxy group, $R^9$ represents a methyl group or $CH_2OH$, $R^{10}$ represents a hydrogen atom or a hydroxy group, $R^{12}$ represents a hydrogen atom, $R^{13}$ represents a hydrogen atom or $OR^{A11}$, $R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form #[15]-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom, $R^{18}$ and $R^{22}$ may combine with each other to form #[18]-$CH_2$-O-, $R^{23}$ represents a hydrogen atom, $R^{22}$ and $R^{23}$ may combine together with carbon atoms to which

they are attached to form a double bond, $R^{24}$ and $R^{25}$ combine with each other to form an oxo group, and $R^{26}$ represents a hydrogen atom or $OR^{A20}$;

$d^2$: a combination wherein $R^1$ and $R^{27}$ combine with each other to form $\#^1$-$CH_2$-O-, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom or a hydroxy group, $R^{12}$ represents a hydrogen atom, $R^{13}$ represents a hydrogen atom, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, a dichloromethyl group, a hydroxy(methoxy)methyl group, or a hydroxy(ethoxy)methyl group, $R^{14}$ represents a hydrogen atom, $R^{15}$ represents a hydrogen atom, $R^{16}$ represents a hydrogen atom or a halogen atom, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents a hydroxy group, $R^{25}$ represents a hydrogen atom, $R^{24}$ and $R^{25}$ may combine with each other to form an oxo group, $R^{26}$ represents a hydrogen atom, and $R^{27}$ represents a C1-C3 alkoxy group or a hydroxy group;

$f^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ and $R^{27}$ combine with each other to form a bond, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom or a halogen atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, and $R^{26}$ represents a hydrogen atom; and

$g^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ represents a hydrogen atom, $R^7$ represents a hydrogen atom, $R^8$ represents a hydrogen atom, $R^9$ represents a methyl group, $R^{10}$ represents a hydrogen atom, $R^{11}$ represents $OR^{A9}$, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a C1-C3 alkoxy group or a hydroxy group.

[6] A composition for controlling a harmful arthropod or a harmful nematode which comprises the compound represented by formula (I) described in any one of [1] to [5] or an N-oxide thereof or a salt thereof.

[7] The composition described in [6] which further comprises one or more ingredients selected from the group consisting of the following Group (a), Group (b), Group (c), Group (d), and Group (e):

Group (a): a group consisting of insecticidal active ingredients, miticidal active ingredients and nematicidal active ingredients;

Group (b): fungicidal active ingredients;

Group (c): plant growth regulatory ingredients;

Group (d): repellent ingredients;

Group (e): biological control materials.

[8] A method for controlling a harmful arthropod or a harmful nematode which comprises applying an effective amount

of the composition described in [7] to a harmful arthropod or a harmful nematode or a habitat for the harmful arthropod or the harmful nematode.

[9] A seed or vegetative reproductive organ carrying an effective amount of the compound described in any one of [1] to [5] or an N-oxide thereof or a salt thereof or an effective amount of the composition described in [7].

[10] A composition for controlling a harmful arthropod or a harmful nematode which comprises the compound represented by formula (I) described in any one of [1] to [5] or an N-oxide thereof or a salt thereof, and an inert carrier.

[11] A compound represented by formula (II):

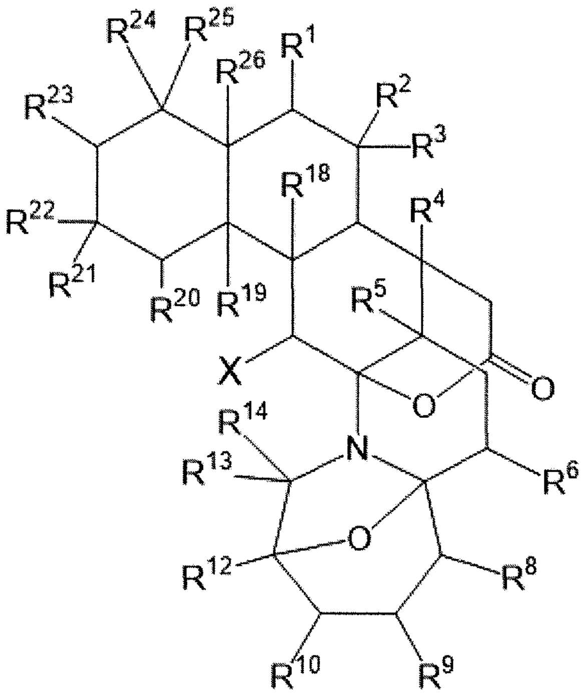

( II )

[wherein

$R^1$ represents a hydrogen atom, a C1-C3 alkyl group, or $CH_2OR^{A1}$,
$R^2$ represents $OR^{A2}$,
$R^3$ represents a hydrogen atom,
$R^4$ represents a methyl group or $CH_2OR^{A3}$,
$R^5$ represents a methyl group or $CH_2OR^{A4}$,
$R^6$ represents a hydrogen atom or $OR^{A5}$,
$R^8$ represents a hydrogen atom, a halogen atom, a methoxy group, or $OR^{A6}$,
$R^9$ represents a methyl group or $CH_2OR^{A7}$,
$R^{10}$ represents a hydrogen atom or $OR^{A8}$,
$R^{12}$ represents a hydrogen atom or $OR^{A10}$,
$R^{13}$ represents a hydrogen atom,
$R^{14}$ represents a hydrogen atom,
X represents a halogen atom,
$R^{18}$ represents a methyl group, or $CH_2OR^{A14}$,
$R^{19}$ represents a hydrogen atom,
$R^{20}$ represents a hydrogen atom or $OR^{A15}$,
$R^{21}$ represents a methyl group, $CH_2OR^{A16}$, or $CH_2NHCH_2C(O)OCH_3$,
$R^{22}$ represents a hydrogen atom or $OR^{A17}$,
$R^{23}$ represents a hydrogen atom or $OR^{A18}$,
$R^{24}$ represents a hydrogen atom, a C1-C3 alkoxy group, or $OR^{A19}$,
$R^{25}$ represents a hydrogen atom,
$R^{26}$ represents a hydrogen atom or $OR^{A20}$,
$R^{A1}$, $R^{A2}$, $R^{A3}$, $R^{A4}$, $R^{A5}$, $R^{A6}$, $R^{A7}$, $R^{A8}$, $R^{A10}$, $R^{A14}$, $R^{A15}$, $R^{A16}$, $R^{A17}$, $R^{A18}$, $R^{A19}$, and $R^{A20}$ are identical to or different from each other and each represents a hydrogen atom, a (C1-C6 alkyl)carbonyl group, or a (C3-C6 cycloalkyl)carbonyl group,

$R^2$ and $R^3$ may combine with each other to form an oxo group,
$R^3$ and $R^{18}$ may combine with each other to form $\#^3$ -O-$CHR^{B1}$-, $\#^3$ represents a binding position of a carbon atom to which $R^3$ is attached,
$R^{13}$ and $R^{14}$ may combine with each other to form an oxo group,
$R^{18}$ and $R^{22}$ may combine with each other to form $\#^{18}$ -$CH_2$-O-, $\#^{18}$ represents a binding position of a carbon atom to which $R^{18}$ is attached,
$R^{18}$ and $R^{23}$ may combine with each other to form -$CH_2$-,
$R^{19}$, $R^{20}$, $R^{22}$, $R^{23}$, $R^{25}$, and $R^{26}$ may combine together with carbon atoms to which they are attached to form a benzene ring,
$R^{21}$ and $R^{22}$ may combine with each other to form =$CH_2$,
$R^{22}$ and $R^{23}$ may combine together with carbon atoms to which they are attached to form a double bond,
$R^{24}$ and $R^{25}$ may combine with each other to form an oxo group, and
$R^{B1}$ represents a hydrogen atom, or a C1-C3 alkoxy group],

or an N-oxide thereof or a salt thereof (hereinafter, the compound represented by formula (II), or N-oxide thereof or salt thereof is referred to as "Compound A of the present invention").

[12] The compound described in [11] wherein in the formula (II), wherein
$R^2$ and $R^3$ combine with each other to form an oxo group, $R^4$ represents a methyl group, $R^5$ represents a methyl group, $R^6$ represents a hydrogen atom, $R^8$ represents a hydrogen atom, $R^9$ represents a methyl group, $R^{10}$ represents a hydrogen atom, $R^{12}$ represents a hydrogen atom, $R^{18}$ represents a methyl group, $R^{20}$ represents a hydrogen atom, $R^{21}$ represents a methyl group, $R^{22}$ represents a hydrogen atom or $OR^{A17}$, $R^{21}$ and $R^{22}$ may combine with each other to form =$CH_2$, $R^{23}$ represents a hydrogen atom or $OR^{A18}$, $R^{18}$ and $R^{23}$ may combine with each other to form -$CH_2$-, $R^{22}$ and $R^{23}$ may combine together with carbon atoms to which they are attached to form a double bond, $R^{24}$ represents a hydrogen atom or $OR^{A19}$, $R^{25}$ represents a hydrogen atom, $R^{24}$ and $R^{25}$ may combine with each other to form an oxo group, and $R^{26}$ represents a hydrogen atom.

[13]A compound represented by formula (AA1), formula (AA2), formula (AA3), formula (BB1), formula (EE1), formula (EE3), formula (EE4), formula (EE5), formula (EE6), formula (EE7), formula (GG1), formula (GG2), or formula (GG3), or an N-oxide thereof or a salt thereof, or a compound represented by formula (EE2) (hereinafter, the compounds described in [13], or an N-oxide thereof or a salt thereof are referred to as "Compound B of the present invention").

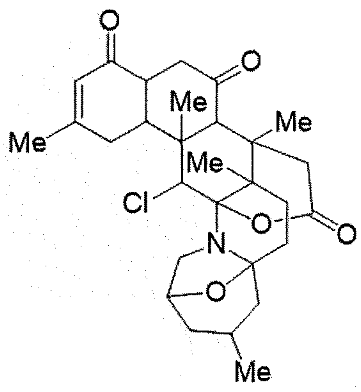
(AA1)

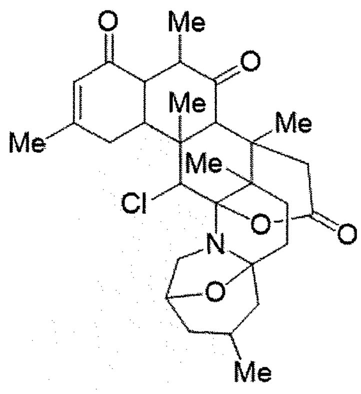
(AA2)

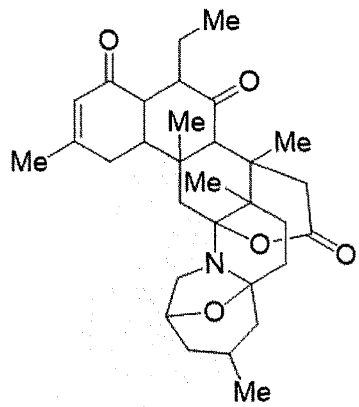
(AA3)

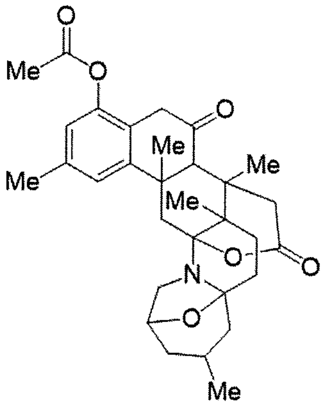
(BB1)

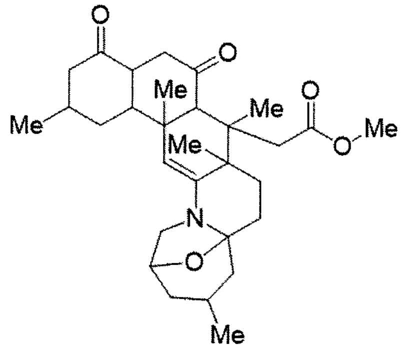
(EE1)

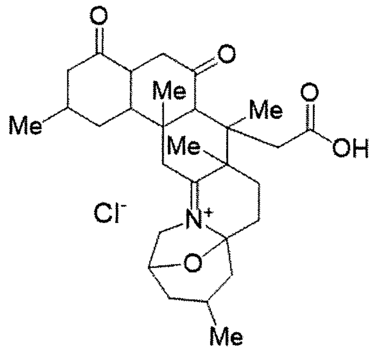
(EE2)

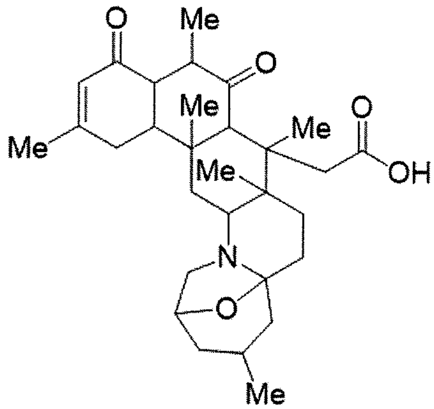
(EE3)

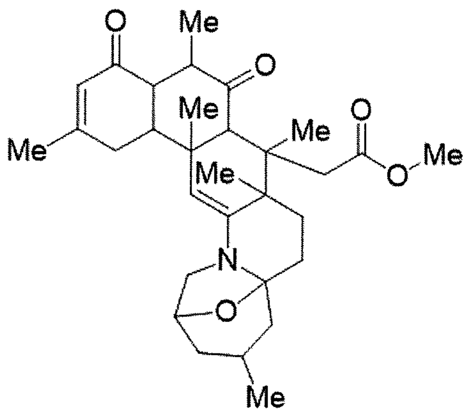
(EE4)

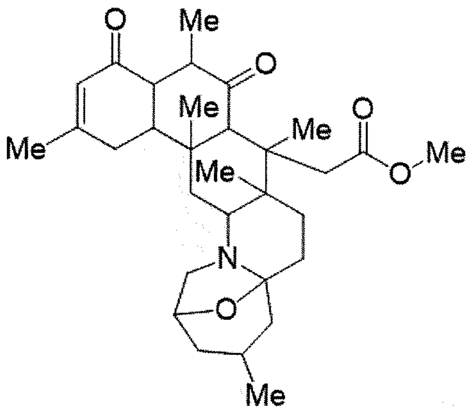
(EE5)

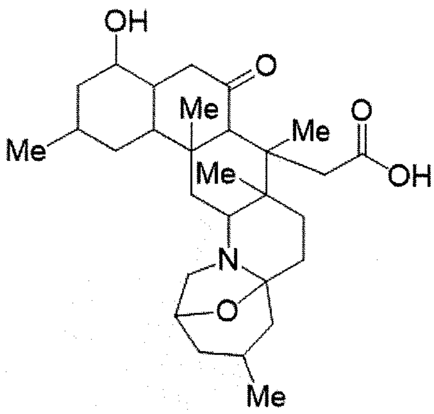
(EE6)

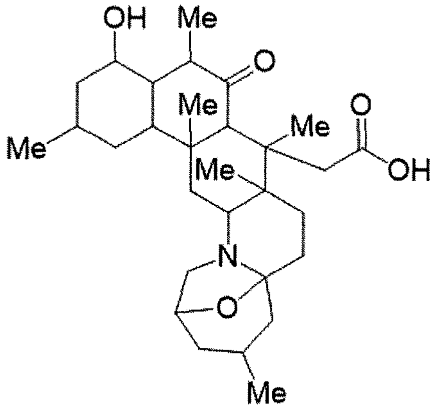

(EE7)

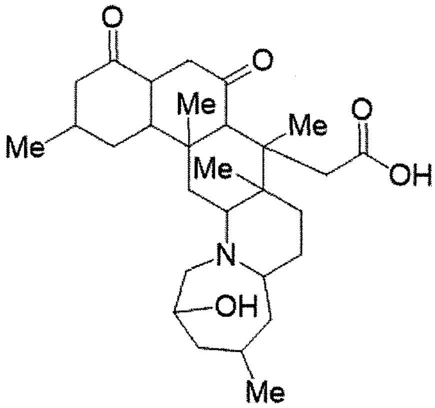

(GG1)

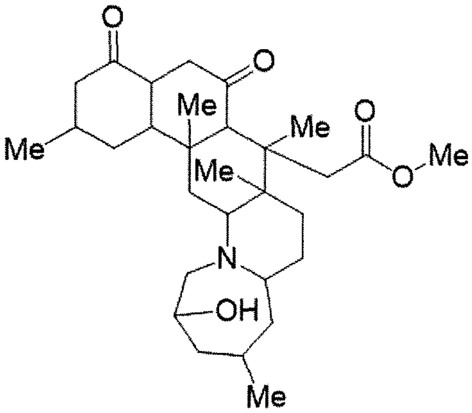

(GG2)

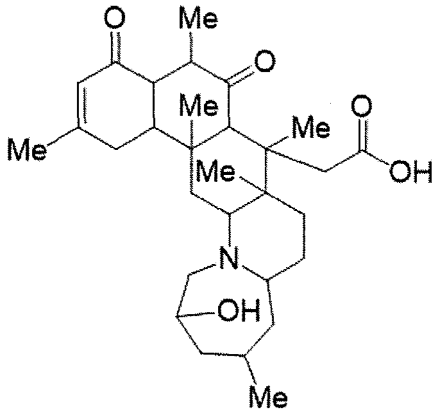

(GG3)

[14] The compound described in [13] which is represented by formula (AA1), formula (AA2), formula (AA3), formula (BB1), formula (EE1), formula (EE3), formula (EE4), formula (EE5), formula (EE6), formula (GG1), or formula (GG3), or an N-oxide thereof or a salt thereof, or a compound represented by formula (EE2).
[15] A composition for controlling a harmful arthropod or a harmful nematode which comprises the compound described in any one of [11] to [14], or an N-oxide thereof or a salt thereof.
[16] The composition described in [15] which further comprises one or more ingredients selected from the group consisting of the following Group (a), Group (b), Group (c), Group (d), and Group (e):

Group (a): a group consisting of insecticidal active ingredients, miticidal active ingredients and nematicidal active ingredients;
Group (b): fungicidal active ingredients;
Group (c): plant growth regulatory ingredients;
Group (d): repellent ingredients;
Group (e): biological control materials.

[17] A method for controlling a harmful arthropod or a harmful nematode which comprises applying an effective amount of the composition described in [16] to a harmful arthropod or a harmful nematode or a habitat for the harmful arthropod or the harmful nematode.
[18] A seed or vegetative reproductive organ carrying an effective amount of the compound described in any one of [11] to [14] or an N-oxide thereof or a salt thereof or an effective amount of the composition described in [16].
[19] A composition for controlling a harmful arthropod or a harmful nematode which comprises the compound described in any one of [11] to [14], or the N-oxide thereof or the salt thereof, and an inert carrier.

EFFECT OF INVENTION

According to the present invention, harmful arthropods or harmful nematodes can be controlled.

MODE FOR CARRYING OUT THE INVENTION

The substituent(s) as described herein is/are explained.

The term "halogen atom" represents fluorine atom, chlorine atom, bromine atom, or iodine atom.

As used herein, Me represents a methyl group, Et represents an ethyl group, and Ac represents an acetyl group.

Hydroxy(methoxy)methyl group represents $CH(OH)(OCH_3)$ group.

"Compound A of the present invention" and "Compound B of the present invention" are collectively referred to as "Compound of the present invention".

Zoanthamine, norzoanthamine, zoanthaminone, and norzoanthaminone are compounds disclosed in Heterocyclic Communications, 1995, 1(2-3), 207-214.

Zoanthamine is a compound represented by formula (X):

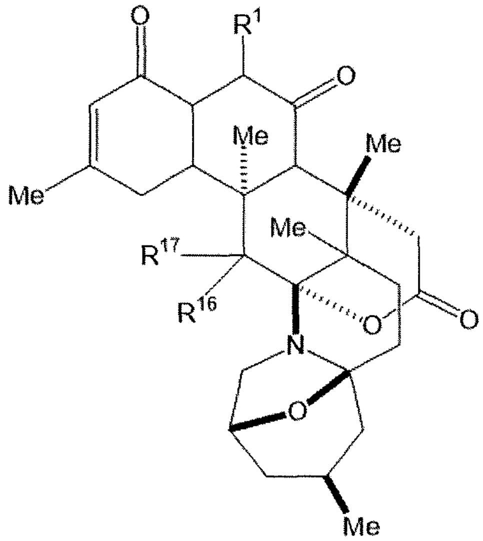

(X)

wherein $R^1$ represents a methyl group, and each of $R^{16}$ and $R^{17}$ represents a hydrogen atom.

Norzoanthamine is a compound represented by formula (X) wherein each of $R^1$, $R^{16}$ and $R^{17}$ represents a hydrogen atom.

Zoanthaminone is a compound represented by formula (X) wherein $R^1$ represents a methyl group, and $R^{16}$ and $R^{17}$ combine with each other to form an oxo group.

Norzoanthaminone is a compound represented by formula (X) wherein $R^1$ represents a hydrogen atom, and $R^{16}$ and $R^{17}$ combine with each other to form an oxo group.

The compound represented by formula (X) is the compound represented by formula (I) wherein $R^2$ and $R^3$ combine with each other to form an oxo group, each of $R^4$ and $R^5$ represents a methyl group, $R^6$ represents a hydrogen atom, $R^7$ and $R^{11}$ combine with each other to form -O-, $R^8$ represents a hydrogen atom, $R^9$ represents a methyl group, each of $R^{10}$, $R^{12}$, $R^{13}$, and $R^{14}$ represents a hydrogen atom, $R^{15}$ and $R^{27}$ combine with each other to form -O-, $R^{18}$ represents a methyl group, each of $R^{19}$ and $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom.

The present compound and the compound of the present invention may contain one or more of stereoisomers. Examples of the stereoisomers include enantiomer, diastereomer, geometric isomer, and the like. In the present compound, and the compound of the present invention, each of the stereoisomer and a mixture of the stereoisomers at an arbitrary ratio thereof are encompassed.

The salt of the compound represented by formula (I) or the N-oxide thereof represents an acid addition salt. Examples of the acid which forms the acid addition salt include inorganic acids such as hydrochloric acid, phosphoric acid, and sulfuric acid; and organic acids such as acetic acid, trifluoroacetic acid, benzoic acid, and p-toluene sulfonic acid. Examples of the salts of the compound represented by formula (I) include the compounds represented by the following formulae.

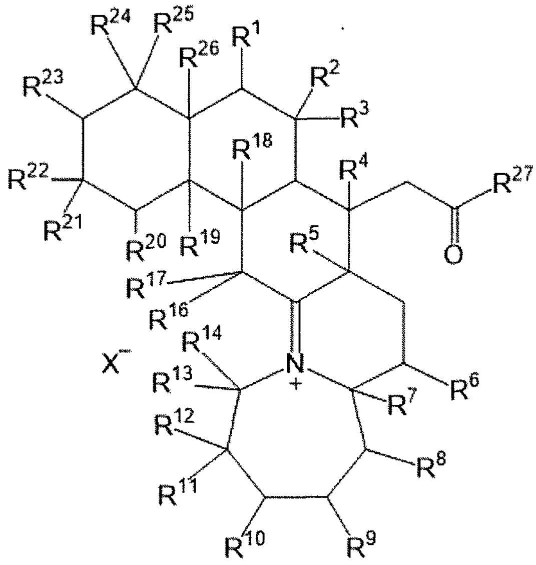 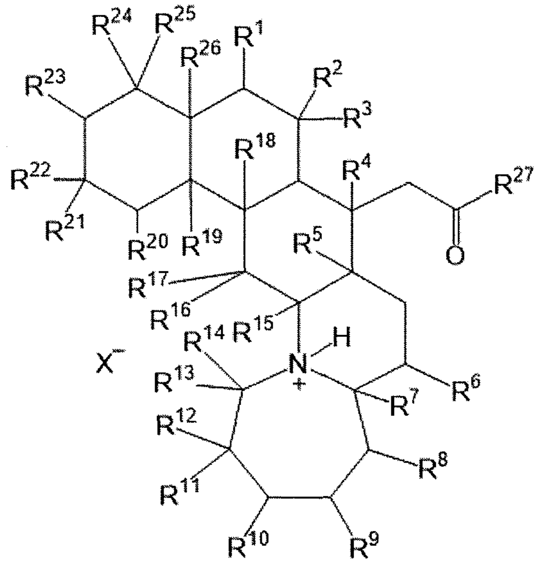

[wherein X- represents an anion which can be formed by losing a proton from an acid, and the other symbols are the same as defined above.]

Embodiments of the present compound include the compounds described below.

[Embodiment 1]

A present compound wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of a to g.

a: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, and $R^{26}$ represents a hydrogen atom or $OR^{A20}$;

b: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ and $R^{27}$ combine with each other to form -O-, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, $CH_2OR^{A16}$, or $CH_2NHCH_2C(O)OCH_3$, and $R^{24}$ represents a C1-C3 alkoxy group, or $OR^{A19}$;

c: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^3$ and $R^{18}$ may combine with each other to form $\#^3$ -O-CHR$^{B1}$- , $R^4$ and $R^{27}$ combine with each other to form $\#^4$ $-CH_2-O-$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group,

$R^{18}$ represents a methyl group, or $CH_2OR^{A14}$, $R^{15}$ and $R^{18}$ may combine with each other to form $\#^{15}$-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)\ OCH_3$, $R^{22}$ represents a hydrogen atom or $OR^{A17}$, $R^{18}$ and $R^{22}$ may combine with each other to form $\#^{18}$-$CH_2$-O-, $R^{23}$ represents a hydrogen atom, or $OR^{A18}$, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

d: a combination wherein $R^1$ and $R^{27}$ combine with each other to form $\#^1$-$CH_2$-O-, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom or $OR^{A20}$;

e: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, $R^{26}$ represents a hydrogen atom or $OR^{A20}$, and $R^{27}$ represents a C1-C3 alkoxy group or a hydroxy group;

f: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ and $R^{27}$ combine with each other to form a bond, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, and $R^{26}$ represents a hydrogen atom or $OR^{A20}$; and

g: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ represents a hydrogen atom, $R^{11}$ represents $OR^{A9}$, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom or $OR^{A20}$, and $R^{27}$ represents a C1-C3 alkoxy group or a hydroxy group.

[Embodiment 2] The compound in the embodiment 1 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination a, b, c, d, e or g.
[Embodiment 3] The compound in the embodiment 1 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination a, b, e or g.
[Embodiment 4] The compound in the embodiment 1 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination a, b or e.

[Embodiment 5] The compound in the embodiment 1 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination a.

[Embodiment 6] The present compound wherein a combination of $R^1$, $R^2$, $R^3$, $R^4$, $R^6$ $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^1$ to $g^1$.

$a^1$: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OR^{A16}$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or $OR^{A19}$, $R^{25}$ represents a hydrogen atom, $R^{24}$ and $R^{25}$ may combine with each other to form an oxo group, and $R^{26}$ represents a hydrogen atom;

$b^1$: a combination wherein $R^1$ represents a hydrogen atom or a methyl group, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{15}$ and $R^{27}$ combine with each other to form -O-, $R^{16}$ represents a hydrogen atom or a halogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a C1-C3 alkoxy group or $OR^{A19}$;

$c^1$: a combination wherein $R^1$ represents a methyl group, $R^2$ represents $OR^{A2}$, $R^3$ represents a hydrogen atom, $R^2$ and $R^3$ may combine with each other to form an oxo group, $R^3$ and $R^{18}$ may combine with each other to form $\#^3$-O-$CHR^{B1}$-, $R^4$ and $R^{27}$ combine with each other to form $\#^4$-$CH_2$-O-, $R^6$ represents a hydrogen atom or $OR^{A5}$, $R^7$ and $R^{11}$ combine with each other to form -O-, $R^{13}$ represents a hydrogen atom or $OR^{A11}$, $R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form $\#^{15}$-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom, $R^{18}$ and $R^{22}$ may combine with each other to form $\#^{18}$-$CH_2$-O-, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

$d^1$: a combination wherein $R^1$ and $R^{27}$ combine with each other to form $\#^1$-$CH_2$-O-, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^1$: a combination wherein $R^1$ represents a hydrogen atom or a C1-C3 alkyl group, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, a dichloromethyl group, a hydroxy(methoxy)methyl group, or a hydroxy(ethoxy)methyl group, $R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, $R^{26}$ represents a hydrogen atom, and $R^{27}$ represents a C1-C3 alkoxy group or a hydroxy group;

$f^1$: a combination wherein $R^1$ represents a hydrogen atom or a C1-C3 alkyl group, $R^2$ and $R^3$ combine with each other to form an oxo group, $R^4$ represents a methyl group, $R^6$ and $R^{27}$ combine with each other to form a bond, $R^7$ and $R^{11}$ combine with each other to form -O-, $R^{13}$ represents a hydrogen atom, $R^{14}$ represents a hydrogen atom,

$R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, and $R^{26}$ represents a hydrogen atom; and

$g^1$: a combination wherein $R^1$ represents a hydrogen atom or a C1-C3 alkyl group, $R^2$ and $R^3$ combine with each other to form an oxo group, $R^4$ represents a methyl group, $R^6$ represents a hydrogen atom, $R^7$ represents a hydrogen atom, $R^{11}$ represents $OR^{A9}$, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a C1-C3 alkoxy group or a hydroxy group.

[Embodiment 7] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^1$, $b^1$, $c^1$, $d^1$, $e^1$ or $g^1$.
[Embodiment 8] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^1$, $b^1$, $e^1$ or $g^1$.
[Embodiment 9] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^1$, $b^1$ or $e^1$.
[Embodiment 10] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^1$.
[Embodiment 11] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $b^1$.
[Embodiment 12] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $e^1$.
[Embodiment 13] The compound described in the embodiment 6 wherein a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $g^1$.
[Embodiment 14] The compound described in any one of the embodiments 1 to 13 or the present compound wherein $R^5$ represents a methyl group.
[Embodiment 15] The compound described in any one of the embodiments 1 to 13 or the present compound wherein $R^5$ represents a methyl group, $R^8$ represents a hydrogen atom, a halogen atom, a methoxy group, or a hydroxy group, $R^9$ represents a methyl group or $CH_2OH$, $R^{10}$ represents a hydrogen atom, an acetyloxy group, or a hydroxy group, and $R^{12}$ represents a hydrogen atom or a hydroxy group.
[Embodiment 16] The present compound wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^2$ to $g^2$:

$a^2$: a combination wherein $R^1$ represents a hydrogen atom, a C1-C3 alkyl group, or $CH_2OH$, $R^2$ represents $OR^{A2}$, $R^3$ represents a hydrogen atom, $R^2$ and $R^3$ may combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group or $CH_2OH$, $R^{10}$ represents a hydrogen atom or $OR^{A8}$, $R^{12}$ represents a hydrogen atom or a hydroxy group, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OR^{A16}$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or a hydroxy group, $R^{25}$ represents a hydrogen atom, $R^{24}$ and $R^{25}$ may combine with each other to form an oxo group, and $R^{26}$ represents a hydrogen atom;

$b^2$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$

represents a hydrogen atom, $R^{15}$ and $R^{27}$ combine with each other to form-O-, $R^{16}$ represents a hydrogen atom or a halogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a C1-C3 alkoxy group or $OR^{A19}$;

$c^2$: a combination wherein $R^1$ represents a methyl group, $R^2$ represents $OR^{A2}$, $R^3$ represents a hydrogen atom, $R^2$ and $R^3$ may combine with each other to form an oxo group, $R^3$ and $R^{18}$ may combine with each other to form $\#^3$-O-CHR$^{B1}$-, $R^4$ and $R^{27}$ combine with each other to form $\#^4$-$CH_2$-O-, $R^5$ represents a methyl group, $R^6$ represents a hydrogen atom or $OR^{A5}$, $R^7$ and $R^{11}$ combine with each other to form -O-, $R^8$ represents a hydrogen atom, a halogen atom, a methoxy group, or a hydroxy group, $R^9$ represents a methyl group or $CH_2OH$, $R^{10}$ represents a hydrogen atom or a hydroxy group, $R^{12}$ represents a hydrogen atom, $R^{13}$ represents a hydrogen atom or $OR^{A11}$, $R^{14}$ represents a hydrogen atom, $R^{15}$ represents a hydrogen atom, $R^{16}$ represents a hydrogen atom, a halogen atom, or $OR^{A13}$ , $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form $\#^{15}$-O-CHR$^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom, $R^{18}$ and $R^{22}$ may combine with each other to form $\#^{18}$ -$CH_2$-O-, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

$d^2$: a combination wherein $R^1$ and $R^{27}$ combine with each other to form $\#^1$-$CH_2$-O-, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom or a hydroxy group, $R^{12}$ represents a hydrogen atom, $R^{13}$ represents a hydrogen atom, $R^{14}$ represents a hydrogen atom, $R^{15}$ represents a hydrogen atom or $OR^{A12}$, $R^{16}$ represents a hydrogen atom, a halogen atom, or $OR^{A13}$ , $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, a dichloromethyl group, a hydroxy(methoxy)methyl group, or a hydroxy(ethoxy)methyl group, $R^{14}$ represents a hydrogen atom, $R^{15}$ represents a hydrogen atom, $R^{16}$ represents a hydrogen atom or a halogen atom, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents a hydroxy group, $R^{25}$ represents a hydrogen atom, $R^{24}$ and $R^{25}$ may combine with each other to form an oxo group, $R^{26}$ represents a hydrogen atom, and $R^{27}$ represents a C1-C3 alkoxy group or a hydroxy group;

$f^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ and $R^{27}$ combine with each other to form a bond, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom or a halogen atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$ , $R^{25}$ represents a hydrogen atom, $R^{24}$ and $R^{25}$ may combine with each other to form an oxo group, and $R^{26}$ represents a hydrogen atom; and

$g^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ represents a hydrogen atom, $R^7$ represents a hydrogen atom, $R^8$ represents a hydrogen atom, $R^9$ represents a methyl group, $R^{10}$ represents a hydrogen atom, $R^{11}$ represents $OR^{A9}$, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$ , $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a

hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a C1-C3 alkoxy group or a hydroxy group.

[Embodiment 17] The compound described in the embodiment 16 wherein $R^{A2}$ represents a hydrogen atom, $R^{A5}$ represents a hydrogen atom, $R^{A8}$ represents a hydrogen atom or an acetyl group, $R^{A9}$ represents a hydrogen atom, $R^{A11}$ represents a hydrogen atom, $R^{A12}$ represents a hydrogen atom, $R^{A13}$ represents a hydrogen atom, $R^{A15}$ represents a hydrogen atom, $R^{A16}$ represents a hydrogen atom, $R^{A17}$ represents a hydrogen atom, $R^{A18}$ represents a hydrogen atom, $R^{A19}$ represents a hydrogen atom or an acetyl group, $R^{A20}$ represents a hydrogen atom, $R^{B1}$ represents a hydrogen atom or a methoxy group, and $R^{B2}$ represents a hydrogen atom or a methoxy group.

[Embodiment 18] The compound described in the embodiment 16 or 17 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^2$, $b^2$, $c^2$, $d^2$, $e^2$ or $g^2$.

[Embodiment 19] The compound described in the embodiment 16 or 17 wherein a combination of $R^1$, $R^2$, $R^3$, $R^4$, $R^5$, $R^6$, $R^7$, $R^8$, $R^9$ $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^2$, $b^2$, $e^2$ or $g^2$.

[Embodiment 20] The compound described in the embodiment 16 or 17 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^2$, $b^2$ or $e^2$.

[Embodiment 21] The compound described in the embodiment 16 or 17 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^2$.

[Embodiment 22] The compound described in the embodiment 16 or 17 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{22}$, $R^{24}$, $R^{25}$, $R^{26}$, and $R^{27}$ represents the combination of $b^2$.

[Embodiment 23] The compound described in the embodiment 16 or 17 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $e^2$.

[Embodiment 24] The compound described in the embodiment 16 or 17 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $g^2$.

[Embodiment 25] The present compound wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^3$ to $g^3$.

$a^3$: a combination wherein $R^1$ represents a hydrogen atom, a methyl group, an ethyl group or $CH_2OH$, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group or $CH_2OH$, $R^{10}$ represents a hydrogen atom, a hydroxy group, or an acetyloxy group, $R^{12}$ represents a hydrogen atom or a hydroxy group, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or a hydroxy group, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OH$, $R^{22}$ represents a hydrogen atom or a hydroxy group, $R^{21}$ and $R^{22}$ may combine with each other to form $=CH_2$, $R^{23}$ represents a hydrogen atom or a hydroxy group, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or a hydroxy group, $R^{25}$ represents a hydrogen atom, $R^{24}$ and $R^{25}$ may combine with each other to form an oxo group, and $R^{26}$ represents a hydrogen atom;

$b^3$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ and $R^{27}$ combine with each other to form-O-, $R^{16}$ represents a hydrogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a hydroxy group or an acetyloxy group;

$c^3$: a combination wherein $R^1$ represents a methyl group, $R^2$ represents a hydroxy group, $R^3$ represents a hydrogen atom, $R^2$ and $R^3$ may combine with each other to form an oxo group, $R^3$ and $R^{18}$ may combine with each

other to form $\#^3$-O-$CH_2$- or $\#^3$-O-CH($OCH_3$)-, $R^4$ and $R^{27}$ combine with each other to form $\#^4$-$CH_2$-O-, $R^5$ represents a methyl group, $R^6$ represents a hydrogen atom or a hydroxy group, $R^7$ and $R^{11}$ combine with each other to form -O-, $R^8$ represents a hydrogen atom, a halogen atom, a methoxy group, or a hydroxy group, $R^9$ represents a methyl group or $CH_2OH$, $R^{10}$ represents a hydrogen atom or a hydroxy group, $R^{12}$ represents a hydrogen atom, $R^{13}$ represents a hydrogen atom or a hydroxy group, $R^{14}$ represents a hydrogen atom, $R^{15}$ represents a hydrogen atom, $R^{16}$ represents a hydrogen atom, a halogen atom, or a hydroxy group, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form $\#^{15}$-O-$CH_2$- or $\#^{15}$-O-CH($OCH_3$)-, $R^{19}$ represents a hydrogen atom, $R^{20}$ represents a hydrogen atom or a hydroxy group, $R^{21}$ represents a methyl group, $CH_2OH$, or $CH_2NHCH_2C(O)OCH_3$, $R^{22}$ represents a hydrogen atom, $R^{18}$ and $R^{22}$ may combine with each other to form $\#^{18}$-$CH_2$-O-, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom;

$d^3$: a combination wherein $R^1$ and $R^{27}$ combine with each other to form $\#^1$-$CH_2$-O-, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom or a hydroxy group, $R^{12}$ represents a hydrogen atom, $R^{13}$ represents a hydrogen atom, $R^{14}$ represents a hydrogen atom, $R^{15}$ represents a hydroxy group, $R^{16}$ represents a hydrogen atom, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^3$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, a dichloromethyl group, or a hydroxy(methoxy)methyl group, $R^{14}$ represents a hydrogen atom, $R^{15}$ represents a hydrogen atom, $R^{16}$ represents a hydrogen atom, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents a hydroxy group, $R^{25}$ represents a hydrogen atom, $R^{24}$ and $R^{25}$ may combine with each other to form an oxo group, $R^{26}$ represents a hydrogen atom, and $R^{27}$ represents a methoxy group or a hydroxy group;

$f^3$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ and $R^{27}$ combine with each other to form a bond, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents a hydroxy group or an acetyloxy group, $R^{25}$ represents a hydrogen atom, $R^{24}$ and $R^{25}$ may combine with each other to form an oxo group, and $R^{26}$ represents a hydrogen atom; and

$g^3$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ represents a hydrogen atom, $R^8$ represents a hydrogen atom, $R^9$ represents a methyl group, $R^{10}$ represents a hydrogen atom, $R^{11}$ represents a hydroxy group, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a methoxy group or a hydroxy group.

[Embodiment 26] The compound described in the embodiment 25 wherein a combination of $R^1$, $R^2$, $R^3$, $R^4$, $R^5$, $R^6$, $R^7$,

$R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^3$, $b^3$, $c^3$, $d^3$, $e^3$ or $g^3$.

[Embodiment 27] The compound described in the embodiment 25 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^3$, $b^3$, $e^3$ or $g^3$.

[Embodiment 28] The compound described in the embodiment 25 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^3$, $b^3$ or $e^3$.

[Embodiment 29] The compound described in the embodiment 25 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^3$.

[Embodiment 30] The compound described in the embodiment 25 wherein a combination of $R^1$, $R^2$, $R^3$, $R^4$, $R^5$, $R^6$, $R^7$, $R^8$, $R^9$ $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $b^3$.

[Embodiment 31] The compound described in the embodiment 25 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $e^3$.

[Embodiment 32] The compound described in the embodiment 25 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $g^3$.

[Embodiment 33] The present compound wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^4$, $b^4$, $e^4$ or $g^4$.

$a^4$: a combination wherein $R^1$ represents a hydrogen atom, a methyl group, an ethyl group or $CH_2OH$, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^3$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ and $R^{27}$ combine with each other to form -O-, $R^{16}$ represents a hydrogen atom or a halogen atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom;

$b^4$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ and $R^{27}$ combine with each other to form - O-, $R^{16}$ represents a hydrogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a hydroxy group or an acetyloxy group;

$e^4$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents a hydroxy group, $R^{25}$ represents a hydrogen atom, $R^{24}$ and $R^{25}$ may combine with each other to form an oxo group, $R^{26}$ represents a hydrogen atom, and $R^{27}$ represents a methoxy group or a hydroxy group; and

$g^4$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ represents a hydrogen atom, $R^8$ represents a hydrogen atom, $R^9$ represents a methyl group, $R^{10}$ represents a hydrogen atom, $R^{11}$ represents a hydroxy group, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen

atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a methoxy group or a hydroxy group.

[Embodiment 34] The compound described in the embodiment 33 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $a^4$.
[Embodiment 35] The compound described in the embodiment 33 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21,}$ $R^{22}$, $R^{23}$, $R^{24}$, $R^{25}$, $R^{26}$, and $R^{27}$ represents the combination $b^4$.
[Embodiment 36] The compound described in the embodiment 33 wherein a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $e^4$.
[Embodiment 37] The compound described in the embodiment 33 wherein a combination of $R^1$, $R^2$, $R^3$, $R^4$, $R^5$, $R^6$, $R^7$, $R^3$ $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents the combination $g^4$.

Examples of the embodiments of the compound of the present invention include the following compounds.
[Embodiment A1] The compound A of the present invention wherein $R^1$ represents a hydrogen atom, a methyl group, an ethyl group or $CH_2OH$, $R^2$ and $R^3$ combine with each other to form an oxo group, $R^4$ represents a methyl group, $R^5$ represents a methyl group, $R^6$ represents a hydrogen atom, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, X represents a halogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom.
[Embodiment A2] The compound A of the present invention wherein $R^1$ represents a hydrogen atom, a methyl group, an ethyl group or $CH_2OH$, $R^2$ and $R^3$ combine with each other to form an oxo group, $R^4$ represents a methyl group, $R^5$ represents a methyl group, $R^6$ represents a hydrogen atom, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, X represents a chlorine atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom.
[Embodiment A3] The compound A of the present invention wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, X represents a halogen atom, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom.
[Embodiment A4] The compound A of the present invention wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, X represents a chlorine atom, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom.
[Embodiment A5] The compound A of the present invention wherein X represents a chlorine atom.
[Embodiment B1] A compound represented by formula (AA1), formula (AA2), formula (AA3), formula (BB1), formula (EE1), formula (EE3), formula (EE4), formula (EE5), formula (EE6), formula (EE7), formula (GG1), formula (GG2), or formula (GG3), or an N-oxide compound thereof or a salt thereof.

[Embodiment B2] A compound represented by formula (AA1), formula (AA2), formula (AA3), formula (BB1), formula (EE1), formula (EE3), formula (EE4), formula (EES), formula (EE6), formula (GG1), or formula (GG3), or an N-oxide compound thereof or a salt thereof.

[Embodiment B3] A compound represented by formula (AA1), or formula (AA2), or an N-oxide compound thereof or a salt thereof.

[Embodiment B4] A compound represented by formula (EE2).

Next, the process for preparing the present compound and the compound of the present invention is explained.

The present compound can be obtained according to the method described in Heterocyclic Communications (1995), 1(2-3), 207: Journal of Organic Chemistry (1985), 50(20), 3757; Heterocycles (1989), 28(1), 103; Tetrahedron (1998), 54(27), 7891; Tetrahedron (1999), 55(17), 5539; Marine Drugs (2014), 12(10), 5188; Tetrahedron Letters (2014), 55(39), 5369; Tetrahedron (2015), 71(45), 8601; Journal of Natural Products (2019), 82(10), 2790; Journal of Natural Products (2016), 79(10), 2674; Marine Drugs (2018), 16(7), 242; Tetrahedron Letters (2008), 49(14), 2189; J. Org. Chem. 2020, 85, 12553; Bioorganic Chemistry 109 (2021) 104700; Bulletin of the Chemical Society of Japan (1998), 71(4), 771; Heterocyclic Communications (1998), 4(6), 575; Angewandte Chemie (International ed. in English) (2009), 48(47), 8905; Chemistry - An Asian Journal (2011), 6(3), 922; Heterocyclic Communications (1995), 1(2-3), 207; Tetrahedron Letters (1997), 38(32), 5683; JP H10-101678 A1 Description; US patent publication No. 2016/00360974 A1 Description; Tetrahedron Letters (2008), 49(14), 2189; 62nd Symposium on the Chemistry of Natural Products, symposium abstract (2020), p.2-8 etc. Also, these compounds can be prepared according to the below-mentioned processes.

Process 1

A compound represented by formula (II) (hereinafter, referred to as "Compound (II)") can be prepared by reacting a compound represented by formula (II-a) (hereinafter, referred to as "Compound (II-a)" with a halogenating agent.

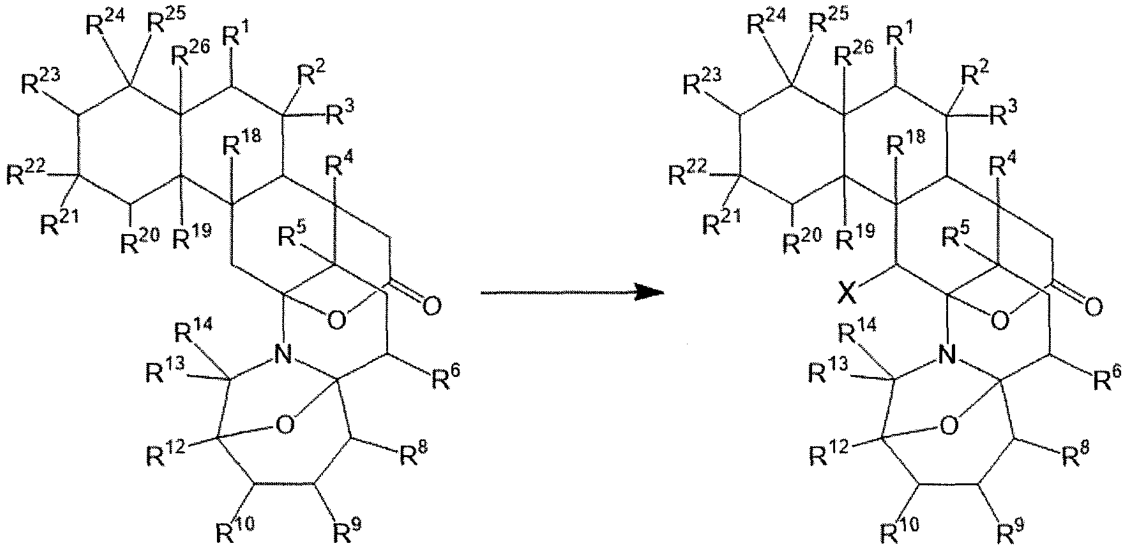

( II - a ) ( II )

[wherein the symbols are the same as defined above.]

The reaction is usually carried out in a solvent. Examples of the solvent include halogenated hydrocarbons (such as dichloromethane, chloroform); nitriles (such as acetonitrile); carbon disulfide; acetic acid; and mixtures of two or more of these solvents.

Examples of the halogenating agents include succinimides (such as N-chlorosuccinimide, N-bromosuccinimide, N-iodosuccinimide); and halogens (such as fluorine, chlorine, bromine, and iodine); and the like.

The reaction may be conducted in the presence of an additive. Examples of the additives include p-toluene sulfonic acid monohydrate.

In the reaction, a halogenating agent is used usually within a range of 1 to 2 molar ratio (s), as opposed to 1 mole of the compound (II-a).

The reaction temperature of the reaction is usually within a range of -10 to 50°C. The reaction period of the reaction is usually within a range of 0.1 to 36 hours.

When the reaction is completed, water is added to reaction mixtures, and the reaction mixtures are extracted with organic solvent(s), and the resulting organic layers are dried and concentrated to obtain the compound (II).

The compound (II-a) is a publicly known, or can be prepared or obtained according to the above-mentioned method for obtaining the present compound or the publicly known methods.

Process 2

The salts of the present compound or the compound of the present invention can be prepared, for example, according to methods described in the JP H10-53589 A1, Bulletin of the Chemical Society of Japan (1998), 71(4), 771; Chemistry - An Asian Journal (2011), 6(3), 922; and Pure and Applied Chemistry (2007), 79, 651.

The present compound can be mixed or combined with one or more ingredients selected from the group consisting of the following Group (a), Group (b), Group (c), Group (d), and Group (e), (hereinafter, referred to as "present ingredient") .

The mixing or combining represents that the present compound and the present ingredient are used concurrently, separately, or at an interval.

When the present compound and the present ingredient are concurrently used, the present compound and the present ingredient may be incorporated as a separate formulation or one formulation.

One aspect of the present invention relates to a composition comprising one or more ingredients selected from the group consisting of the Group (a), the Group (b), the Group (c), the Group (d), and the Group (e), and the present compound (hereinafter, referred to as "Composition A").

The compound of the present invention can be mixed or combined with one or more ingredients selected from the group consisting of the following Group (a), Group (b), Group (c), Group (d), and Group (e), (hereinafter, referred to as "present ingredient").

The mixing or combining represents that the compound of the present invention and the present ingredient are used concurrently, separately, or at an interval.

When the compound of the present invention and the present ingredient are concurrently used, the present compound and the present ingredient may be incorporated as a separate formulation or one formulation.

One aspect of the present invention relates to a composition comprising one or more ingredients selected from the group consisting of the Group (a), the Group (b), the Group (c), the Group (d), and the Group (e), and the compound of the present invention (hereinafter, referred to as "Composition B").

The Group (a) represents insecticidal active ingredients, miticidal active ingredients and nematicidal active ingredients that are a group consisting of acetylcholinesterase inhibitors (for example, carbamate insecticides and organophosphate insecticides), GABA-gated chloride ion channel blockers (for example, phenylpyrazole insecticides), sodium channel modulators (for example, pyrethroid insecticides), nicotinic acetylcholine receptor antagonist modulators (for example, neonicotinoid insecticides), nicotinic acetylcholine receptor allosteric modulators, glutamate-gated chloride ion channel competitive modulators (for example, macrolide insecticides), juvenile hormone mimics, multisite inhibitors, chordotonal organ TRPV channel modulators, mites growth regulators, microbial disruptors of insect midgut membranes, mitochondrial ATP synthase inhibitors, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, Inhibitors of mitochondrial electron transport chain complex I, II, III, and IV, voltage-dependent sodium channel blockers, Inhibitors of acetyl CoA carboxylase, ryanodine receptor modulators (for example, diamide insecticides), chordotonal organ modulators, as well as other insecticidal active ingredients, miticidal active ingredients and nematicidal active ingredients. These agents are described in the classification based on the IRAC mode of action.

The Group (b) is a group consisting of nucleic acid synthesis inhibitors (for example, phenylamide fungicides and acylamino acid fungicides), cytostatic and cytoskeletal inhibitors (for example, MBC fungicides), respiration inhibitors (for example, QoI fungicides and QiI fungicides), amino-acid synthesis and protein synthesis inhibitors (for example, anilinopyridine fungicides), signal-transduction inhibitors, lipid synthesis and membrane synthesis inhibitors, sterol biosynthesis inhibitors (for example, DMI fungicides such as triazoles), cell wall synthesis inhibitors, melanin synthesis inhibitors, plant defense inducer, multisite fungicides, and other fungicidal active ingredients. These agents are described in the classification based on the FRAC mode of action.

The Group (c) represents a group of plant growth regulatory ingredients.

The Group (d) represents a group of repellent active ingredients.

The Group (e) represents a group of biological control materials consisting of bacteria, fungus, yeast, protist, virus, a product produced by an organism, plant, and plant extract

Examples of combinations of the present ingredient and the present compound or the compound of the present invention are recited as follows. For example, the "alanycarb + SX" indicates a combination of alanycarb and SX.

The abbreviation "SX" means to any one of the present compounds or the compounds of the present invention

selected from the present compounds A-1 to A-23, B-1, B-2, C-1 to C-44, D-1, D-2, E-1 to E-5, F-1 to F-5, G-1 and G-2, as well as the compounds AA-1 to AA-3, BB-1, EE-1 to EE-7, and GG-1 to GG-3 of the present invention. Also, any of the present ingredients as described below are a known ingredient, and can be obtained from a commercially available drug or can be prepared according to a known method. When the present ingredient represents a microorganism, the present ingredient can be obtained from a microorganism depositary authority. The number in parentheses represents CAS RN (registered trademark).

A combination of the present ingredient in the above-mentioned Group (a) and the present compound or the compound of the present invention:

abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat) + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, emamectin-benzoate + SX, empenthrin + SX, endosulfan + SX, EPN(O-ethyl-O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium

+ SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yllindan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl] phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(3-pyridinyl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX.

A combination of the present ingredient in the above-mentioned Group (b) and the present compound or the compound of the present invention:

acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarbisopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chloroinconazide + SX, chloroneb + SX, chlorothalonil + SX, chlozolinate + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin +SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, quinconazole + SX, quinofumelin + SX, quinoxyfen + SX, quintozene + SX, Saponins of Chenopodium quinoa + SX,

seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl] ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl) butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl) carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl] -4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-S-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino] -N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl] -5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl] - 5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX.

A combination of the present ingredient in the above-mentioned Group (c) and the present compound or the compound of the present invention:

1-methylcyclopropene + SX, 1,3,5-triazinane-2,4,6-trithione + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy) acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, pyroglutamic acid + SX, sintofen + SX, sodium 1-naphthaleneacetate + SX, sodium cyanate + SX, thidiazuron + SX, titanium apatite + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyic acid + SX, methyl [5-(trifluoromethyl)benzo[b] thiophen-2-carboxylate + SX, and 3-[(6-chloro-4-phenylquinazolin-2-yl)amino)propane-1-ol + SX.

A combination of the present ingredient in the above-mentioned Group (d) and the present compound or the compound of the present invention:

anthraquinone + SX, deet + SX, icaridin + SX

A combination of the present ingredient in the above-mentioned Group (e) and the present compound or the compound of the present invention:

Abbreviate caucasica + SX, Acuaria spp. + SX, Agamermis decaudata + SX, Agrobacterium radiobacter strain K1026 + SX, Agrobacterium radiobacter strain K84 + SX, Agrobacterium vitis strain VAR03-1 + SX, Allantonema spp. + SX, Alternaria destruens strain 59 + SX, Alternaria destruens + SX, Ampelomyces quisqualis strain AQ10 + SX, Amphimermis spp. + SX, Arthrobotrys dactyloides + SX, Arthrobotrys superba + SX, Aschersonia aleyrodis + SX, Aspergillus flavus strain AF36 + SX, Aspergillus flavus strain NRRL21882 + SX, Aureobasidium pullulans strain DSM14940 + SX, Aureobasidium pullulans strain DSM14941 + SX, Azorhizobium caulinodans strain ZB-SK-5 + SX, Azotobacter chroococcum strain H23 + SX, Azotobacter chroocuccum + SX, Azotobacter vinelandii strain ATCC12837 + SX, Azotobacter + SX, Bacillus acidocaldarius + SX, Bacillus acidoterrestris + SX, Bacillus Albolactis + SX, Bacillus alcalophilus + SX, Bacillus alvei + SX, Bacillus aminoglucosidicus + SX, Bacillus aminovorans + SX, Bacillus amyloliquefaciens strain PTA-4838 (Aveo (trade mark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain FZB24 + SX, Bacillus amyloliquefaciens strain FZB42 + SX, Bacillus amyloliquefaciens strain GB03 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. Plantarum strain D747 +SX, Bacillus amylolyticus + SX, Bacillus aneurinolyticus + SX, Bacillus agri + SX, Bacillus atrophaeus + SX, Bacillus atrophaeus strain Abi05 + SX, Bacillus azotoformans + SX, Bacillus badius + SX, Bacillus cereus strain BP01 + SX, Bacillus cereus CNCM strain I-1562 + SX, Bacillus chitinosporus strain AQ746 + SX, Bacillus chitinosporus strain CM-1 + SX, Bacillus circulans + SX, Bacillus coagulans strain TQ33 + SX, Bacillus fastidiosus + SX, Bacillus firmus strain GB-126 + SX, Bacillus firmus strain I-1582 + SX, Bacillus firmus strain NCIM2637 + SX, Bacillus lacticola + SX, Bacillus lactimorbus + SX, Bacillus lactis + SX, Bacillus lautus + SX, Bacillus Lentimorbus + SX, Bacillus lentus + SX, Bacillus licheniformis strain FMCH001 + SX, Bacillus licheniformis strain HB-2 + SX, Bacillus licheniformis strain SB3086 + SX, Bacillus maroccanus + SX, Bacillus medusa + SX, Bacillus megaterium strain YFM3.25 + SX, Bacillus methylotrophicus strain BAC-9912 + SX, Bacillus metiens + SX, Bacillus mojavensis strain SR11 + SX, Bacillus mycoides strain AQ726 + SX, Bacillus mycoides isolate J + SX, Bacillus nematocida + SX, Bacillus nigrificans + SX, Bacillus nigrum + SX, Bacillus popilliae + SX, Bacillus psychrosaccharolyticus + SX, Bacillus pumilus strain AQ717 + SX, Bacillus pumilus strain BUF-33 + SX, Bacillus pumilus strain GB34 + SX, Bacillus pumilus strain QST2808 + SX, Bacillus siamensis + SX, Bacillus simplex strain CGF2856 + SX, Bacillus smithii + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus subtilis strain AFS032321 + SX, Bacillus subtilis strain AQ153 + SX, Bacillus subtilis strain AQ743 + SX, Bacillus subtilis strain B246 + SX, Bacillus subtilis strain C-3102 + SX, Bacillus subtilis strain D747 + SX, Bacillus subtilis strain DB101 + SX, Bacillus subtilis strain DB102 + SX, Bacillus subtilis strain FMCH002 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens

strain FZB24 + SX, Bacillus subtilis strain Y1336 + SX, Bacillus subtillis subsp.natto + SX, Bacillus subtilis strain RTI477 + SX, Bacillus subtilis strain N5 NRRL B-50391 + SX, Bacillus subtilis strain DSM17231 + SX, Bacillus tequilensis strain NII-0943 + SX, Bacillus thuringiensis strain AQ52 + SX, Bacillus thuringiensis strain BD#32 + SX, Bacillus thuringiensis strain CR-371 + SX, Bacillus thuringiensis strain EX297512 + SX, Bacillus thuringiensis subsp. Aizawai strain ABTS-1857 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG2371 + SX, Bacillus thuringiensis subsp. Kurstaki strain EG7673 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain M-200 + SX, Bacillus thuringiensis subsp. Kurstaki strain NCIM2514 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Kurstaki strain VBTS-2546 + SX, Bacillus thuringiensis subsp. Kurstaki strain Z-52, serotype H-3a,3b + SX, Bacillus thuringiensis subsp. morrisoni + SX, Bacillus thuringiensis subsp. Tenebriosis strain NB 176 + SX, Bacillus thuringiensis subsp. tenebriosis strain SA-10 + SX, Bacillus thuringiensis subsp. Thuringiensis strain MPPL002 + SX, Bacillus thuringiensis subsp. var. colmeri + SX, Bacillus thuringiensis subsp. var. darmstadiensis strain 24-91 + SX, Bacillus thuringiensis subsp. var. dendrolimus + SX, Bacillus thuringiensis subsp. var. galleriae + SX, Bacillus thuringiensis subsp. israelensis strain BMP144 + SX, Bacillus thuringiensis subsp. israelensis strain AM65-52 + SX, Bacillus thuringiensis subsp. israelensis (serotype H-14) strain AM65-52 + SX, Bacillus thuringiensis subsp. israelensis strain EG2215 + SX, Bacillus thuringiensis subsp. israelensis strain SA3A + SX, Bacillus thuringiensis subsp. israelensis strain SUM-6218 + SX, Bacillus thuringiensis subsp. israelensis strain VCRC B17 serotype 11-14 + SX, Bacillus thuringiensis subsp. var. japonensis strain buibui + SX, Bacillus thuringiensis subsp. var. aegypti + SX, Bacillus thuringiensis var. 7216 + SX, Bacillus thuringiensis var. sandiego strain M-7 + SX, Bacillus thuringiensis var. T36 + SX, Bacillus velezensis + SX, Bacillus velezensis strain RTI301 + SX, Bacillus velezensis strain FZB42 + SX, bacteriophage of Clavibacter michiganesis + SX, Beauveria bassiana strain ANT-03 + SX, Beauveria bassiana strain ATCC74040 + SX, Beauveria bassiana strain GHA + SX, Beauveria bassiana strain PPRI 5339 + SX, Beauveria bassiana strain IMI389521 + SX, Beauveria brongniartii + SX, Deladenus siridicola + SX, Bovienema spp. + SX, Brevibacillus brevis strain 2904 + SX, Brevibacillus brevis strain SS86-3 + SX, Brevibacillus brevis strain SS86-4 + SX, Brevibacillus brevis strain SS86-5 + SX, Brevibacillus laterosporus strain ATCC64 + SX, Brevibacillus laterosporus strain BPM3 + SX, Brevibacillus laterosporus strain G4 + SX, Brevibacillus laterosporus strain NCIMB 41419 + SX, Brevibacillus laterosporus strain NRS1111 + SX, Brevibacillus laterosporus strain NRS1645 + SX, Brevibacillus laterosporus strain NRS1647 + SX, Burkholderia cepacia type Wisconsin strain J82 + SX, Burkholderia cepacia type Wisconsin strain M54 + SX, Burkholderia cepacia + SX, Burkholderia gladii strain BA-7 + SX, Burkholderia rinojensis strain A396 + SX, Cameronia spp. + SX, Candida oleophila strain O + SX, Candida saitoana + SX, Candida spp. + SX, Chaetomium cupreum + SX, Chitwoodiella ovofilamenta + SX, Chromobacterium subtsugae strain PRAA4-1T + SX, Cladosporium cladosporioides strain H39 + SX, Conidiobolus obscurus + SX, Coniothyrium minitans strain CGMCC8325 + SX; Coniothyrium minitans strain CON/M/91-08 + SX, Contortylenchus spp. + SX, cryptococcus albidus + SX, Culicimermis spp. + SX, Dactyllela ellipsospora + SX, Dectylaria thaumasia + SX, Delftia acidovorans strain RAY209 + SX, Dilophosphora alopecuri + SX, Diplotriaena spp. + SX, Empidomermis spp. + SX, Entomophthora virulenta + SX, Erwinia carotovora subsp. carotovora strain CGE234M403 + SX, Filipjevimermis leipsandra + SX, Fusarium oxysporum strain Fo47 + SX, Fusarium oxysporum strain RS-21 + SX, Fusarium oxysporum strain RS-25 + SX, Fusariumproliferatum + SX, Gastromermis spp. + SX, Gliocladium catenulatum strain J1446 + SX, gluconacetobacter diazotrophicus + SX, Gongylonema spp. + SX, Gynopoecilia pseudovipara + SX, Harpin protein + SX, Heterorhabditis bacteriophora + SX, Heterorhabditis baujardi + SX, Heterorhabditis heliothidis + SX, Heterorhabditis indica + SX, Heterorhabditis marelatus + SX, Heterorhabditis megidis + SX, Heterorhabditis zealandica + SX, Hexamermis spp. + SX, Hirsutella minnesotensis + SX, Hirsutella rhossiliensis + SX, Hirsutella thompsonii + SX, Hydromermis spp. + SX, Isaria fumosorosea + SX, Isomermis spp. + SX, Lactobacillus acidophilus + SX, Lactobacillus brevis + SX, Lactobacillus plantarum + SX, Lactobacillus spp. + SX, Lagenidium giganteum + SX, Lecanicillium lecanii KV01 + SX, Lecanicillium lecanii conidia of strain DAOM198499 + SX, Lecanicillium lecanii conidia of strain DAOM216596 + SX, Lecanicillium muscarium strain Ve6 + SX, Limnomermis spp. + SX, Lysobacter antibioticus strain 13-1 + SX, Lysobacter enzymogenes strain C3 + SX, Maupasina weissi + SX, Mermis nigrescens + SX, Mesomermis spp. + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Metschnikowia fructicola strain NRRLY-30752 + SX, Microsphaeropsis ochracea + SX, Monacrosporium phymatopagum + SX, Mucor hiemalis + SX, Muscodor albus strain 620 + SX, Muscodor albus strain QST 20799 + SX, Muscodor albus strain SA13 + SX, Muscodor roseus strain A3-5 + SX, Myrothecium verrucaria strain AARC-0255 + SX, Neomesomermis spp. + SX, Neoparasitylenchus rugulosi + SX, Nomuraea rileyi strain CG128 + SX, Nomuraea rileyi strain GU87401 + SX, Nomuraea rileyi strain SA86101 + SX, Nomuraea rileyi strain SR86151 + SX, Nomuraea rileyi strain VA9101 + SX, Nosema locustae + SX, Octomyomermis spp. + SX, Ophiostoma piliferum strain D97

+ SX, Isaria fumosorosea Apopka strain 97 + SX, Paecilomyces lilacinus strain 251 + SX, Paecilomyces tenuipes strain T1 + SX, Paecilomyces variotii strain Q-09 + SX, Paenibacillus alvei strain 2771 + SX, Paenibacillus alvei strain 46C3 + SX, Paenibacillus alvei strain III2E + SX, Paenibacillus alvei strain III3DT-1A + SX, Paenibacillus alvei strain T36 + SX, Paenibacillus macerans + SX, Paenibacillus polymyxa strain AC-1 + SX, Paenibacillus polymyxa strain BS-0105 + SX, Paenibacillus popilliae + SX, Pandora delphacis + SX, Pantoea agglomerans strain E325 + SX, Pantoea vagans strain C9-1 + SX, Parasitaphelenchus spp. + SX, Parasitorhabditis spp. + SX, Parasitylenchus spp. + SX, Pasteuria nishizawae strain Pn1 + SX, Pasteuria penetrans + SX, Pasteuria ramose + SX, Pasteuria usgae + SX, Pectobacterium carotovorum + SX, Penicillium bilaiae strain ATCC22348 + SX, Penicillium citrinum strain VFI-51 + SX, Penicillium vermiculatum + SX, Perutilimermis culicis + SX, Pasteuria thornei + SX, Phasmarhabditis hermaphrodita + SX, phlebiopsis gigantea strain VRA1992 + SX, Physaloptera spp. + SX, phytophthora palmivora + SX, Pichia anomala strain WRL-076 + SX, pichia guilliermondii strain Z1 + SX, pichia guilliermondii strain Z8 + SX, Pochonia chlamydosporia isolate strain PC10 + SX, Pochonia chlamydosporia + SX, Protrellatus spp. + SX, Pseudomonas aeruginosa strain PN1 + SX, Pseudomonas aeruginosa strain WS-1 + SX, Pseudomonas aureofaciens strain TX-1 + SX, Pseudomonas cepacia type Wisconsin strain J82 + SX, Pseudomonas cepacia type Wisconsin strain M54 + SX, Pseudomonas chlororaphis strain 63-28 + SX, Pseudomonas chlororaphis strain AFS009 + SX, Pseudomonas chlororaphis strain MA342 + SX, pseudomonas fluorescens strain 1629RS + SX, pseudomonas fluorescens strain A506 + SX, pseudomonas fluorescens strain CL145A + SX, Pseudomonas fluorescens strain G7090 + SX, Pseudomonas fluorescens biover B strain XJ3 + SX, Pseudomonas fluorescens biover B strain XS18 + SX, Pseudomonas fluorescens biover A strain LRS12 + SX, Pseudomonas proradix + SX, Pseudomonas putida + SX, Pseudomonas reactans + SX, Pseudomonas resinovorans + SX, Pseudomonas rhodesiae strain HAI-0804 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pterygodermatites spp. + SX, puccinia thlaspeos strain woad + SX, Purpureocillium lilacinum + SX, Pythium oligandrum strain DV74 + SX, Pythium oligandrum strain M1 + SX, Rhodococcus globerulus strain AQ719 + SX, Romanomermis spp. + SX, saccharomyces cerevisiae strain LAS02 + SX, saccharomyces cerevisiae strain DDSF623 + SX, sclerotinia minor strain IMI34414 + SX, Serratia entomophila + SX, Serratia marcescens strain 35 + SX, Serratia marcescens strain SRM + SX, Seuratum cadarachense + SX, Sphaerulariopsis spp. + SX, Spirura guianensis + SX, Sporothrix insectorum + SX, Steinernema bibionis + SX, Steinernema carpocapsae + SX, Steinernema feltiae + SX, Steinernema glaseri + SX, Steinernema kraussei + SX, Steinernema riobrave + SX, Steinernema scapterisci + SX, Steinernema scarabaei + SX, Steinernema siamkayai + SX, Steinernema thailandse + SX, Steinernema spp. + SX, Strelkovimermis peterseni + SX, Streptomyces acidiscabies strain RL-110T + SX, Streptomyces candidus strain Y21007-2 + SX, Streptomyces colombiensis + SX, Streptomyces galbus strain QST6047 + SX, Streptomyces goshikiensis + SX, Streptomyces griseoviridis strain K61 + SX, Streptomyces lavendulae + SX, Streptomyces lydicus strain WYCD108US + SX, Streptomyces lydicus strain WYEC108 + SX, Streptomyces microflavus strain AQ6121 + SX, Streptomyces prasinus + SX, Streptomyces rimosus + SX, Streptomyces saraceticus + SX, Streptomyces sp. strain NRRL No.B-30145 + SX, Streptomyces sp. strain WYE20 + SX, Streptomyces sp. strain WYE 324 + SX, Streptomyces venezuelae + SX, Subulura spp. + SX, Sulphuretylenchus elongatus + SX, Talaromyces flavus strain SAY-Y-94-01 + SX, Talaromyces flavus strain V117b + SX, Tetrameres spp. + SX, Thelohania solenopsis + SX, Thiobacillus spp. + SX, Trichoderma album + SX, Trichoderma asperelloides strain JM41R + SX, Trichoderma asperellum strain ICC012 + SX, Trichoderma asperellum T25 + SX, Trichoderma asperellum strain T34 + SX, Trichoderma asperellum strain SKT-1 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB 104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma harzianum rifai + SX, Trichoderma harzianum strain K2 + SX, Trichoderma harzianum strain FllBab + SX, Trichoderma harzianum strain RR17Bc + SX, Trichoderma koningii + SX, Trichoderma lignorum + SX, Trichoderma polysporum strain IMI206039 + SX, Trichoderma spp. + SX, trichoderma stromaticum + SX, Trichoderma virens strain G-41 + SX, Trichoderma virens strain GL-21, formaly Gliocladium virens GL-21 + SX, Trichoderma viride + SX, Trichoderma viride strain K5 + SX, Trichoderma viride strain NRRL B-S0S20 + SX, Tsukamurella paurometabola + SX, Typhula phacorrhiza strain 94671 + SX, Ulocladium oudemansii strain HRU3 + SX, Vairimorpha spp. strain Q-09 + SX, Variovorax paradoxus strain GF4526 + SX, Verticillium alboatrum strain WCS850 + SX, Verticillium chlamydosporium + SX, Verticillium dahliae + SX, Verticillium lecani strain NCIM1312 + SX, Virgibacillus pantothenticus strain ATCC14576 + SX, Virgibacillus pantothenticus strain DSM491 + SX, Wolbachia pipientis + SX, Xanthomonas campestris pv poae + SX, Xanthomonas campestris + SX, Xenorhabdus luminescens + SX, Xenorhabdus nematophila + SX, Zoophtora radicans + SX, Acaulospora spp. + SX, Ambispora spp. + SX, Archaeospora spp. + SX, Claroideoglomus spp. + SX, Claroideoglomus

etunicatum + SX, Claroideoglomus claroideum + SX, Diversispora spp. + SX, Entrophospora spp. + SX, Funneliformis spp. + SX, Funneliformis mosseae + SX, Geosiphon spp. + SX, Gigaspora spp. + SX, Gigaspora margarita + SX, Gigaspora rosea + SX, Glomus spp. + SX, Glomus aggregatum + SX, Glomus deserticola + SX, Glomus monosporum + SX, Pacispora spp. + SX, Paraglomus spp. + SX, Paraglomus brasilianum + SX, Racocetra spp. + SX, Rhizophagus spp. + SX, Rhizophagus clarus + SX, Rhizophagus intraradices + SX, Rhizophagus irregularis + SX, Rhizophagus irregularis strain DAOM 197198 + SX, Scutellospora spp. + SX, Azorhizobium spp. + SX, Azorhizobium caulinodans + SX, Azospirillum amazonense + SX, Azospirillum brasilense + SX, Azospirillum brasilense strain XOH + SX, Azospirillum brasilense strain Ab-V5 + SX, Azospirillum brasilense strain Ab-V6 + SX, Azospirillum caulinodans + SX, Azospirillum halopraeferens + SX, Azospirillum irakense + SX, Azospirillum lipoferum + SX, Bradyrhizobium spp. + SX, Bradyrhizobium elkanii + SX, Bradyrhizobium elkanii strain SEMIA 587 + SX, Bradyrhizobium elkanii strain SEMIA 5019 + SX, Bradyrhizobium japonicum + SX, Bradyrhizobium japonicum strain TA-11 + SX, Bradyrhizobium japonicum strain USDA 110 + SX, Bradyrhizobium liaoningense + SX, Bradyrhizobium lupini + SX, Bradyrhizobium spp. (Arachis) + SX, Bradyrhizobium spp. (Vigna) + SX, Delftia acidovorans + SX, Delftia acidovorans strain RAY209 + SX, Metylobacterium spp. + SX, Mesorhizobium ciceri + SX, Mesorhizobium huakii + SX, Mesorhizobium loti + SX, Mesorhizobium spp. + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Arkansas Fungus 18 + SX, Beauveria bassiana strain ATP02 + SX, Beauveria bassiana strain CG716 + SX, Candida oleophila isolate 1-182 + SX, Colletotrichum gloeosporioides + SX, Cryptococcus flavescens + SX, Gliocladium roseum + SX, Glomus etunicatum + SX, Glomus iranicum + SX, Laccaria bicolor + SX, Laccaria laccata + SX, Lecanicillium muscarium strain 1/1 + SX, Metarhizium anisopliae var. acridum isolate IMI 330189 + SX, Isaria fumosorosea strain FE9901 + SX, Phlebiopsis + SX, Phoma macrostroma strain94-44B + SX, Acaulospora longula + SX, Ambispora leptoticha + SX, cell walls of Saccharomyces cerevisiae strain LAS117 + SX, Claroideoglomus claroideum + SX, Claroideoglomus luteum + SX, Pisolithus tinctorius + SX, Rhizopogon amylopogon + SX, Rhizopogon fulvigleba + SX, Rhizopogon luteolus + SX, Rhizopogon villosullus + SX, Scleroderma cepa + SX, Scleroderma citrinum + SX, Suillus granulatus + SX, Suillus punctatapies + SX, Trichoderma asperellum strain kd + SX, Trichoderma atroviride strain LU132 + SX, Trichoderma atroviride strain 77B + SX, Trichoderma atroviride strain K4 + SX, Trichoderma atroviride strain WW10TC4 + SX, Trichoderma harmatum strain TH382 + SX, Trichoderma lignorum strain TL-0601 + SX, Trichoderma saturnisporium strain PBP-TH-001 + SX, Tsukamurella paurometabola strain C-924 + SX, Gluconactobacter diazotrophicus strain IMI504853 + SX, Clonostachys rosea strain IDAC 040913-01 + SX, Bacillus cereus Bromelia strain NRRL B-50766 + SX, Bacillus cereus Peumo strain NRRL B-50767 + SX, Bacillus thuringiensis Anemophyla strain NRRL B-50765 + SX, Trichoderma harzianuna strain Gomorteka NRRL 67322 + SX, Bacillus licheniformis strain DSM17236 + SX, Bacillus licheniformis strain copihue NRRL B-67023 + SX, Trichoderma reesei + SX, Corynebacterium flavescens strain B2575 + SX, Chlorella + SX, Methylobacterium isolate ISO01 + SX, Methylobacterium isolate ISO02 (NRRL B-50930) + SX, Methylobacterium isolate ISO03 (NRRL B-50931) + SX, Methylobacterium isolate ISO04 (NRRL B-50932) + SX, Methylobacterium isolate ISO07 + SX, Methylobacterium isolate ISO09 (NRRL B-50937) + SX, Methylobacterium isolate ISO10 (NRRL B-50938) + SX, Methylobacterium isolate ISO11 (NRRL B-50939) + SX, Klebsiella variicola 137 + SX, Adoxophyes orana GV (granulovirus) strain BV-0001 + SX, Anticarsia gemmatalis MNPV (multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Cydia pomonella GV strain CP4 + SX, Cydia pomonella GV strain R5 + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, BT crop protein Cry1Ab + SX, BT crop protein CrylAc + SX, BT crop protein CrylFa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, chitin + SX, colletochlorin B + SX, concanamycin A + SX, ryanodine + SX, cevadine + SX, veratridine + SX, pyrethrin I + SX, pyrethrin II + SX, cinerin I + SX, cinerin II + SX, jasmolin I + SX, jasmolin II + SX, leaves and bark of Quercus + SX, dried leaves of Dryopteris filix-mas + SX, extract of Artemisia absinthium + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, oil of the seeds of Chenopodium anthelminticum + SX, wood extract of Quassia amara + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, extract of Azadirachta indica + SX, Quillaja extract + SX, yellow mustard powder + SX, seed extract of Schoenocaulon spp. + SX, extract of Pyrethrum + SX, Gougerotin + SX, matrine + SX, ascaridole + SX, capsicin + SX, deguelin + SX, nootkatone + SX, pellitorine + SX, piperine + SX, extract from Swinglea glutinosa + SX, cytokinin + SX, N,N'-diformylurea + SX, salicylic acid + SX.

Examples of a ratio of the present compound or the compound of the present invention to the present ingredient include, but are not particularly limited to 1000 : 1 - 1 : 1000, 500 . 1 - 1 . 500, 100 . 1 - 1 . 100, 50 . 1, 20 . 1, 10 . 1, 9 : 1, 8 : 1, 7 : 1, 6 . 1, 5 : 1, 4 : 1, 3 . 1, 2 : 1, 1 : 1, 1 : 2, 1 : 3, 1 : 4, 1 : 5, 1 : 6, 1 : 7, 1 : 8, 1 : 9, 1 : 10, 1 : 20, and 1 : 50 in the weight ratio (the present compound or the compound of the present invention : the present ingredient).

The present compound and the compound of the present invention has control effect on harmful arthropods (such as harmful insects and harmful mites), and harmful nematodes. Examples of the harmful arthropods and the harmful nematodes include the followings.

Hemiptera:

from the family Delphacidae, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), Tagosodes orizicolus, Stenocranus pacificus, and the like;

from the family Cicadellidae, green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), Amrasca biguttula biguttula, and the like;

from the family Aphrophoridae, Philaenus spumarius and the like;

from the family Cercopidae, Mahanarva posticata, Mahanarva fimbriolata, and the like;

from the family Aphididae, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid (Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), English grain aphid (Sitobion avenae) and the like;

from the family Phylloxeridae, grapevine phylloxera (Daktulosphaira vitifoliae), Pecan phylloxera (Phylloxera devastatrix), Pecan leaf phylloxera (Phylloxera notabilis), Southern pecan leaf phylloxera (Phylloxera russellae), and the like;

from the family Adelgidae, hemlock woolly aphid (Adelges tsugae), Balsam woolly aphid (Adelges piceae), Aphrastasia pectinatae, and the like;

from the family Pentatomidae, black rice bug (Scotinophara lurida), Malayan rice black bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), Brown stink bug (Euschistus heros), Red banded stink bug (Piezodorus guildinii), Oebalus pugnax, Dichelops melacanthus, one-banded stingbug (Piezodorus hybneri) and the like;

from the family Cydnidae, Burrower brown bug (Scaptocoris castanea);

from the family Alydidae, for example, bean bug (Riptortus pedestris), corbett rice bug (Leptocorisa chinensis), rice bug (Leptocorisa acuta), and the like;

from the family Coreidae, Cletus punctiger, Australian leaf-footed bug (Leptoglossus australis), and the like;

from the family Lygaeidae, oriental chinch bug (Caverelius saccharivorus), seed bug (Togo hemipterus), chinch bug (Blissus leucopterus), and the like;

from the family Miridae, rice leaf bug (Trigonotylus caelestialium), sorghum plant bug (Stenotus rubrovittatus), wheat leaf bug (Stenodema calcarata), American tarnished plant bug (Lygus lineolaris), and the like;

from the family Aleyrodidae, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), Pealius euryae, and the like;

from the family Diaspididae, Abgrallaspis cyanophylli, red scale (Aonidiella aurantii), San Jose scale (Diaspidiotus perniciosus), white peach scale (Pseudaulacaspis pentagona), arrowhead scale (Unaspis yanonensis), citrus snow scale (Unaspis citri), and the like;

from the family Coccidae, pink wax scale (Ceroplastes rubens);

from the family Margarodidae, fluted scale (Icerya purchasi) seychelles fluted scale (Icerya seychellarum), and the like;

from the family Pseudococcidae, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), tuttle mealybug (Brevennia rehi), and the like;
from the family Psyllidae, citrus psylla (Diaphorina citri), two-spotted citrus psyllid (Trioza erytreae), pear sucker (Cacopsylla pyrisuga), Cacopsylla chinensis, potato psyllid (Bactericera cockerelli), Pear psylla (Cacopsylla pyricola), and the like;
from the family Tingidae, sycamore lace bug (Corythucha ciliata), aster tingid (Corythucha marmorata), Japanese pear lace bug (Stephanitis nashi), azalea lace bug (Stephanitis pyrioides), and the like;
from the family Cimicidae, common bed bug (Cimex lectularius), tropical bed bug (Cimex lectularius), and the like;
from the family Cicadidae, Quesada gigas, and the like;
from the family Reduviidae, Triatoma infestans, Triatoma rubrofasciata, Triatoma dimidiata, Rhodonius prolixus, and the like.

Lepidoptera:

from the family Crambidae, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Pediasia teterrellus), rice case-worm (Nymphula depunctalis), Sugarcane borer (Diatraea saccharalis), egg plant fruit borer (Leucinodes orbonalis), and the like;
from the family Pyralidae, lesser cornstalk borer (Elasmopalpus lignosellus), mealworm moth (Plodia interpunctella), persimmon bark borer (Euzophera batangensis), fig moth (Cadra cautella), and the like;
from the family Noctuidae, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), Spodoptera cosmioides, semitropical armyworm (Spodoptera eridania), black cutworm (Agrotis ipsilon), turnip moth (Agrotis segetum), beet worm (Autographa nigrisigna), rice looper (Plusia festucae), soybean looper (Chrysodeixis includens), Trichoplusia spp., Heliothis spp. (such as tobacco budworm (Heliothis virescens)), Helicoverpa spp. (such as tobacco budworm (Helicoverpa armigera), corn earworm (Helicoverpa zea)), Velvetbean caterpillar (Anticarsia gemmatalis), Cotton leafworm (Alabama argillacea), Hop vine borer (Hydraecia immanis), and the like;
from the family Pieridae, common cabbage worm (Pieris rapae), and the like;
from the family Tortricidae, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), Bean Shoot Borer (Epinotia aporema), Citrus fruit borer (Ecdytolopha aurantiana), European wine moth (Lobesia botrana) and the like;
from the family Gracillariidae, tea leaf roller (Caloptilia theivora), Asiatic apple leaf miner (Phyllonorycter ringoniella), and the like;
from the family Carposinidae, peach fruit moth (Carposina sasakii), and the like;
from the family Lyonetiidae, Coffee Leaf miner (Leucoptera coffeella), peach leaf miner (Lyonetia clerkella), Lyonetia prunifoliella, and the like;
from the family Lymantriidae, Lymantria spp. (such as gypsy moth (Lymantria dispar)), Euproctis spp. (such as tea lymantriid (Euproctis pseudoconspersa)), and the like;
from the family Plutellidae, diamondback moth (Plutella xylostella), and the like;
from the family Gelechiidae, peach worm (Anarsia lineatella), sweetpotato leaf folder (Helcystogramma triannulella), pink bollworm (Pectinophora gossypiella), potato moth (Phthorimaea operculella), tomato leaf miner Tuta absoluta), and the like;
from the family Arctiidae, American white moth (Hyphantria cunea), and the like;
from the family Castniidae, Giant Sugarcane borer (Telchin licus), and the like;
from the family Cossidae, Cossus insularis, and the like;
from the family Geometridae, Ascotis selenaria, and the like;
from the family Limacodidae, blue-striped nettle grub (Parasa lepida), and the like;
from the family Stathmopodidae, persimmon fruit moth (Stathmopoda masinissa), and the like;

from the family Sphingidae, tobacco hornworm (Acherontia lachesis), and the like;
from the family Sesiidae, Nokona feralis, cherry borer (Synanthedon hector), Synanthedon tenuis, and the like:

from the family Hesperiidae, rice skipper (Parnara guttata), and the like;
from the family Tineidae, casemaking clothes moth (Tinea translucens), common clothes moth (Tineola bisselliella), and the like.

Thysanoptera:

from the family Thripidae, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, avocado thrips (Scirtothrips perseae), and the like;
from the family Phlaeothripidae, aculeated rice thrips (Haplothrips aculeatus), and the like.

Diptera:

from the family Anthomyiidae, seedcorn maggot (Delia platura), onion maggot (Delia antiqua), beet leaf miner (Pegomya cunicularia), and the like;
from the family Ulidiidae, sugarbeet root maggot (Tetanops myopaeformis), and the like;
from the family Agromyzidae, rice leaf miner (Agromyza oryzae), tomato leaf miner (Liriomyza sativae), chrysanthemum leaf miner (Liriomyza trifolii), pea leafminer (Chromatomyia horticola), and the like;
from the family Chloropidae, rice stem maggot (Chlorops oryzae), and the like;
from the family Tephritidae, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), Japanese cherry fruit fly (Rhacochlaena japonica), and the like;
from the family Ephydridae, smaller rice leaf miner (Hydrellia griseola), whorl maggot (Hydrellia philippina), paddy stem maggot (Hydrellia sasakii), and the like;
from the family Drosophilidae, cherry drosophila (Drosophila suzukii), and fruit fly (Drosophila melanogaster), and the like;
from the family Phoridae, Megaselia spiracularis, and the like;
from the family Psychodidae, Clogmia albipunctata, and the like;
from the family Sciaridae, Bradysia difformis, and Bradysia odoriphaga, and the like;
from the family Cecidomyiidae, hessian fly (Mayetiola destructor), paddy gall fly (Orseolia oryzae), and the like;
from the family Diopsidae, Diopsis macrophthalma, and the like;
from the family Glossinidae, Glossina palpalis, and Glossina morsitans, and the like;
from the family Simuliidae, Simulium japonicum, and Simulium damnosum, and the like;
from the family Phlebotominae;
from the family Tipulidae, rice crane fly (Tipula aino), Common cranefly (Tipula oleracea), European cranefly (Tipula paludosa), and the like;
from the family Culicidae, southern house mosquito (Culex pipiens pallens), Culex tritaeniorhynchus ,Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, Anopheles minimus, and the like;
from the family Simulidae, Prosimulium yezoensis, Simulium ornatum, and the like;
from the family Tabanidae, Tabanus trigonus, and the like;
from the family Muscidae, house fly (Musca domestica), false stable fly (Muscina stabulans), biting house fly (Stomoxys calcitrans), buffalo fly (Haematobia irritans) , and the like;
from the family Calliphoridae;
from the family Sarcophagidae;
from the family Chironomidae, Chironomus plumosus, Chironomus yoshimatsui, Glyptotendipes tokunagai, and the like;
from the family Fannidae.

Coleoptera:

from the family Chrysomelidae, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), Cucurbit Beetle (Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), Cabbage flea beetle (Phyllotreta cruciferae), Western black flea beetle (Phyllotreta pusilla), Cabbage stem flea beetle (Psylliodes chrysocephala), hop flea-beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, tobacco flea beetle (Epitrix hirtipennis), radish Leaf beetle (Phaedon brassicae), motschulsky (Medythia nigrobilineata), and the like;

from the family Carabidae, Seedcorn beetle (Stenolophus lecontei), Slender seedcorn beetle (Clivina impressifrons), and the like;

from the family Scarabaeidae, cupreus chafer (Anomala cuprea), soybean beetle (Anomala rufocuprea), Anomala albopilosa, Japanese beetle (Popillia japonica), yellowish elongate chafer (Heptophylla picea), European Chafer (Rhizotrogus majalis), Tomarus gibbosus, Holotrichia spp., Phyllophaga spp. (such as June beetle (Phyllophaga crinita)), and Diloboderus spp. (such as Diloboderus abderus), and the like;

from the family Anthriibidae, coffee bean weevil (Araecerus coffeae), and the like;

from the family Aponidae, sweet-potato weevil (Cylas formicarius), and the like;

from the family Bruchidae, Mexican bean weevil (Zabrotes subfasciatus), and the like;

from the family Scolytidae, pine beetle (Tomicus piniperda), Coffee Berry Borer (Hypothenemus hampei), and the like;

from the family Curculionidae, coffee bean weevil (Araecerus coffeae), sweet-potato weevil (Cylas formicarius), West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineatocollis, boll weevil (Anthonomus grandis), nunting billbug (Sphenophorus venatus), Southern Corn Billbug(Sphenophorus callosus), Soybean stalk weevil(Sternechus subsignatus), Sugarcane weevil(Sphenophorus levis), rusty gourd-shaped weevil (Scepticus griseus), brown gourd-shaped weevil (Scepticus uniformis), Aracanthus spp. (such as Aracanthus mourei), cotton root borer (Eutinobothrus brasiliensis), and the like;

from the family Tenebrionidae, red meal beetle (Tribolium castaneum), mason beetle (Tribolium confusum), lesser mealworm (Alphitobius diaperinus), and the like;

from the family Coccinellidae, twenty-eight-spotted ladybird (Epilachna vigintioctopunctata), and the like;

from the family Bostrychidae, common powder-post beetle (Lyctus brunneus), lesser grain borer (Rhizopertha dominica), and the like;

from the family Ptinidae;

from the family Cerambycidae, citrus long-horned beetle (Anoplophora malasiaca), Migdolus fryanus, and the like;

from the family Elateridae, Melanotus okinawensis, barley wireworm (Agriotes fuscicollis), Melanotus legatus, Anchastus spp., Conoderus spp., Ctenicera spp., Limonius spp., Aeolus spp., and the like;

from the family Staphylinidae, Paederus fuscipes, and the like;

from the family Dermestidae, varied carpet beetle (Anthrenus verbasci), hide beetle (Dermestes maculates), khapra beetle (Trogoderma granarium), and the like;

from the family Anobidae, tobacco beetle (Lasioderma serricorne) and biscuit beetle (Stegobium paniceum), and the like;

from the family Laemophloeidae, flat grain beetle (Cryptolestes ferrugineus), and the like;

from the family Silvanidae, saw-toothed grain beetle (Oryzaephilus surinamensis), and the like.

from the family Nitidulidae, blossom beetle (Brassicogethes aeneus), and the like.

Orthoptera:

from the family Acrididae, oriental migratory locust (Locusta migratoria), Moroccan locust (Dociostaurus maroccanus), Australian plague locust (Chortoicetes terminifera), red locust (Nomadacris septemfasciata), Brown Locust (Locustana pardalina), Tree Locust (Anacridium melanorhodon), Italian Locust (Calliptamus italicus), Differential grasshopper (Melanoplus differentialis), Two striped grasshopper (Melanoplus bivittatus), Migratory grasshopper (Melanoplus sanguinipes), Red-Legged grasshopper (Melanoplus femurrubrum), Clearwinged grasshopper (Camnula pellucida), desert locust (Schistocerca gregaria), Yellow-winged locust(Gastrimargus musicus), Spur-throated locust (Austracris guttulosa) , Japanese grasshopper (Oxya yezoensis), rice grasshopper (Oxya japonica), Bombay locust (Patanga succincta), and the like;
from the family Gryllotalpidae, oriental mole cricket (Gryllotalpa orientalis), and the like;
from the family Gryllidae, house cricket (Acheta domestica), emma field cricket (Teleogryllus emma), and the like;
from the family Tettigoniidae, for example, Mormon cricket (Anabrus simplex), and the like.

Hymenoptera:

from the family Tenthredinidae, beet sawfly (Athalia rosae), nippon cabbage sawfly (Athalia japonica), and the like;
from the family Formicidae, Solenopsis spp. (such as red imported fire ant (Solenopsis invicta), tropical fire ant (Solenopsis geminata)), Atta spp. (such as Brown leaf-cutting ant (Atta capiguara)), Acromyrmex spp., Paraponera clavata, black house ant (Ochetellus glaber), little red ant (Monomorium pharaonis), Argentine ant (Linepithema humile), Formica fusca japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), long-legged ant (Anoplolepis gracilipes), and the like;
from the family Vespidae, Asian giant hornet (Vespa mandarinia japonica), Vespa simillima, Vespa analis Fabriciusi, Asian hornet (Vespa velutina), Polistes jokahamae, and the like;
from the family Siricidae, pine wood wasp (Urocerus gigas), and the like;
from the family Bethylidae.

Blattodea:

from the family Blattellidae, German cockroach (Blattella germanica), and the like;
from the family Blattidae, smoky-brown cockroach (Periplaneta fuliginosa), American cockroach (Periplaneta americana), Australian cockroach (Periplaneta australasiae), brown cockroach (Periplaneta brunnea), black cockroach (Blatta orientalis), and the like;
from the family Termitidae, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, Cornitermes cumulans, and the like.

Siphonaptera:

from the family Pulicidae, human flea (Pulex irritans), cat flea (Ctenocephalides felis), dog flea (Ctenocephalides canis), oriental rat flea (Xenopsylla cheopis), chicken flea (Echidnophaga gallinacea), and the like;
from the family Hectopsyllidae, chigoe flea (Tunga penetrans)), and the like;
from the family Ceratophyllidae, European rat flea (Nosopsyllus fasciatus), and the like.

Psocodae:

from the family Pediculidae, head louse (Pediculus humanus capitis), and the like;
from the family Pthiridae, crab louse (Pthirus pubis), and the like;
from the family Haematopinidae, short-nosed cattle louse (Haematopinus eurysternus), pig louse (Haematopinus suis), and the like;
from the family Linognathidae, blue cattle louse (Linognathus vituli), sheep face louse (Linognathus ovillus), capillate louse (Solenopotes capillatus), and the like;
from the family Bovicoliidae, cattle biting louse (Bovicola bovis), sheep louse (Bovicola ovis), Bovicola breviceps, Damalinia forficula, Werneckiella spp., and the like;
from the family Menoponidae, chicken louse (Menopon gallinae), chicken body louse (Menacanthus stramineus), Trinoton spp., and the like;
from the family Trimenoponidae, Cummingsia spp., and the like;
from the family Trogiidae, booklice (Trogium pulsatorium), and the like;
from the family Liposcelidae or Liposcelididae, Liposcelis corrodens, Liposcelis bostrychophila, Liposcelis pearmani, Liposcelis entomophila, and the like.

Thysanura:
from the family Lepismatidae, oriental silverfish (Ctenolepisma villosa), moth fish (Lepisma saccharina), and the like.

Acari:

from the family Tetranychidae, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), Oligonychus spp., and the like;
from the family Eriophyidae, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, Shevtchenkella sp., and the like;
from the family Tarsonemidae, broad mite (Polyphagotarsonemus latus), and the like;
from the family Tenuipalpidae, Brevipalpus phoenicis, and the like;
from the family Tuckerellidae;
from the family Ixodidae, ixodid tick (Haemaphysalis longicornis), Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), Dermacentor reticulatus, Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), bull tick (Rhipicephalus microplus), cattle tick (Rhipicephalus annulatus), brown dog tick (Rhipicephalus sanguineus), Rhipicephalus appendiculatus, Rhipicephalus decoloratus, and the like;
from the family Argasidae, fowl tick (Argas persicus), Ornithodoros hermsi, Ornithodoros turicata, and the like;
from the family Pyroglyphidae, American house dust mite (Dermatophagoides farinae), European house dust mite (Dermatophagoides pteronyssinus), and the like;
from the family Cheyletidae, Cheyletus eruditus, Cheyletus malaccensis, Chelacaropsis moorei, Cheyletiella yasguri, and the like;
from the family Psoroptidae, sheep scab mite (Psoroptes ovis), horse psoroptic mange mite (Psoroptes equi), Knemidocoptes mutans, ear mange mite (Otodectes cynotis), Chorioptes spp., and the like;
from the family Sarcoptidae, Notoedres cati, Notoedres muris, itch mite (Sarcoptes scabiei), and the like;
from the family Listrophoridae, Listrophorus gibbus, and the like;
from the family Dermanyssidae, bird mite (Dermanyssus gallinae), and the like;
from the family Macronyssidae, feather mite (Ornithonyssus sylviarum), tropical rat mite (Ornithonyssus bacoti), and the like;
from the family Varroidae, Varroa jacobsoni, and the like;
from the family Demodicidae, dog follicle mite (Demodex canis), cat follicle mite (Demodex cati), and he like;
from the family Trombiculidae, scrub typhus mite (Leptotrombidium akamushi), Leptotrombidium pallidum, Leptotrombidium scutellare), and the like.

Araneae:

from the family Eutichuridae, Cheiracanthium japonicum, and the like;
from the family Theridiidae, red-back spider (Latrodectus hasseltii), and the like.

Polydesmida:
from the family Paradoxosomatidae, flat-backed millipede (Oxidus gracilis), Nedyopus tambanus, and the like;

Isopoda:
from the family Armadillidiidae, common pill bug (Armadillidium vulgare), and the like;

Chilopoda:

from the family Scutigeridae, Thereuonema hilgendorfi, and the like;
from the family Scolopendridae, giant tropical centipede (Scolopendra subspinipes), and the like;
from the family Ethopolyidae, Bothropolys rugosus, and the like.

Nematoda:

from the family Aphelenchoididae, rice white-tip nematode (Aphelenchoides besseyi), and the like;
from the family Pratylenchidae, root lesion nematode (Pratylenchus coffeae), Pratylenchus brachyurus, California meadow nematode (Pratylenchus neglectus), Radopholus similis, and the like;
from the family Heteroderidae, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), white potato cyst nematode (Globodera pallida), Columbia root-knot nematode (Meloidogyne chitwoodi), and the like;
from the family Hoplolaimidae, Rotylenchulus reniformis, and the like;
from the family Anguinidae, strawberry bud nematode (Nothotylenchus acris), stem nematode (Ditylenchus dipsaci), and the like;
from the family Tylenchulidae, citrus nematode (Tylenchulus semipenetrans), and the like;
from the family Longidoridae, dagger nematode (Xiphinema index), and the like;
from the family Trichodoridae;
from the family Parasitaphelenchidae, pine wilt disease (Bursaphelenchus xylophilus), and the like;
from the family Trichuridae, Trichuris suis, Trichuris ovis, Trichuris discolor, and the like;
from the family Capillariidae, Capillaria suis, Calodium hepaticum, Paracapillaria philippinensis, Pearsonema plica, Pearsibena spp., and the like;
from the family Trichinellidae, Trichinella spiralis, Trichinella britovi, and the like;
from the family Strongyloididae, Strongyloides papillosus, Strongyloides planiceps, Strongyloides ransomi, Strongyloides stercoralis, and the like;
from the family Strongylidae, Strongylus edentatus, Strongylus vulgaris, Triodontophorus spp., Oesophagodontus spp., Cylicostephanus spp., and the like;
from the family Chabertiidae, Oesophagostomum dentatum, Oesophagostomum radiatum, Chabertia ovina, and the like;
from the family Stephanuridae, Stephanurus dentatus, and the like;
from the family Dioctophymatidae, Dioctophyme renale and the like;
from the family Ancylostomatidae, Ancylostoma braziliense, Ancylostoma caninum, Ancylostoma duodenale, Uncinaria stenocephala, Bunostomum phlebotomum, Bunostomum trigonocephalum, Globocephalus spp., and the like;
from the family Syngamidae, Syngamus skrjabinomorpha, Syngamus trachea, Cyathostoma bronchialis, and the like;
from the family Metastrongylidae, Metastrongylus apri, and the like;
from the family Dictyocaulidae, Dictyocaulus filaria), Dictyocaulus viviparus, and the like;
from the family Crenosomatidae, Crenosoma aerophila, Crenosoma vulpis, and the like;
from the family Angiostrongylidae, Angiostrongylus cantonensis, Angiostrongylus vasorum, Aelurostrongylus abstrusus, and the like;
from the family Filaroididae, Filaroides hirthi, Filaroides osleri, and the like;
from the family Trichostrongylidae, Trichostrongylus axei, Trichostrongylus colubriformis, Obeliscoides cuniculi, and the like;
from the family Haemonchidae, Haemonchus contortus, Hyostrongylus rubidus, Mecistocirrus digitatus, Ostertagia ostertagi, and the like;
from the family Molineidae, Nematodirus filicollis, Nematodirus spathiger, and the like;
from the family Oxyuridae, Enterobius vermicularis, Oxyuris equi, Passalurus ambiguus, and the like;
from the family Heterakidae, Heterakis gallinarum, and the like;
from the family Ascarididae, Ascaris lumbricoides, Ascaris suum, Parascaris equorum, and the like;
from the family Toxocaridae, Toxocara canis, Toxocara cati, Toxocara vitulorum, and the like;
from the family Anisakidae, Anisakis spp., and the like;
from the family Ascaridiidae, Ascaridia galli, and the like;
from the family Gnathostomatidae, Gnathostoma doloresi, and the like;
from the family Physalopteridae, Physaloptera felidis, Physaloptera rara, and the like;
from the family Thelaziidae, Thelazia callipaeda, Thelazia gulosa, and the like;
from the family Gongylonematidae, Gongylonema pulchrum, and the like;
from the family Habronematidae, Habronema majus, Habronema muscae, Draschia megastoma, and the like;
from the family Dracunculidae, Dracunculus medinensis, and the like;
from the family Spirocercidae, Ascarops strongylina, and the like;
from the family Filariidae, Stephanofilaria okinawaensis, Parafilaria multipapillosa, Parafilaria bovicola, and the like;

from the family Setariidae, Setaria digitata, Setaria equina, and the like;
from the family Onchocercidae, Dirofilaria immitis, Brugia malayi, Onchocerca cervicalis, Onchocerca gibsoni, Dipetalonema reconditum, Wuchereria bancrofti, and the like.

As a target, the harmful arthropods such as harmful insects and harmful mites, and the harmful nematodes may be the harmful arthropods such as harmful insects and harmful mites, and the harmful nematodes, each of which have a reduced agent-sensitivity to or a developed agent-resistance to an insecticide, a miticide, or a nematocide, respectively.

The method for controlling a harmful arthropod or a harmful nematode of the present invention is conducted by applying an effective amount of the Present compound, the compound of the present invention, the composition A or the composition B to a harmful arthropod or a harmful nematode directly and/or a habitat where the harmful pest lives (for example, plant, soil, an interior of a house, and animal). Examples of a method for controlling a harmful arthropod or a harmful nematode of the present invention include foliar application, soil application, root application, shower application, smoking application, water-surface application, and seed application.

The Present compound, the compound of the present invention, the composition A, or the composition B is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the composition A, or the composition B may be used by formulating it into a dosage form described in Manual on development and use of FAO and WHO Specifications for pesticides, FAO Plant Production and Protection Papers-271-276, prepared by the FAO/WHO Joint Meeting on Pesticide Specifications, 2016, ISSN:0259-2517.

These formulations usually comprise 0.0001 to 99% by weight ratio of the Present compound, the compound of the present invention, the composition A, or the composition B.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, as an ingredient for increasing or assisting a potency which inherent in the present compound and the compound of the present invention, an adjuvant can be used. The specific examples of the surfactant include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability

even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method of controlling a harmful arthropod or a harmful nematode by applying an effective amount of the present compound, the compound of the present invention, the composition A, or the composition B to soil include a method of applying an effective amount of the present compound, the compound of the present invention, the composition A, or the composition B to soil before planting plants or after planting plants, a method of applying an effective amount of the present compound, the compound of the present invention, the composition A, or the composition B to a root part of a crop to be protected from damage such as ingestion by a harmful arthropod or a harmful nematode, and a method of controlling a harmful arthropod or a harmful nematode which ingest plants by permeating and transferring an effective amount of the present compound, the compound of the present invention, the composition A, or the composition B from roots and the like into the interior of the plant. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the present compound, the compound of the present invention, the composition A or the composition B to seeds or vegetative reproductive organs, and specific examples thereof include spraying treatment in which a suspension of the present compound, the compound of the present invention, the composition A or the composition B is sprayed onto seed surface or the vegetative reproductive organ surface in the form of mist; smearing treatment in which the present compound, the compound of the present invention, the composition A or the composition B is coated a surface of seeds or the vegetative reproductive organ; a soaking treatment in which the seeds are soaked into the solution of the present compound, the compound of the present invention, the composition A or the composition B for a certain time; and a method for coating the seeds or the vegetative reproductive organ with a carrier containing the present compound, the compound of the present invention, the composition A or the composition B (film coating treatment, pellet coating treatment). Examples of the above-described vegetative reproductive organ include particularly seed potato.

When the composition A or the composition B is applied to seeds or vegetative reproductive organs, the composition A or the composition B may be also applied to seeds or vegetative reproductive organs as a single formulation, or the composition A or the composition B may be applied to seeds or vegetative reproductive organs as a divided plural of formulations by a plurality of times. Examples of the method in which the composition A or the composition B is applied as a divided plural of formulations by a plurality of times include, for exmaple, a method in which the formulations comprising as an active component the present compound or the compound of the present invention only are applied, and seeds or vegetative reproductive organs are air dried, followed by applying the formulations comprising the present ingredient: and a method in which the formulations comprising as an active component the present compound or the compound of the present invention and the present ingredients are applied, and seeds or vegetative reproductive organs are air dried, followed by applying the formulations comprising the present ingredients other than the already-applied present ingredients, are included.

As used herein, seeds or vegetative reproductive organs carrying the present compound, the compound of the

present invention, the composition A or the composition B means seeds or vegetative reproductive organs in the state where the present compound, the compound of the present invention, the composition A or the composition B is adhered to a surface of the seeds or the vegetative reproductive organ. The above-described seeds or vegetative reproductive organs carrying the present compound, the compound of the present invention, the composition A or the composition B may be adhered by any other materials that are different from the present compound X or the composition A before or after being adhered the present compound, the compound of the present invention, the composition A or the composition B to the seeds or vegetative reproductive organs.

Also, when the composition A or the composition B is adhered in a form of layer (s) to a surface of seeds or vegetative reproductive organ, the layer(s) is/are composed of one layer or a plural of layers. Also, when a plural layers are formed, each of the layer may be composed of a layer comprising one or more active ingredients, or a combination of a layer comprising one or more active ingredients and a layer not comprising an active ingredient.

Seeds or vegetative reproductive organs carrying the present compound, the compound of the present invention, the composition A or the composition B can be obtained, for example, by applying the formulations comprising the present compound, the compound of the present invention, the composition A or the composition B by the above-described application method to seeds to seeds or vegetative reproductive organs.

When the present compound, the compound of the present invention, the composition A, or the composition B is applied for controlling a harmful arthropod or a harmful nematode in agricultural fields, the application dose thereof is usually within a range of 1 to 10,000g g of the present compound or the compound of the present invention per 10,000 $m^2$. In the case of being applied to seeds or vegetative reproductive organs, the dose of application dose thereof is usually within a range of 0.001 to 100 g of the present compound or the compound of the present invention per 1 Kg of seeds. When the present compound, the compound of the present invention, the composition A, or the composition B is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the present compound, the compound of the present invention, the composition A, or the composition B can be applied as a resin preparation which is processed into a sheet or a string by winding a plant with the sheet or the string of the resin preparation, putting a string of the resin preparation around a crop so that the plant is surrounded by the string, or laying a sheet of the resin preparation on the soil surface near the root of a plant.

When the present compound, the compound of the present invention, the composition A, or the composition B is used to control a harmful arthropod or a harmful nematode that live inside a house, the application dose as an amount of the present compound or the compound of the present invention is usually within a range from 0.01 to 1,000 mg per 1 $m^2$ of an area to be treated, in the case of using it on a planar area. In the case of using it spatially, the application dose as an amount of the present compound or the compound of the present invention is usually within a range from 0.01 to 500 mg per 1 $m^3$ of the space to be treated. When the present compound, the compound of the present invention, the composition A, or the composition B is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the present compound, the compound of the present invention, the composition A, or the composition B is used for controlling a harmful arthropod or a harmful nematode that parasitize small animals such as livestock (such as cows, horses, pigs, sheep, goats and chickens), dogs, cats, rats, and mice, they may be applied to the animals by a known method in the veterinary field. Specific examples of the use method include a method administered orally by mixing them as a tablet, a capsule, a chewable tablet or a feed, etc., a method administered as a suppository and by injection (including intramuscular, subcutaneous, intravenous, intraperitoneal, etc. injections), a method by spraying, applying or drop treating of an oil, an aqueous solution, a spot-on agent, a pour-on agent, a shampoo agent, a lotion agent, a paste agent, etc., or a method by processing the resin formulations into a collar, an ear tag, a bracelet, and a clothing and wearing them to the animal. In the case of being administered to an animal body, the dose of the compound of the present compound or the compound of the present invention is usually within a range from 0.1 to 1,000 mg per 1 kg of an animal body weight.

Also, the Present compound, the compound of the present invention, the composition A or the composition B may be used as an agent for controlling a harmful arthropod or a harmful nematode in agricultural lands such as paddy fields, fields, turfs, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest

soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

EXAMPLES

Hereinafter, the present invention is explained in more detail by using Preparation Examples, Formulation Examples, and Test Examples, etc., however, the present invention should not be limited to these examples.

When a physical property value of a compound is measured by a liquid chromatography / mass spectrometry (hereinafter, referred to as "LCMS"), the measured molecular ion value $[M + H]^+$ or $[M - H]^-$ and a retention time (hereinafter, referred to as "RT") are described. The condition of the liquid chromatography (hereinafter, referred to as "LC") and mass spectrometry (hereinafter, referred to as "MS") are described below.

[LC condition]

Column: L-column2 CDS, inner diameter 4.6 mm, length 30 mm, particle size 3 um (Chemicals Evaluation and Research Institute, Japan)
UV measurement wavelength: 254 nm
mobile phase: A solution: 0.1 % aqueous solution of formic acid, B solution: 0.1 % acetonitrile solution of formic acid,
Flow rates: 2.0 mL/min.
Pump: LC-20 AD (manufactured by Shimadzu Corporation) two machines (high pressure gradient)
Gradient condition: a solution for elution was transferred at a concentration gradient indicated in [Table LC1] .

[Table LC1]

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

[MS condition]

Detector: LCMS-2020 (manufactured by Shimadzu Corporation) Ionization Methods: DUIS

Preparation Example 1

The present compounds A-1 to A-4 can be obtained according to the method described in Heterocyclic Communications (1995), 1(2-3), 207, etc. The identification of the obtained compound can be conducted by a measurement of $^{1}$H-NMR spectra or a measurement of $^{13}$C-NMR spectra, etc.

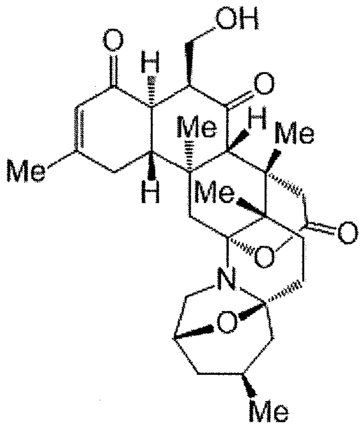
A-1

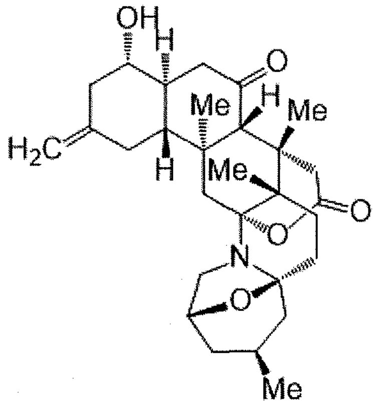
A-2

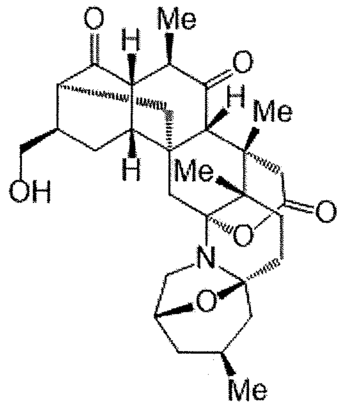
A-3

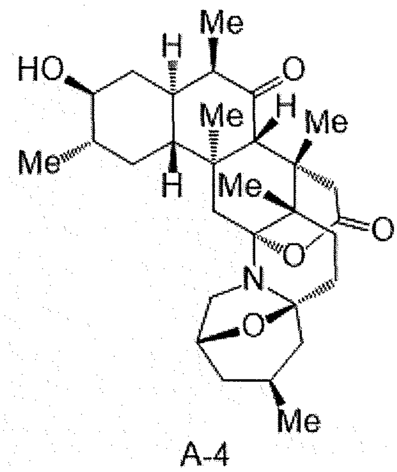
A-4

Preparation Example 2

The present compounds C-1 to C-3 can be obtained according to the method described in Journal of Organic Chemistry (1985), 50(20), 3757; or Heterocycles (1989), 28(1), 103, etc. The identification of the obtained compounds can be conducted by a measurement of $^{1}$H-NMR spectra or a measurement of $^{13}$C-NMR spectra, etc.

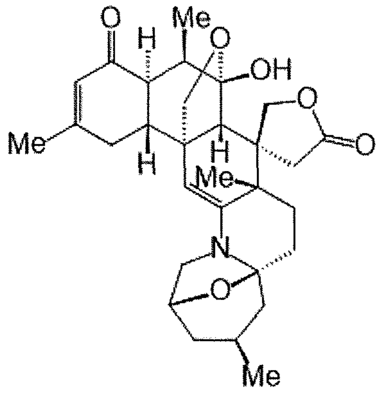

C-1

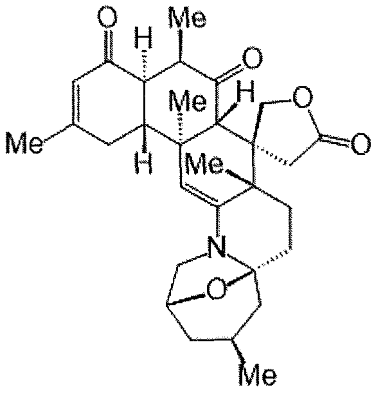

C-2

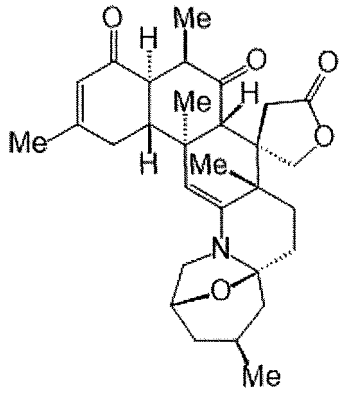

C-3

The physical property value of the compounds obtained by the above-mentioned method is shown below. Present compound C-1: $^{1}$H-NMR ($CDCl_3$) δ: 0.92(3H, d, J=6.4Hz), 1.06 (3H, d, J=6.9Hz), 1.16 (1H, d, J=13Hz), 1.25 (3H, d, J=7.0Hz), 1.47-1.75 (5H, m), 1.87 (1H, dd, J=13Hz, 4.7Hz), 1.91-1.96 (2H, m), 1.98 (3H, s), 2.03-2.10 (2H, m), 2.26 (1H, s), 2.41 (1H, d, J=18Hz), 2.33-2.42 (2H, m), 2.59 (1H, dd, J=13Hz, 5.4Hz), 3.00 (1H, br s), 3.14 (1H, d, J=6.6Hz), 3.15 (1H, br s), 3.52 (1H, d, J=9.0Hz), 3.57 (1H, d, J=18Hz), 3.84 (1H, s), 3.95 (1H, d, J=9.0Hz), 4.22 (1H, d, J=9.3Hz), 4.62 (1H, br s), 4.92 (1H, d, J=9.3Hz) , 5.88 (1H, s) .

Preparation Example 3

The present compounds A-5 to A-12 and B-1 can be obtained according to the method described in Tetrahedron (1998), 54(27), 7891; Tetrahedron (1999), 55(17), 5539; Marine Drugs (2014), 12(10), 5188; Angewandte Chemie (International ed. in English) (2009), 48(47), 8905-8, Chemistry - An Asian Journal (2011), 6(3), 922-931, etc. The identification of the obtained compounds can be conducted by a measurement of $^{1}$H-NMR spectra or a measurement of $^{13}$C-NMR spectra, etc.

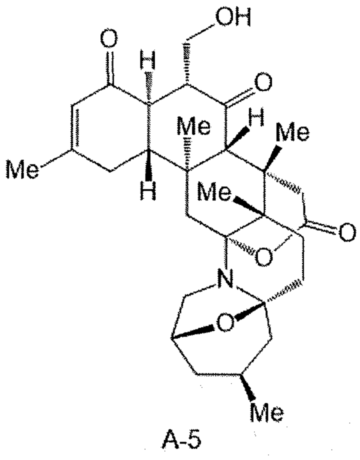

A-5

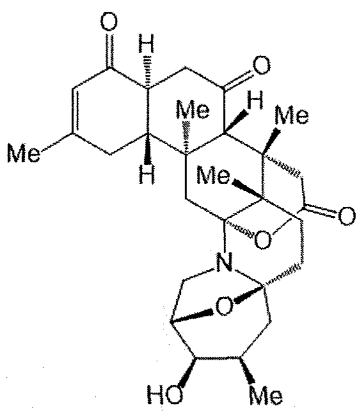

A-6

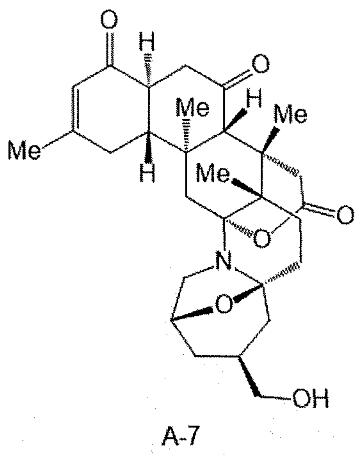

A-7

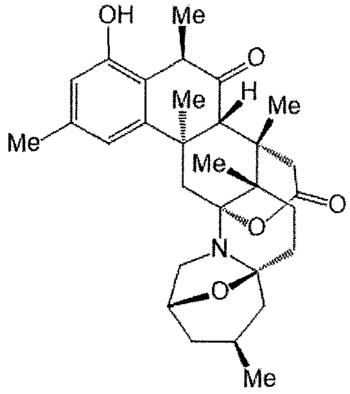

B-1

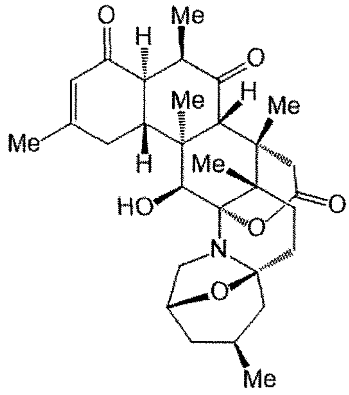

A-8

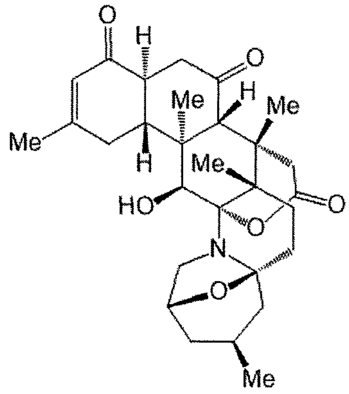

A-9

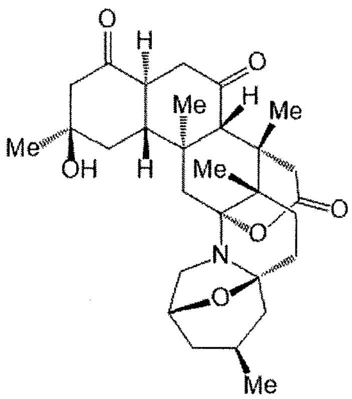

A-10

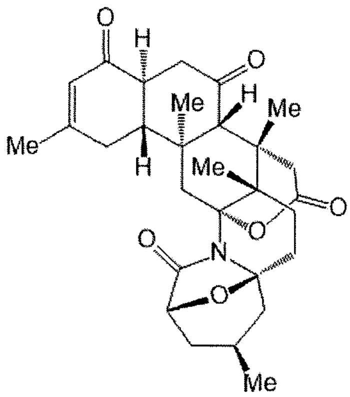

A-11

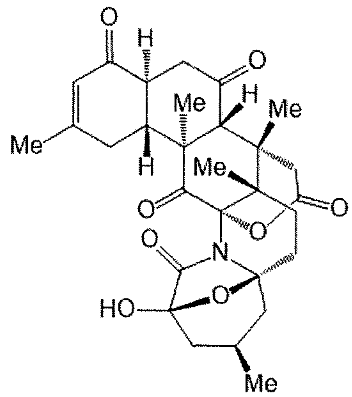

A-12

The physical property value of the compounds obtained by the above-mentioned method is shown below. Present compound B-1: $^1$H-NMR ($CDCl_3$) $\delta$: 0.93 (3H, d, J=6.5Hz), 1.07 (3H, s), 1.11 (3H, s), 1.12-1.15 (1H, m), 1.17 (3H, s), 1.54 (3H, d, J=7.0Hz), 1.46-1.55 (3H, m), 1.72-1.82 (2H, m), 1.91 (1H, dd, J=14Hz, 4.5Hz), 2.15 (1H, dd, J=13Hz, 5.0Hz), 2.30 (3H, s), 2.31-2.37 (1H, m), 2.44 (1H, dd, J=20Hz, 1.0Hz), 2.50 (1H, d, J=14Hz), 2.65 (1H, d, J=14Hz), 3.37 (1H, t, J=6.4Hz), 3.42 (1H, d, J=6.4Hz), 3.55 (1H, q, J=6.9Hz), 3.56 (1H, s), 3.91 (1H, d, J=20Hz), 4.57-4.61 (1H, m), 4.89 (1H, br s), 6.48 (1H, s), 6.55 (1H, s).

Preparation Example 4

The present compounds C-4 to C-28 and D-1 can be obtained according to the method described in Tetrahedron Letters (2014), 55(39), 5369; Tetrahedron (2015), 71(45), 8601; Journal of Natural Products (2019), 82(10), 2790; Journal of Natural Products (2016), 79(10), 2674, etc. The identification of the obtained compounds can be conducted by a measurement of $^1$H-NMR spectra or a measurement of $^{13}$C-NMR spectra, etc.

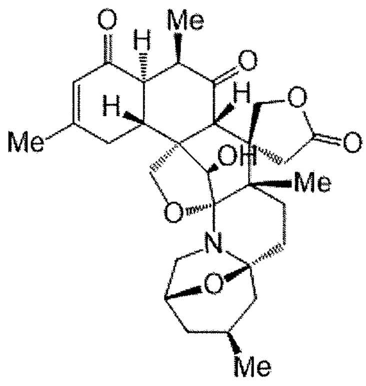

C-4

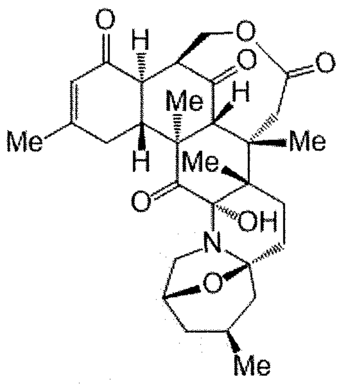

D-1

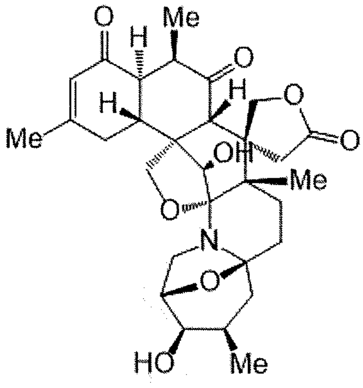

C-5

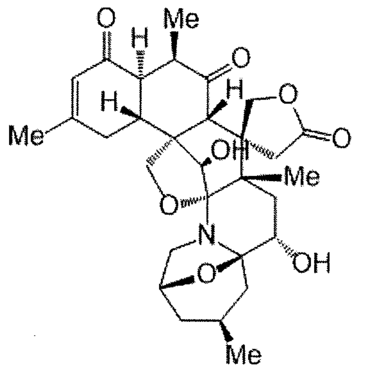

C-6

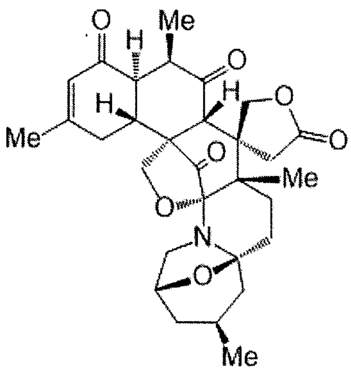

C-7

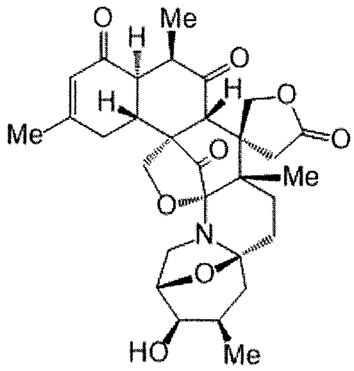

C-8

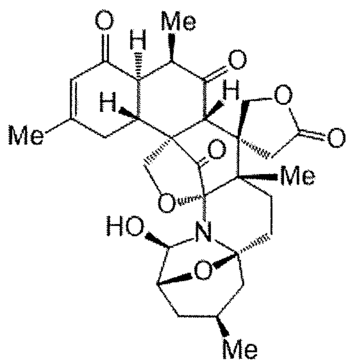

C-9

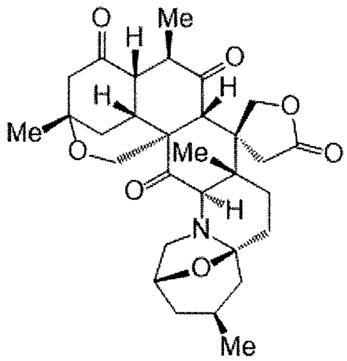

C-10

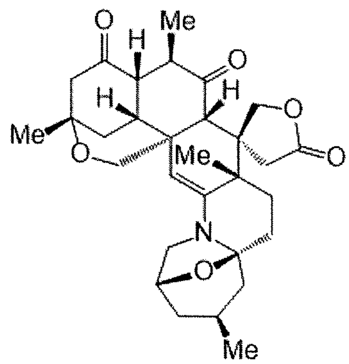

C-11

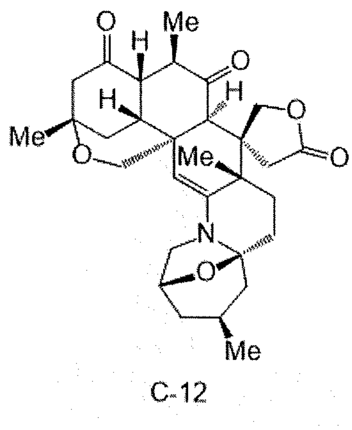

C-12

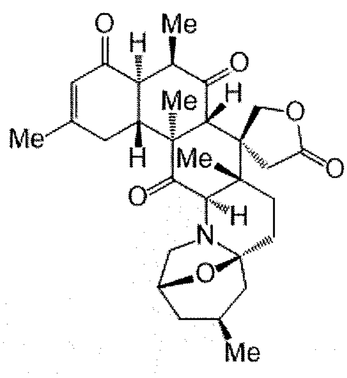

C-13

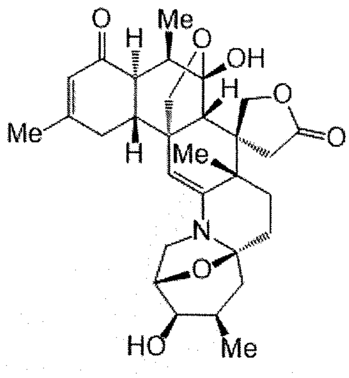

C-14

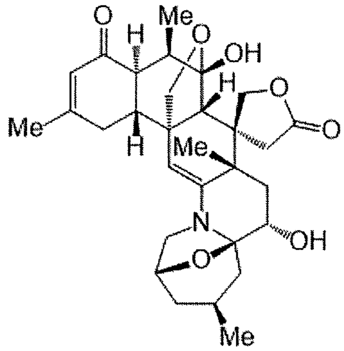
C-15

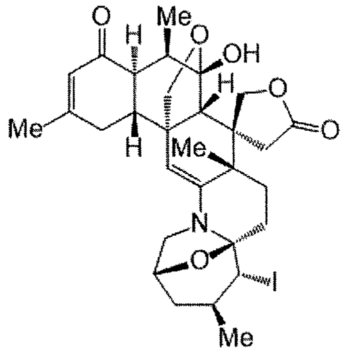
C-16

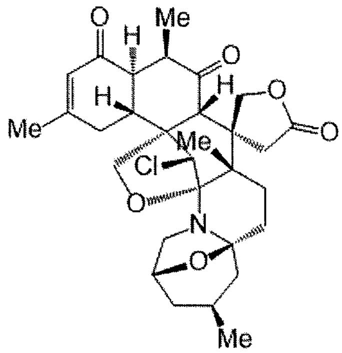
C-17

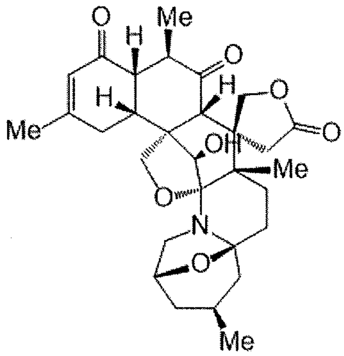
C-18

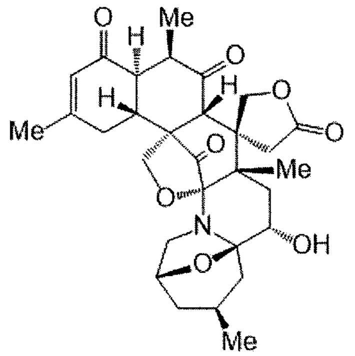
C-19

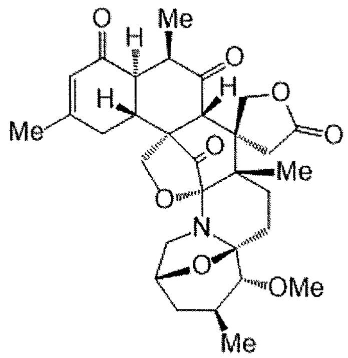
C-20

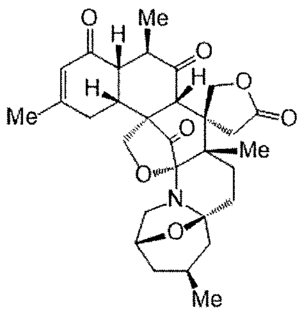
C-21

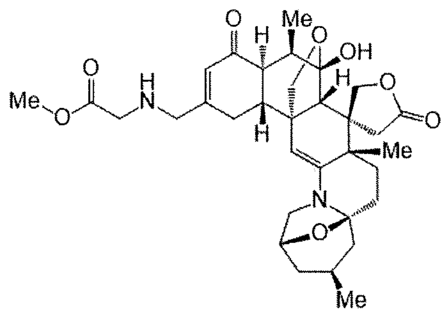
C-22

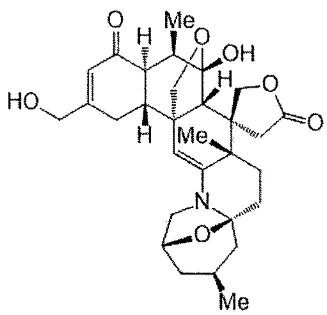
C-23

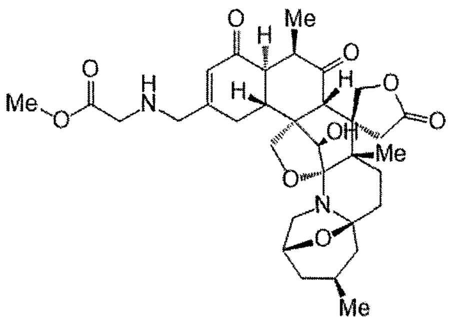
C-24

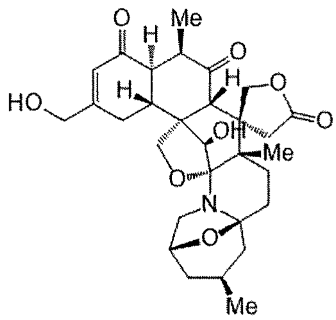
C-25

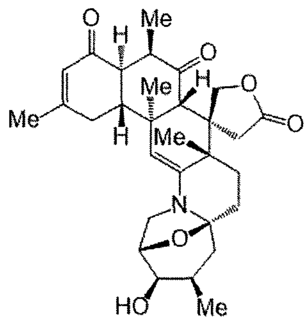
C-26

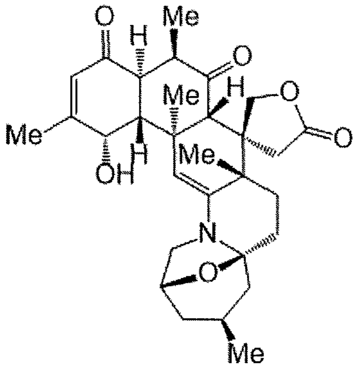

C-27

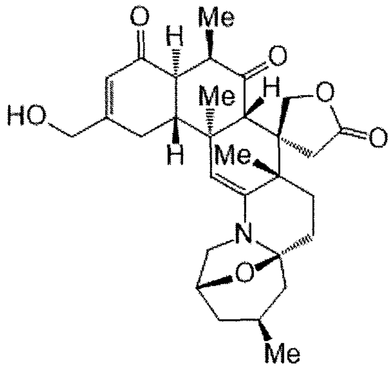

C-28

The physical property values of the compounds obtained by the above-mentioned method are shown below.

Present compound C-4: $^1H$-NMR ($C_5D_5N$) $\delta$: 0.86(3H, d, J=6.4Hz), 1.09-1.19 (2H, m), 1.22 (3H, d, J=7.3Hz), 1.43 (2H, dd, J=8.6Hz, 2.4Hz), 1.47 (3H, s), 1.63 (3H, s), 1.68-1.72 (1H, m), 1.91-1.99 (1H, m), 2.25-2.35 (3H, m), 2.42-2.49 (1H, m), 2.54 (1H, dd, J=14Hz, 4.6Hz), 2.58 (1H, d, J=4.1Hz), 3.05-3.12 (1H, m), 3.30(1H, d, J=6.6Hz), 3.49 (1H, dq, J=7.3Hz, 4.6Hz), 3.60 (1H, t, J=6.6Hz), 3.64 (2H, s), 3.71 (1H, s), 3.93 (1H, d, J=8.7Hz), 4.14 (1H, d, J=8.7Hz), 4.35 (1H, d, J=9.7Hz), 4.47 (1H, br s), 4.56 (1H, d, J=9.7Hz), 4.62-4.63 (1H, m), 5.99 (1H, s).

Present compound C-7: $^1H$-NMR ($C_5D_5N$) $\delta$: 0.85(3H, d, J=6.2Hz), 1.06 (3H, s), 1.08-1.15 (1H, m), 1.24 (3H, d, J=7.3Hz), 1.37-1.42 (3H, m), 1.70 (1H, dt, J=12Hz, 3.6Hz), 1.81 (1H, td, J=13Hz, 3.6Hz), 1.87 (3H, s), 2.20-2.23 (2H, m), 2.32 (1H, td, J=13Hz, 3.7Hz), 2.52 (1H, dd, J=17Hz, 12Hz), 2.66 (1H, dd, J=15Hz, 4.8Hz), 2.71 (1H, dd, J=17Hz, 4.2Hz), 2.73 (1H, d, J=6.5Hz), 3.27 (1H, ddd, J=16Hz, 12Hz, 4.2Hz), 3.47 (1H, dq, J=7.3Hz, 4.8Hz), 3.63 (1H, s), 3.73 (1H, t, J=6.5Hz), 3.81 (2H, s), 4.26 (1H, d, J=9.8Hz), 4.42 (1H, d, J=9.8Hz), 4.49 (1H, br s), 4.51 (1H, d, J=9.4Hz) , 4.62 (1H, d, J=9.4Hz) , 6.01 (1H, s).

Preparation Example 5

The present compounds A-13 and A-14 can be obtained according to the method described in Marine Drugs (2018), 16(7), 242, etc. The identification of the obtained compounds can be conducted by a measurement of $^1H$-NMR spectra or a measurement of $^{13}C$-NMR spectra, etc.

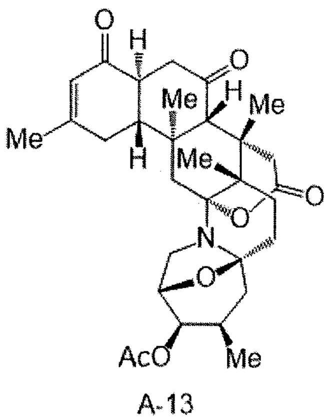

A-13

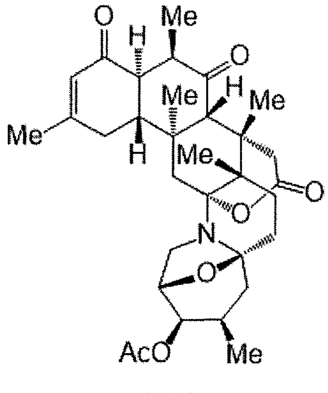

A-14

Preparation Example 6

The present compound A-15 can be obtained according to the method descried in Tetrahedron Letters (2008), 49(14), 2189, etc. The identification of the obtained compound can be conducted by a measurement of $^1H$-NMR spectra or a measurement of $^{13}C$-NMR spectra, etc.

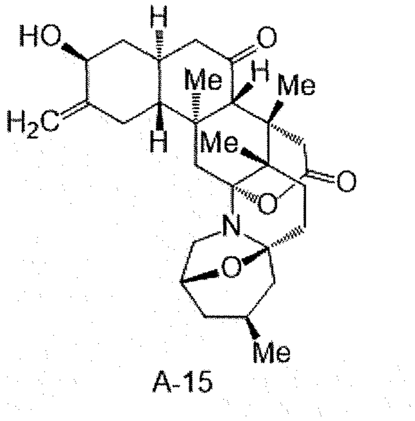

A-15

Preparation Example 7

The present compounds C-29 to C-35 and D-2 can be obtained according to the method described in J. Org. Chem. 2020, 85, 12553, etc. The identification of the obtained compounds can be conducted by a measurement of $^{1}$H-NMR spectra or a measurement of $^{13}$C-NMR spectra, etc.

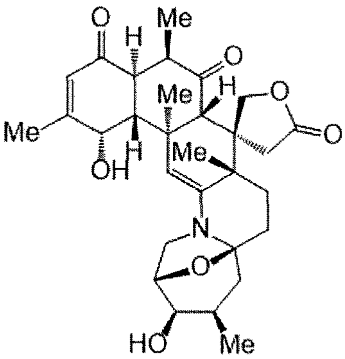

C-29

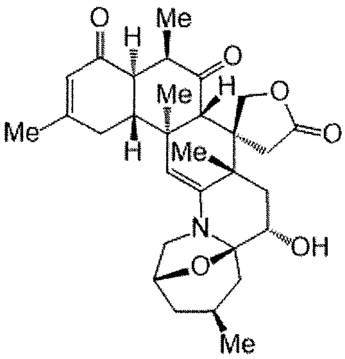

C-30

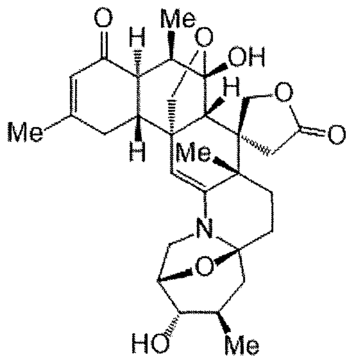

C-31

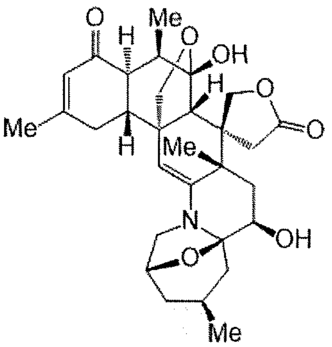

C-32

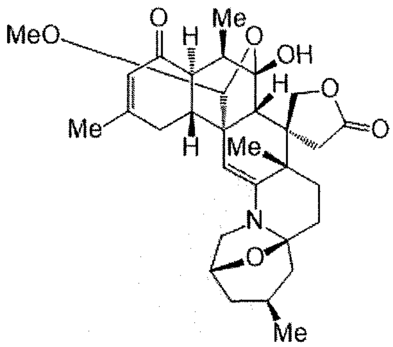

C-33

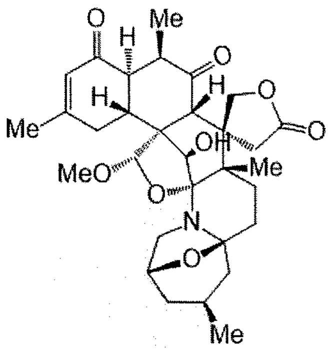

C-34

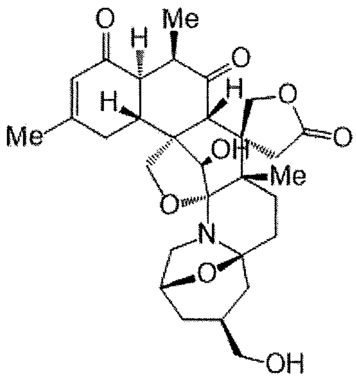

C-35

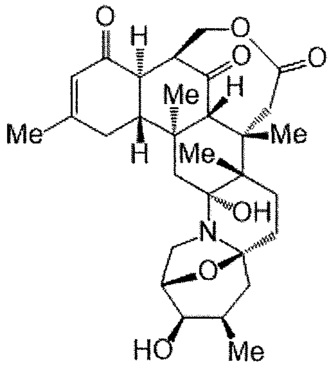

D-2

Preparation Example 8

The present compounds C-36 to C-44, A-16 and A-17 can be obtained according to the method described in Bioorganic Chemistry 109 (2021) 104700, etc. The identification of the obtained compounds can be conducted by a measurement of $^{1}$H-NMR spectra or a measurement of $^{13}$C-NMR spectra, etc.

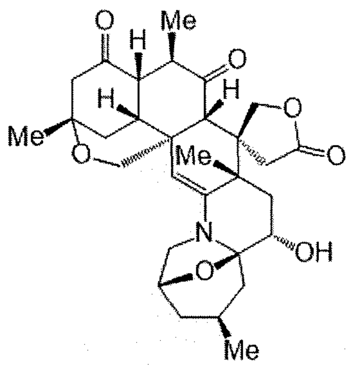

C-36

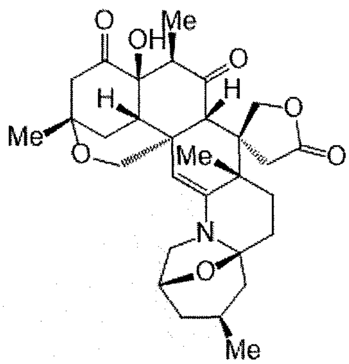

C-37

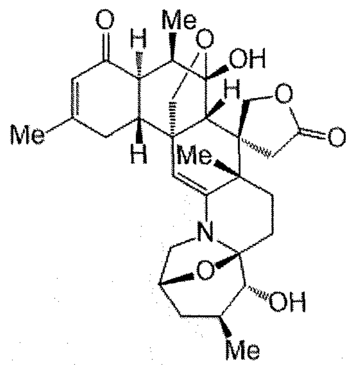

C-38

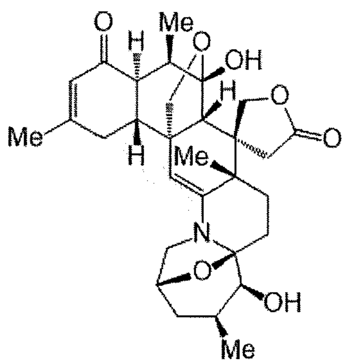

C-39

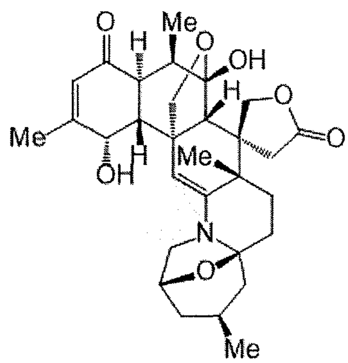

C-40

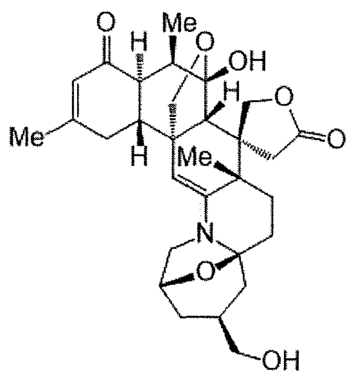

C-41

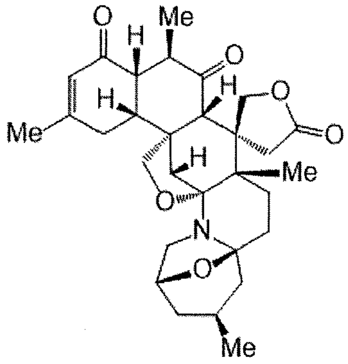

C-42

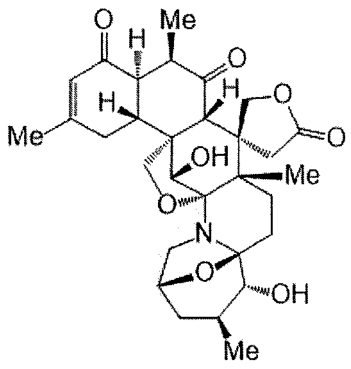

C-43

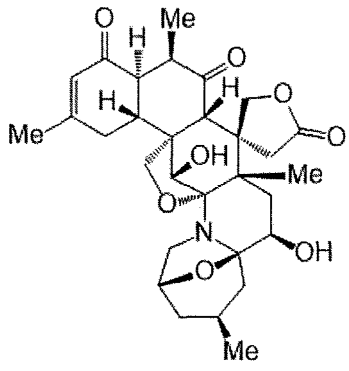

C-44

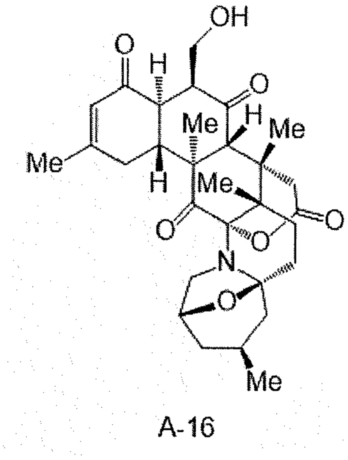

A-16

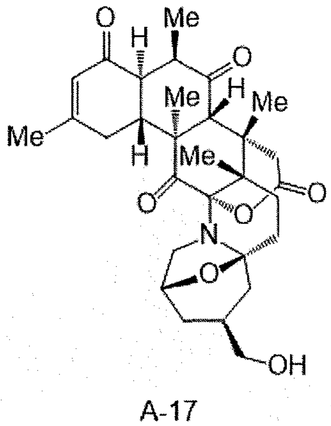

A-17

Preparation Example 9

The present compounds E-1, A-18, G-1, G-2, and A-20 can be obtained according to the method described in Bulletin of the Chemical Society of Japan (1998), 71(4), 771-779, etc. The identification of the obtained compounds can be conducted by a measurement of $^{1}H$-NMR spectra or a measurement of $^{13}C$-NMR spectra, etc.

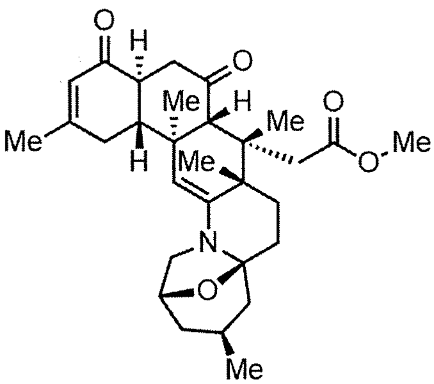

E-1

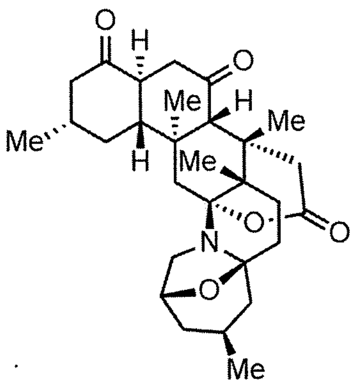

A-18

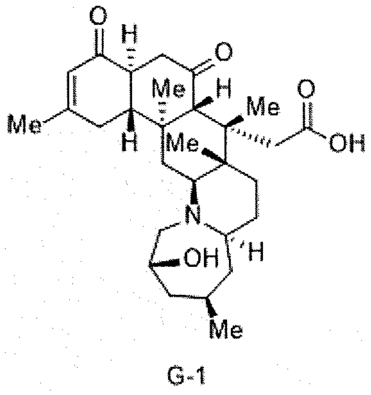

G-1

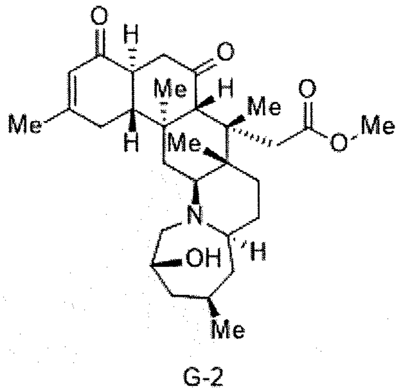

G-2

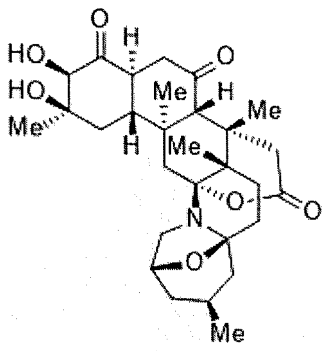

A-20

The physical property values of the compounds obtained by the above-mentioned method are shown below.

Present compound E-1: $^1H$-NMR ($CDCl_3$) δ: 0.92 (3H, d, J=6.5Hz), 1.00 (3H, s), 1.09-1.17 (1H, m), 1.14 (3H, s), 1.23 (3H, s), 1.50 (1H, dd, J=12Hz, 9.0Hz), 1.76-2.01 (6H, m), 2.00 (3H, s), 2.16 (1H, dd, J=11Hz, 4.1Hz), 2.26-2.35 (1H, m), 2.43 (1H, dd, J=18Hz, 4.1Hz), 2.52 (1H, d, J=15Hz), 2.51-2.67 (3H, m), 2.89 (1H, s), 3.13 (1H, dd, J=7.4Hz, 6.6Hz), 3.23 (1H, d, J=15Hz), 3.22-3.29 (1H, m), 3.68 (3H, s), 3.72 (1H, dd, J=7.4Hz, 7.0Hz) 4.10 (1H, s), 4.60-4.62 (1H, m), 5.90 (1H, s).

Present compound A-18: $^1H$-NMR ($CDCl_3$) δ:0.91 (3H, d, J=6.5Hz), 0.99 (3H, s), 0.99 (3H, s), 1.05-1.09 (1H, m), 1.11 (3H, d, J=6.1Hz), 1.15 (3H, s), 1.24-1.30 (2H, m), 1.39-1.50 (2H, m), 1.64-1.81 (3H, m), 1.84-1.93 (3H, m), 1.87 (1H, d, J=14Hz), 2.06 (1H, dd, J=13Hz, 13Hz), 2.09 (1H, dd, J=13Hz, 5.0Hz), 2.18 (1H, d, J=14Hz), 2.24-2.35 (2H, m), 2.35 (1H, d, J=20Hz), 2.48 (1H, dd, J=13Hz, 3.9Hz), 2.66-2.78 (2H, m), 2.81 (1H, s), 3.23 (1H, dd, J=6.8Hz, 6.1Hz), 3.27 (1H, d, J=6.1Hz) , 3.62 (1H, d, J=20Hz), 4.54-4.56 (1H, m).

Preparation Example 10

The present compounds A-19, F-4 and F-5 can be obtained according to the method described in Heterocyclic Communications (1998), 4(6), 575-580, etc.
The identification of the obtained compounds can be conducted by a measurement of $^1H$-NMR spectra, a measurement of $^{13}C$-NMR spectra, or a measurement of mass spectra, etc.

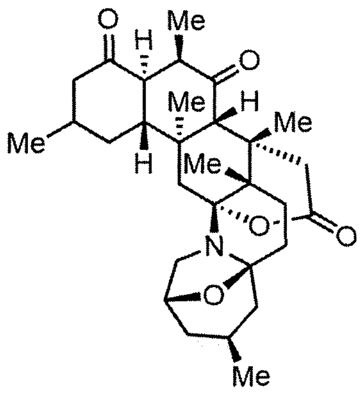

A-19

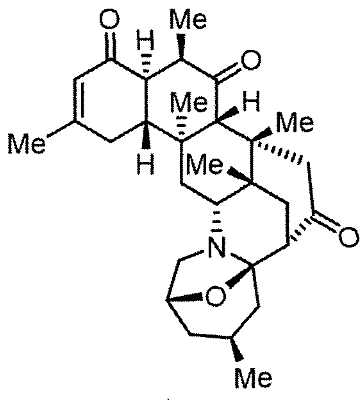

F-4

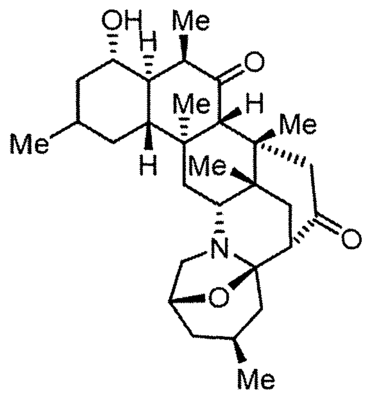

F-5

The physical property value of the compound obtained by the above-mentioned method is shown below.
Present compound A-19: LCMS: 498 $[M+H]^+$, RT = 1.25 min.

Preparation Example 11

The present compounds B-2 and A-23 can be obtained according to the method described in Angewandte Chemie (International ed. in English) (2009), 48(47), 8905-8, or Chemistry - An Asian Journal (2011), 6(3), 922-931, etc.
The identification of the obtained compounds can be conducted by a measurement of $^1H$-NMR spectra or a measurement of $^{13}C$-NMR spectra, etc.

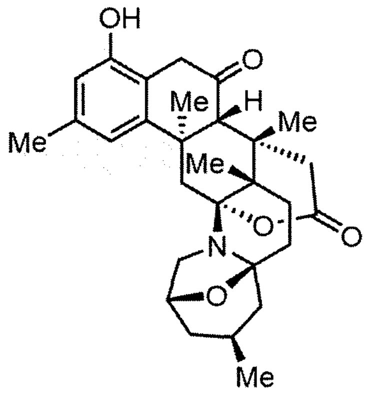

B-2

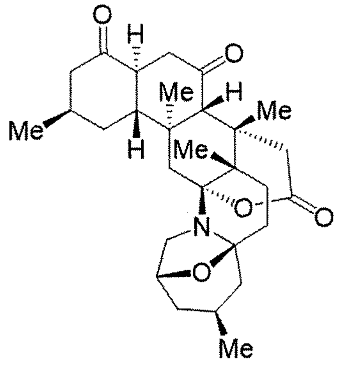

A-23

The physical property value of the compounds obtained by the above-mentioned method is shown below. Present compound B-2: $^1$H-NMR ($CDCl_3$) $\delta$: 0.93 (3H, d, J=6.5Hz), 0.98-1.04 (1H, m), 1.10(3H, s), 1.11 (3H, s), 1.17 (3H, s), 1.48 (1H, dd, J=14Hz, 2.4Hz), 1.59-1.62 (2H, m), 1.74 (1H, dd, J=13Hz, 3.8Hz), 1.79 (1H, dd, J=10Hz, 2.4Hz), 1.89 (1H, dd, J=13Hz, 4.8Hz), 2.14 (1H, dd, J=13Hz, 4.7Hz), 2.32 (3H, s), 2.34-2.40 (1H, m), 2.46 (1H, dd, J=20Hz, 1.1Hz), 2.53 (1H, d, J=14Hz), 2.61 (1H, d, J=14Hz), 3.22 (1H, s), 3.38 (1H, d, J=6.5Hz), 3.38 (1H, d, J=21Hz), 3.42 (1H, d, J=6.5Hz), 3.64 (1H, d, J=21Hz), 3.76 (1H, d, J=20Hz), 4.58-4.61 (1H, m), 4.87 (1H, br s), 6.51 (1H, s), 6.59 (1H, s)

Preparation Example 12

The present compounds F-1 to F-3 can be obtained according to the method described in Tetrahedron Letters (1997), 38(32), 5683-5686, or Bulletin of the Chemical Society of Japan (1998), 71(4), 771-779, etc. The identification of the obtained compounds can be conducted by a measurement of $^1$H-NMR spectra or a measurement of $^{13}$C-NMR spectra, etc.

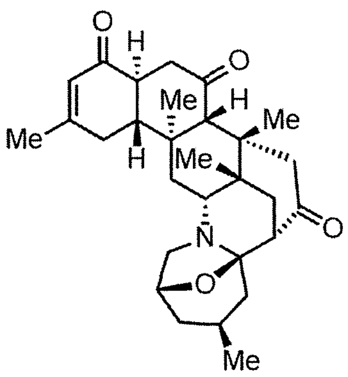

F-1

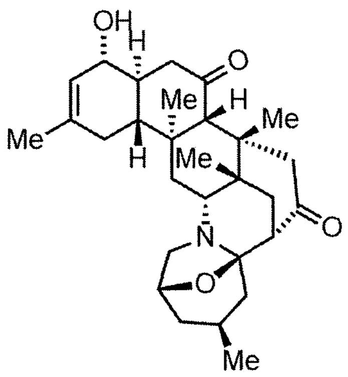

F-2

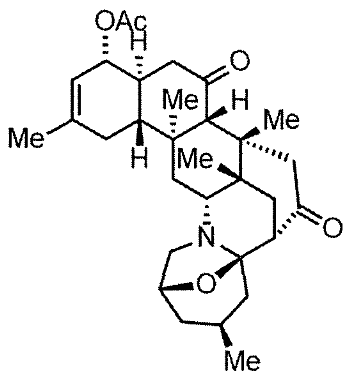

F-3

The physical property values of the compounds obtained by the above-mentioned method are shown below.

Present compound F-1: $^1$H-NMR ($CDCl_3$) $\delta$: 0.86 (3H, d, J=6.5Hz), 0.91 (3H, s), 1.01 (3H, s), 1.09 (1H, dd, J=13Hz, 12Hz), 1.32 (1H, ddd, J=14Hz, 11Hz, 2.4Hz), 1.40 (3H, s), 1.43-1.50 (2H, m), 1.71 (1H, dd, J=14Hz, 4.4Hz), 1.80 (1H, dd, J=14Hz, 2.3Hz), 1.86 (1H, dd, J=14Hz, 3.3Hz), 1.92 (1H, dd, J=14Hz, 3.4Hz), 1.90-1.96 (1H, m), 2.02 (3H, s), 2.09 (1H, ddd, J=15Hz, 8.5Hz, 5.1Hz), 2.27-2.34 (2H, m), 2.37 (1H, dd, J=13Hz, 11Hz), 2.54 (1H, s), 2.56-2.64 (2H, m), 2.70-2.75 (2H, m), 2.79 (1H, dd, J=11Hz, 7.4Hz), 3.18 (1H, dd, J=14Hz, 2.0Hz), 3.25 (1H, dd, J=11Hz, 1.3Hz), 3.48 (1H, d, J=15Hz) 4.49-4.53 (1H, m), 5.91 (1H, s).

Present compound F-2: $^1$H-NMR ($CDCl_3$) $\delta$: 0.85 (3H, d, J=6.5Hz), 0.92 (3H, s), 1.02 (3H, s), 1.08 (1H, dd, J=13Hz, 12Hz), 1.26-1.31 (1H, m), 1.33 (3H, s), 1.39-1.48 (2H, m), 1.68 (1H, dd, J=15Hz, 4.1Hz), 1.74 (3H, s), 1.73-1.80 (3H, m), 1.85 (1H, dd, J=14Hz, 3.1Hz), 1.89-1.96 (4H, m), 2.08 (1H, dd, J=14Hz, 11Hz), 2.55 (1H, s), 2.54-2.57 (1H, m), 2.72 (1H, dd, J=5.0Hz, 3.1Hz), 2.78 (1H, dd, J=11Hz, 7.5Hz), 2.84 (1H, dd, J=14Hz, 4.5Hz), 3.18 (1H, dd, J=14Hz, 2.0Hz), 3.23 (1H, dd, J=11Hz, 1.3Hz), 3.52 (1H, d, J=14Hz) , 3.95 (1H, br s), 4.48-4.52 (1H, m), 5.38 (1H, s).

Present compound F-3: $^1$H-NMR ($CDCl_3$) $\delta$: 0.85 (3H, d, J=6.5Hz), 0.92 (3H, s), 1.01 (3H, s), 1.08 (1H, dd, J=13Hz, 12Hz), 1.25-1.32 (1H, m), 1.34 (3H, s), 1.42 (1H, dd, J=13Hz, 5.0Hz), 1.44-1.49 (1H, m), 1.69 (1H, dd, J=14Hz, 4.1Hz),

1.74 (3H, s), 1.76-1.87 (3H, m), 1.88 (1H, dd, J=11Hz, 3.1Hz), 1.93-2.03 (4H, m), 2.05 (3H, s), 2.07-2.12 (1H, m), 2.53-2.58 (2H, m), 2.61 (1H, s), 2.72 (1H, dd, J=5.0Hz, 3.1Hz), 2.78 (1H, dd, J=11Hz, 7.4Hz), 3.17 (1H, dd, J=14Hz, 2.0Hz), 3.23 (1H, d, J=11Hz), 3.51 (1H, d, J=14Hz), 4.50 (1H, d, J=5.9Hz), 5.22 (1H, d, J=7.6Hz), 5.31 (1H, s).

Preparation Example 13

The present compounds A-21 and A-22 can be obtained according to the method described in JP H10-101678 A1, etc. The identification of the obtained compounds can be conducted by a measurement of $^{1}$H-NMR spectra or a measurement of $^{13}$C-NMR spectra, etc.

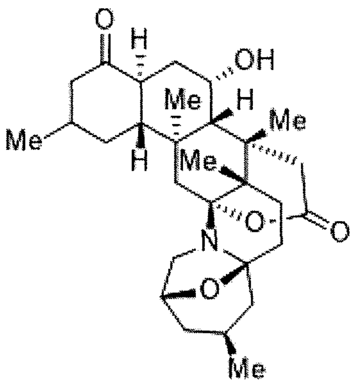

A-21

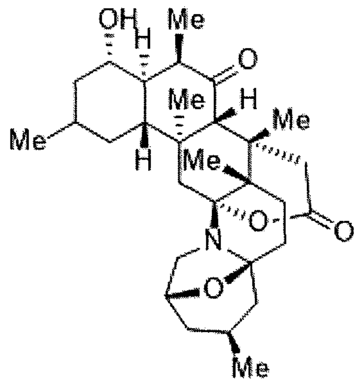

A-22

Preparation Example 14

The present compounds E-2 and E-3 can be obtained according to the method described in US 2016/0036094 A1, etc. The identification of the obtained compounds can be conducted by a measurement of $^{1}$H-NMR spectra or a measurement of $^{13}$C-NMR spectra, etc.

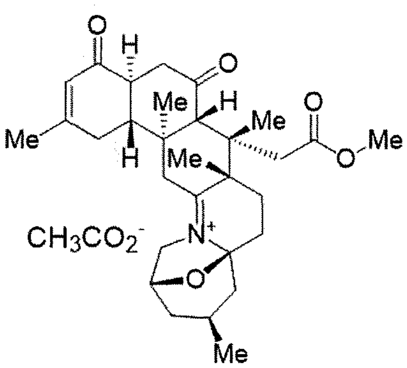

E-2

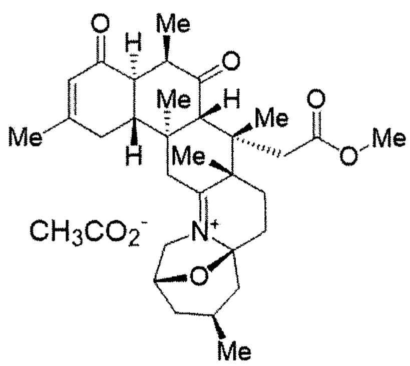

E-3

Preparation Example 15

The present compounds E-4 and E-5 can be obtained according to the method described in 62nd Symposium on the Chemistry of Natural Products, symposium abstract (2020), p2-8, etc. The identification of the obtained compounds can be conducted by a measurement of $^{1}$H-NMR spectra or a measurement of $^{13}$C-NMR spectra, etc.

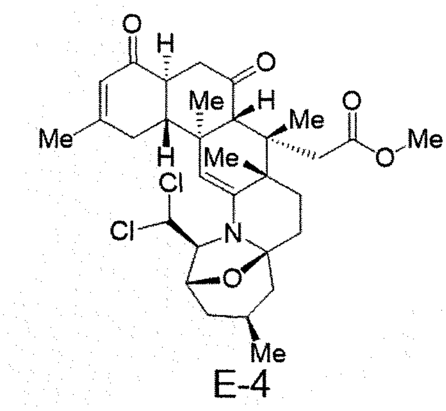

E-4

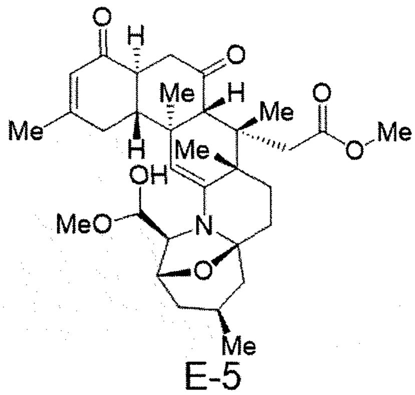

E-5

Preparation Example 16

Norzoanthamine 300 mg was dissolved in methanol 54 mL, and thereto was added 10 % palladium carbon (NX type, hydrate, manufactured by N.E. ChemCat Corporation) 100 mg, and the inner atmosphere of the reactor was purged with hydrogen, and the mixture was stirred at room temperature for 3 hours. After the completion of the reaction, the resulting mixture was filtered through Celite (registered trade mark), and the obtained solids were washed with methanol 20 mL three times. The obtained filtrates and the washing solutions were combined, and the mixture was concentrated under reduced pressure, and then was subjected to a silica gel column chromatography (chloroform : methanol = 1:0 → 9:1) to obtain the compound GG-1 of the present invention as white solid 110 mg.

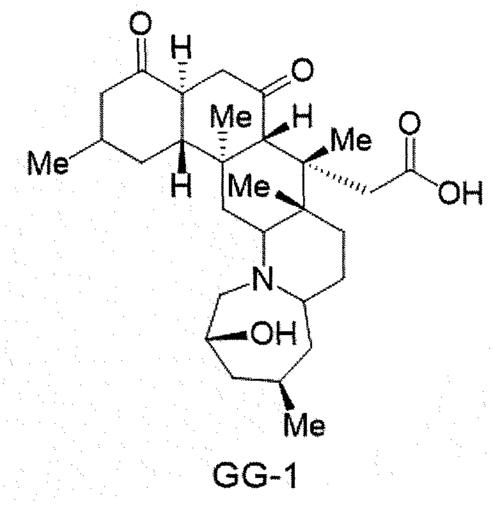

GG-1

Compound GG-1 of the present invention: $^1$H-NMR ($CDCl_3$) δ: 0.95 (3H, d, J=6.3Hz), 0.96 (3H, s), 1.06 (3H, s), 1.11 (3H, d, J=6.5Hz), 1.12 (3H, s), 1.14-1.42 (4H, m), 1.59-1.73 (4H, m), 1.76-1.89 (2H, m), 1.84 (1H, dd, J=13Hz, 3.8Hz), 1.92-2.03 (3H, m), 2.05 (1H, d, J=13Hz), 2.40-2.49 (4H, m), 2.53 (1H, s), 2.56-2.60 (2H, m), 2.63-2.67 (2H, m), 2.94 (1H, d, J=15Hz), 3.40 (1H, dt, J=13Hz, 4.4Hz), 3.72-3.77 (1H, m) , 4.05 (1H, d, J=15Hz).

Preparation Example 17

The compound GG-1 of the present invention 61 mg was dissolved in chloroform 2 mL, and thereto was added methanol 0.5 mL, and the mixture was cooled in an ice bath. Trimethylsilyl diazomethane 313 mg was added thereto, and the mixture was stirred in an ice bath for 1 hour. After the completion of the reaction, the resulting mixture was concentrated under a reduced pressure, and the residue was subjected to a silica gel column chromatography (chloroform : methanol 1:0 → 9:1) to obtain the compound GG-2 of the present invention as white amorphous 49.8 mg.

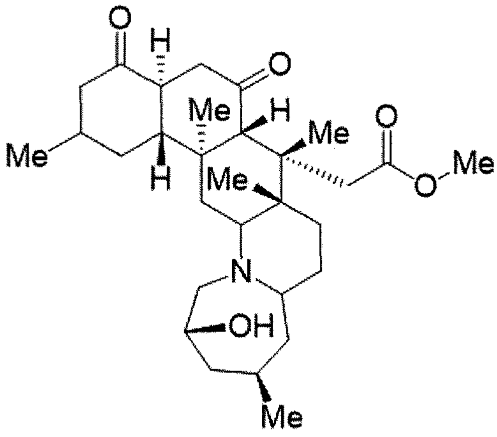

GG-2

Compound GG-2 of the present invention: $^1$H-NMR ($CDCl_3$) $\delta$: 0.88 (3H, s), 0.93 (3H, d, J=6.8Hz), 0.99-1.01 (2H, m), 1.09 (3H, d, J=6.5Hz), 1.10-1.13 (1H, m), 1.15 (3H, s), 1.16 (3H, s), 1.25-1.39 (3H, m), 1.43-1.52 (1H, m), 1.64-1.70 (1H, m), 1.65 (1H, dd, J=12Hz), 1.78-1.90 (3H, m), 1.96-2.06 (2H, m), 2.01 (1H, d, J=12Hz), 2.14 (1H, dd, J=13Hz, 10Hz), 2.37-2.44 (5H, m), 2.49 (1H, s), 2.53-2.62 (2H, m), 3.29 (1H, dd, J=14Hz, 3.3Hz), 3.47 (1H, d, J=16Hz), 3.66 (3H, s), 3.82-3.89 (1H, m), 3.99 (1H, d, J=16Hz).

Preparation Example 18

The present compound A-18 30 mg was dissolved in chloroform 3 mL, and thereto were added methyl iodide 0.3 mL and silver oxide 86.6 mg, and the mixture was heated at reflux for 50 hours. After the completion of the reaction, the resulting mixture was concentrated under reduced pressure, and the residue was subjected to a silica gel column chromatography (chloroform : methanol = 1:0 → 9:1) to obtain the compound EE-1 of the present invention as pale yellow amorphous 34 mg.

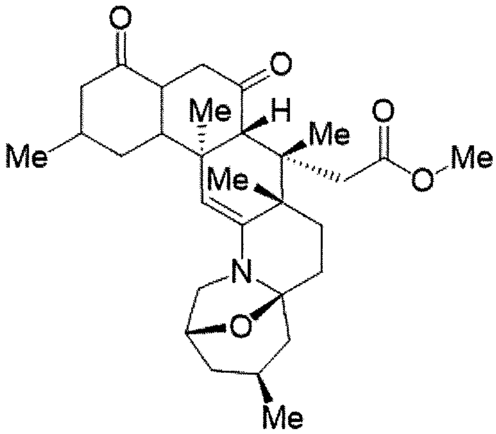

EE-1

Compound EE-1 of the present invention: LCMS: 498 $[M + H]^+$, RT = 1.28 min.

Preparation Example 19

The present compound A-18 30 mg was dissolved in methanol 3 mL, and thereto was added 1.2 N hydrochloric acid 0.5 mL, and the mixture was stirred at room temperature for 1 hour. After the completion of the reaction, the resulting mixture was concentrated under reduced pressure to obtain the compound EE-2 of the present invention 28.6 mg.

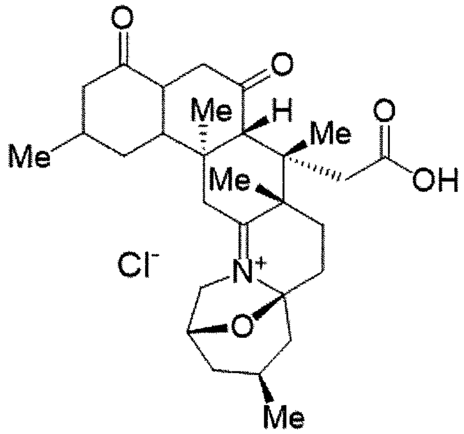

EE-2

Compound EE-2 of the present invention: $^1$H-NMR ($CD_3OD$) δ: 1.01 (3H, s), 1.02 (3H, d, J=6.8Hz), 1.13 (3H, d, J=6.3Hz), 1.32 (3H, s), 1.39 (1H, t, J=12Hz), 1.45 (3H, s), 1.45-1.49 (1H, m), 1.61-1.68 (1H, m), 1.79 (1H, dt, J=15Hz, 3.5Hz), 1.86-2.09 (6H, m), 1.97 (1H, d, J=13Hz), 2.19 (1H, t, J=13Hz), 2.22-2.29 (2H, m), 2.36-2.41 (2H, m), 2.46-2.52 (1H, m), 2.52 (1H, dd, J=15Hz, 4.8Hz), 2.73 (1H, d, J=13Hz), 2.80 (1H, dd, J=13Hz, 4.8Hz), 2.84 (1H, d, J=15Hz), 3.24 (1H, s), 3.24 (1H, d, J=15Hz), 4.18 (1H, dd, J=13Hz, 6.5Hz), 4.29 (1H, d, J=13Hz), 4.89-4.90 (1H, m).

The compound EE-2 of the present invention is a hydrochloride salt of the present compound A-18.

Preparation Example 20

The present compound B-2 29 mg was dissolved in tetrahydrofuran 3 mL, and thereto were added pyridine 23.1 mg and acetic anhydride 14.9 mg, and the mixture was stirred at room temperature for four hours. After the completion of the reaction, the resulting mixture was concentrated under reduced pressure, and the obtained residue was subjected to a silica gel column chromatography (chloroform : methanol = 1:0 → 9:1) to obtain the compound BB-1 of the present invention as pale yellow amorphous 35 mg.

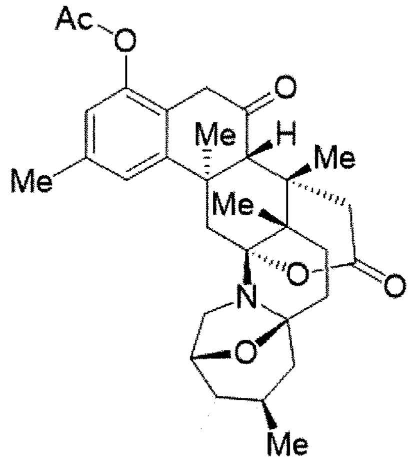

BB-1

Compound BB-1 of the present invention: $^1$H-NMR ($CDCl_3$) δ: 0.93 (3H, d, J=6.5Hz), 1.09 (3H, s), 1.10 (3H, s), 1.10-1.15 (1H, m), 1.17 (3H, s), 1.50 (1H, dt, J=17Hz, 6.3Hz), 1.46-1.53 (2H, m), 1.71-1.82 (2H, m), 1.89 (1H, dd, J=14Hz, 4.8Hz), 2.13 (1H, dd, J=13Hz, 4.5Hz), 2.31 (3H, s), 2.33-2.36 (1H, m), 2.38 (3H, s), 2.46 (1H, dd, J=20Hz, 1.2Hz), 2.53 (1H, d, J=14Hz), 2.64 (1H, d, J=14Hz), 3.20 (1H, s), 3.36 (1H, d, J=22Hz), 3.41 (1H, d, J=6.3Hz), 3.43 (1H, d, J=6.3Hz), 3.50 (1H, d, J=22Hz), 3.72 (1H, d, J=20Hz), 4.60 (1H, s), 6.82 (1H, s), 6.89 (1H, s).

Preparation Example 21

Zoanthamine 30 mg was dissolved in methanol 1 mL, and thereto was added sodium borohydride 6.0 mg in an ice

bath, and the ice bath was removed, and the mixture was stirred at room temperature for 1 hour. After the completion of the reaction, water 10 mL was added to the resulting mixture, and the mixture was extracted with chloroform 20 mL three times. The obtained organic layers were combined, and the mixture was dried over sodium sulfate, and concentrated under reduced pressure. The obtained residue was subjected to a silica gel column chromatography (chloroform : methanol = 1:0 -. 19:1) to obtain the compound EE-3 of the present invention as white solid 26.4 mg.

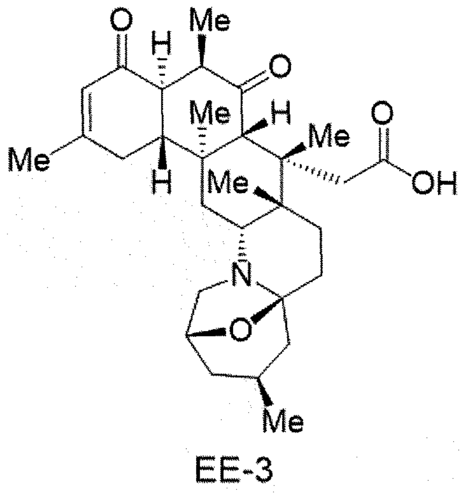

EE-3

Compound EE-3 of the present invention: $^1$H-NMR ($CDCl_3$) δ: 0.90 (3H, d, J=6.5Hz), 0.99 (1H, t, J=5.6Hz), 1.03 (3H, s), 1.04 (3H, s), 1.16 (3H, s), 1.16 (3H, d, J=7.0Hz), 1.34-1.40 (2H, m), 1.45-1.50 (1H, m), 1.66 (1H, dd, J=15Hz, 5.2Hz), 1.79-1.86 (3H, m), 1.95 (1H, dd, J=15Hz, 1.9Hz), 2.02 (3H, s), 2.26-2.42 (5H, m), 2.59 (1H, dd, J=13Hz, 5.1Hz), 2.69-2.76 (2H, m), 2.80 (1H, d, J=8.7Hz), 2.83 (1H, s), 2.88 (1H, d, J=14Hz), 3.19 (1H, dd, J=7.1Hz, 5.3Hz), 3.54 (1H, d, J=14Hz), 4.38 (1H, br s), 5.94 (1H, s), 10.28 (1H, br s).

Preparation Example 22

Zoanthamine 110 mg was dissolved in chloroform 100 mL, and thereto were added methanol 3 mL and sodium cyanoborohydride 69.7 mg, and the mixture was stirred at room temperature for 40 minutes. After the completion of the reaction, water 20 mL was added thereto, and the mixture was extracted with chloroform 30 mL twice. The resulting organic layers were dried over anhydrous sodium sulfate, and filtered. The obtained filtrates were concentrated under reduced pressure, and the obtained residue was subjected to a silica gel column chromatography (chloroform : ethanol = 1:0 -. 9:1) to obtain the compound EE-3 of the present invention 47.7 mg and the compound GG-3 of the present invention 26.9 mg.

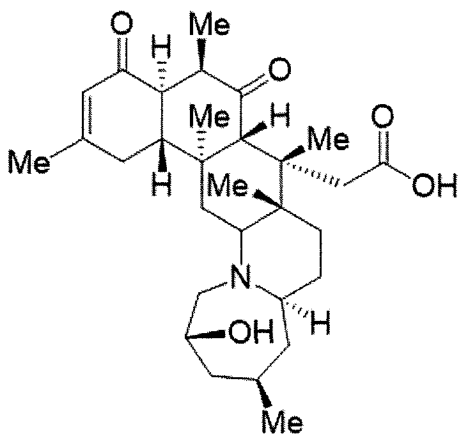

GG-3

Compound GG-3 of the present invention: $^1$H-NMR ($CDCl_3$) δ: 0.93 (3H, d, J=6.8Hz), 1.03 (3H, s), 1.16 (3H, d, J=6.8Hz), 1.20 (3H, s), 1.23-1.26 (1H, m), 1.30 (3H, s), 1.33-1.55 (6H, m), 1.78-1.83 (3H, m), 1.90 (1H, d, J=15Hz), 1.93 (1H, dd, J=14Hz, 5.8Hz), 1.99 (3H, s), 2.16 (1H, dd, J=17Hz, 11Hz), 2.34 (1H, dd, J=17Hz, 4.9Hz), 2.42-2.52 (3H, m), 2.60 (1H, dd, J=13Hz, 4.9Hz), 2.66 (1H, dd, J=13Hz, 7.7Hz), 2.74 (1H, s), 2.92 (1H, d, J=13Hz), 2.99 (1H, t, J=6.5Hz), 3.48 (1H, d,

J=15Hz), 3.92 (1H, td, J=6.5Hz, 3.1Hz), 4.99 (1H, br s), 5.92 (1H, s).

Preparation Example 23

Zoanthamine 30 mg was dissolved in chloroform 3 mL, and thereto were added methyl iodide 0.3 mL and silver(I) oxide 84.1 mg, and the mixture was heated at reflux for 34 hours. After the completion of the reaction, the resulting mixture was subjected to a silica gel column chromatography (chloroform: methanol = 1:0 → 19:1) to obtain the compound EE-4 of the present invention as while solid 28.8 mg.

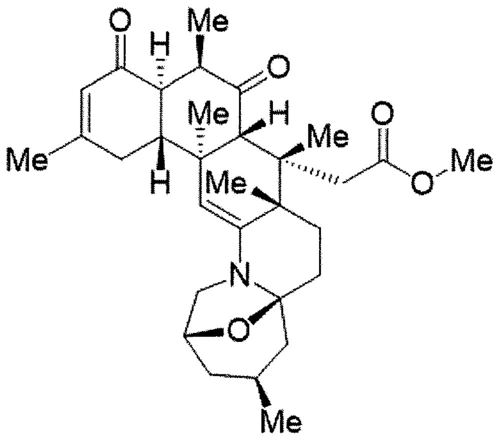

EE-4

Compound EE-4 of the present invention: $^1$H-NMR ($CDCl_3$) δ: 0.92 (3H, d, J=6.5Hz), 1.00 (3H, s), 1.09-1.15 (1H, m), 1.18 (3H, d, J=6.9Hz), 1.18 (3H, s), 1.20 (3H, s), 1.22-1.26 (1H, m), 1.44-1.51 (2H, m), 1.77-1.97 (5H, m), 2.00 (3H, s), 2.23-2.44 (3H, m), 2.52 (1H, d, J=15Hz), 2.58 (1H, dd, J=13Hz, 5.7Hz), 3.02 (1H, dd, J=6.9Hz, 5.7Hz), 3.13 (1H, dd, J=8.2Hz, 6.6Hz), 3.24 (1H, t, J=8.2Hz), 3.27 (1H, d, J=15Hz), 3.28 (1H, s), 3.68 (3H, s), 4.18 (1H, s), 4.61 (1H, d, J=6.6Hz), 5.90 (1H, s).

Preparation Example 24

The compound EE-3 of the present invention 25 mg was dissolved in chloroform 1 mL. Methanol 0.25 mL and trimethylsilyl diazomethane (2M diethyl ether solution) 125 mg were added thereto in an ice bath, and the temperature of the mixture was returned to room temperature, and stirred for 1 hour. After the completion of the reaction, the mixture was concentrated under reduced pressure, and the obtained residue was subjected to a silica gel column chromatography (chloroform : methanol = 1:0 → 9:1) to obtain the compound EE-5 of the present invention as while solid 23.1 mg.

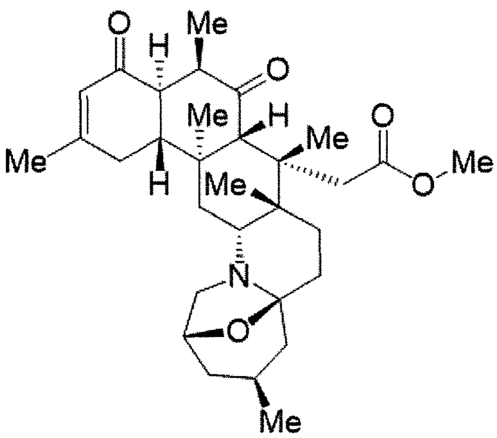

EE-5

Compound EE-5 of the present invention: $^1$H-NMR ($CDCl_3$) δ: 0.89 (3H, d, J=6.5Hz), 0.94-1.00 (1H, m), 1.09 (3H, s), 1.11 (3H, d, J=7.0Hz), 1.14 (3H, s), 1.28-1.38 (2H, m), 1.40 (3H, s), 1.43-1.50 (1H, m), 1.64 (1H, dd, J=15Hz, 5.6Hz), 1.73-1.81 (2H, m), 1.90 (1H, dd, J=13Hz, 3.2Hz), 1.94-1.99 (1H, m), 2.00 (3H, s), 2.09 (1H, dd, J=12Hz, 5.0Hz), 2.19-2.34 (3H, m),

2.43 (1H, td, J=15Hz, 5.1Hz), 2.53 (1H, dd, J=13Hz, 5.0Hz), 2.58-2.61 (2H, m), 2.67 (1H, d, J=6.8Hz), 2.73 (1H, s), 2.84 (1H, d, J=16Hz), 3.09 (1H, dd, J=7.0Hz, 5.2Hz), 3.28 (1H, d, J=16Hz), 3.68 (3H, s), 4.38 (1H, br s), 5.92 (1H, s).

Preparation Example 25

The present compound A-18 21.3 mg was dissolved in ethanol 3 mL. Thereto was added sodium borohydride 5.0 mg in an ice bath, and the mixture was stirred for 1 hour. After the completion of the reaction, water 10 mL was added to the resulting mixture, and the mixture was extracted with chloroform 10 mL three times, and the resulting organic layers were dried over anhydrous sodium sulfate, and concentrated under reduced pressure. The obtained residue was subjected to a silica gel column chromatography (chloroform : methanol = 1:0 → 9:1) to obtain the compound EE-6 of the present invention as pale yellow solid 17.8 mg.

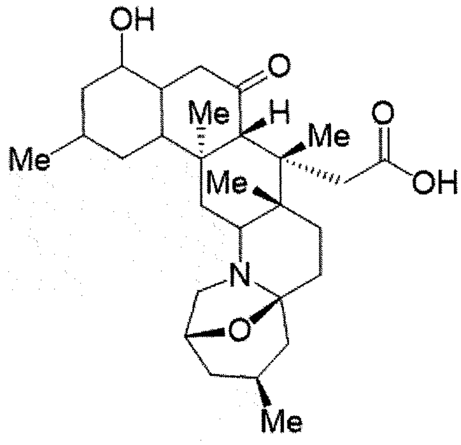

EE-6

Compound EE-6 of the present invention: LCMS: 488 [M + H]$^+$, RT = 1.10 min.

Preparation Example 26

Zoanthamine 30 mg was dissolved in ethanol 4.5 mL. Thereto was added sodium borohydride 10.3 mg in an ice bath, and the mixture was stirred for 1 hour. After the completion of the reaction, acetone 5 mL was added to the resulting mixture in an ice bath, and the mixture was stirred for 10 minutes, and the temperature of the mixture was then returned to the room temperature, and the mixture was stirred for 30 minutes. Water 10 mL was added to the resulting mixture, and the mixture was extracted with chloroform 10 mL three times. The resulting organic layers were dried over anhydrous sodium sulfate, and the mixture was filtered and concentrated under reduced pressure. The obtained residue was subjected to a silica gel column chromatography (chloroform : ethanol = 1:0 -. 9:1) to obtain the compound EE-7 of the present invention 34.8 mg.

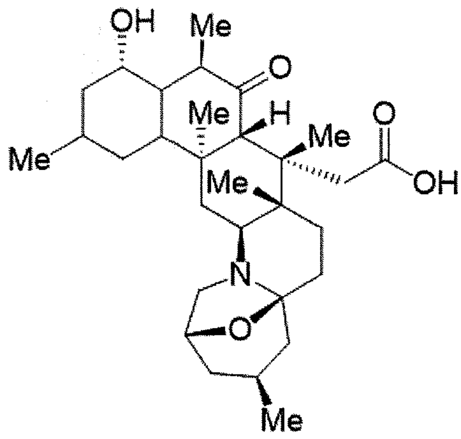

EE-7

Compound EE-7 of the present invention: LCMS: 502 [M + H]$^+$ , RT = 1.29 min.

Preparation Example 27

Norzoanthamine 20 mg was dissolved in acetonitrile 5 mL, and thereto were added p-toluene sulfonic acid monohydrate 3.84 mg and N-chlorosuccinimide 5.93 mg, and the mixture was stirred at room temperature for 20 hours. After the completion of the reaction, water 5 mL was added to the resulting mixture, and the mixture was extracted with chloroform 5 mL three times. The resulting organic layers were dried over anhydrous sodium sulfate, and concentrated under reduced pressure. The obtained residue was subjected to a silica gel column chromatography (chloroform : methanol = 1:0 → 9:1) to obtain the compound AA-1 of the present invention as white solid 23.8 mg.

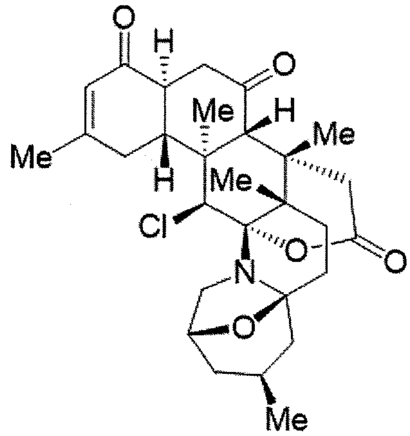

AA-1

Compound AA-1 of the present invention: $^1$H-NMR ($CDCl_3$) δ: 0.92 (3H, d, J=6.7Hz), 1.01 (3H, s), 1.11 (1H, t, J=13Hz), 1.19 (3H, s), 1.49 (3H, s), 1.51-1.61 (3H, m), 1.77 (1H, d, J=13Hz), 1.69-1.79 (1H, m), 1.94 (1H, dd, J=13Hz, 4.3Hz), 2.02 (3H, s), 2.12 (1H, dd, J=13Hz, 4.9Hz), 2.22-2.36 (2H, m), 2.42 (1H, d, J=20Hz), 2.45 (1H, dd, J=6.4Hz, 5.8Hz), 2.54 (1H, dd, J=18Hz, 3.2Hz), 2.62-2.66 (1H, m), 2.72 (1H, dd, J=14Hz, 5.8Hz), 2.92 (1H, ddd, J=14Hz, 10Hz, 3.2Hz), 3.00 (1H, s), 3.24 (1H, d, J=6.8Hz), 3.83 (1H, d, J=20Hz), 3.84 (1H, t, J=6.8Hz), 4.27 (1H, s), 4.62 (1H, d, J=5.9Hz), 5.91 (1H, s).

Preparation Example 28

The method was conducted by using zoanthamine in place of norzoanthamine according to the Preparation Example 27 to obtain the compound AA-2 of the present invention as while solid quantitatively.

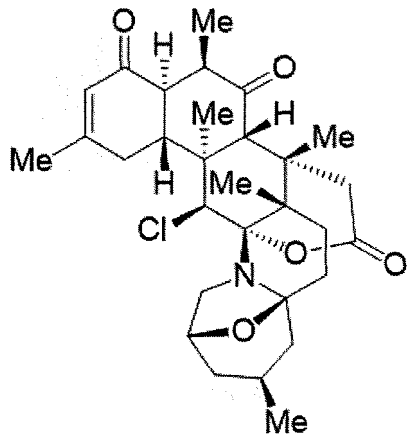

AA-2

Compound AA-2 of the present invention: $^1$H-NMR ($CDCl_3$) δ: 0.92 (3H, d, J=6.3Hz), 0.97 (3H, s), 1.11 (1H, t, J=13Hz), 1.18 (3H, s), 1.19 (3H, d, J=6.9Hz), 1.51 (3H, s), 1.52-1.61 (3H, m), 1.77 (1H, d, J=13Hz), 1.69-1.78 (1H, m), 1.95 (1H, dd,

J=13Hz, 3.9Hz), 2.01 (3H, s), 2.13 (1H, dd, J=14Hz, 5.1Hz), 2.19 (1H, dd, J=18Hz, 11Hz), 2.27-2.35 (1H, m), 2.42 (1H, d, J=21Hz), 2.49 (1H, dd, J=18Hz, 3.6Hz), 2.60 (1H, dd, J=14Hz, 5.3Hz), 3.06 (1H, dd, J=6.9Hz, 5.3Hz), 3.14 (1H, ddd, J=14Hz, 11Hz, 3.6Hz), 3.24 (1H, d, J=7.2Hz), 3.39 (1H, s), 3.83 (1H, d, J=21Hz), 3.85 (1H, t, J=7.2Hz), 4.28 (1H, s), 4.62 (1H, d, J=6.1Hz), 5.91(1H, s).

Preparation Example 29

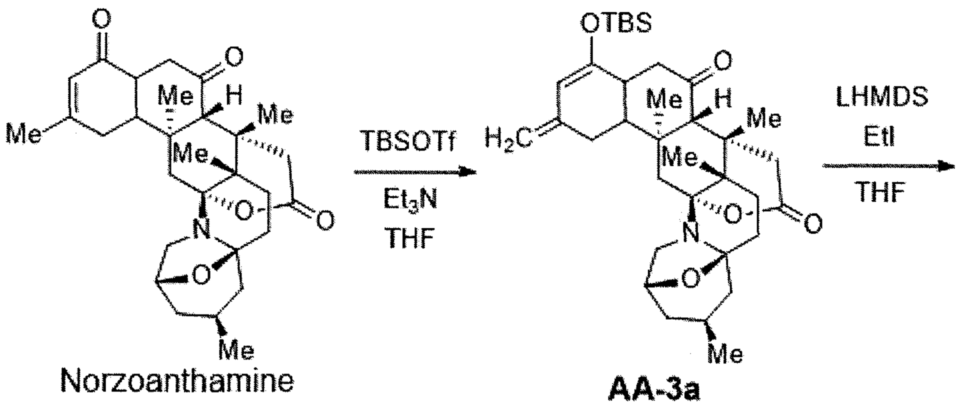


Norzoanthamine

**AA-3a**

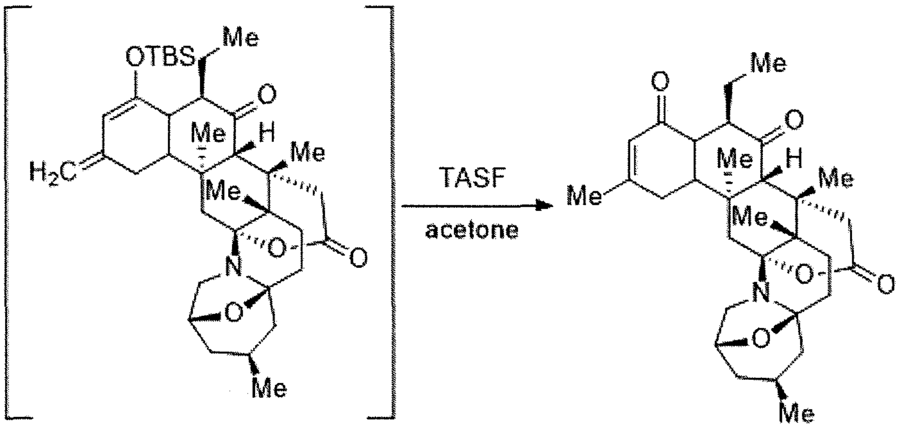


**AA-3**

Norzoanthamine 300 mg was dissolved in tetrahydrofuran 10 mL, and triethylamine 630 mg was added thereto. The mixture was cooled in a dry ice-ethanol bath, and tertbutyldimethylsilyl trifluoromethane sulfonate 823 mg was added thereto, and the mixture was stirred at -70°C for 3 hours. After the completion of the reaction, saturated aqueous solution of sodium hydrogen carbonate 5 mL was added thereto at -70°C, and the temperature of the mixture was then returned to room temperature, and stirred for 10 minutes. Water 10 mL was added thereto, and the mixture was extracted with chloroform three times. The resulting organic layers were dried over anhydrous sodium sulfate, and was concentrated under reduced pressure. The obtained residue was subjected to a silica gel column chromatography (hexane : ethyl acetate = 4:1 → 1:2) to obtain the compound AA-3a as white solid 257 mg.

The compound AA-3a 40 mg was dissolved in tetrahydrofuran 1.5 mL, and the mixture was cooled in a salt-ice bath, and lithium hexamethyldisilazane (LHMDS) (1.1 M tetrahydrofuran solution) 305 μL was added thereto, and the mixture was stirred at 20°C for 1 hour. Ethyl iodide 106 μL was added thereto, and the mixture was stirred at 20°C for 3 hours. After the completion of the reaction, water was added thereto, and the mixture was extracted with ethyl acetate three times. The resulting organic layers were dried over anhydrous sodium sulfate, and was concentrated under reduced pressure. Tris(dimethylamino)sulfonium difluorotrimethylsilicate (TASF) 73.9 mg which was dissolved in acetone 2 mL was added to the obtained residue, and the mixture was stirred at room temperature for 1 hour. After the completion of the reaction, a phosphoric acid buffer 5 mL which was adjusted pH 7 was added thereto, and the mixture was extracted with ethyl acetate three times, and the resulting organic layers were dried over anhydrous sodium sulfate, and concentrated under reduced pressure. The obtained residue was subjected to a reverse phase column chromatography (water : acetonitrile = 4:1 → 3:1) to obtain the compound AA-3 of the present invention as pale yellow solid 6.0 mg.

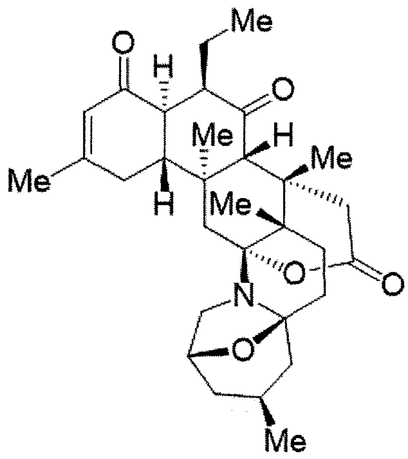

AA-3

Compound AA-3 of the present invention: $^1$H-NMR ($CDCl_3$) $\delta$: 0.85 (3H, t, J=7.4Hz), 0.92 (3H, d, J=6.5Hz), 1.00 (6H, s), 1.10 (1H, t, J=13Hz), 1.21 (3H, s), 1.36-1.51 (3H, m), 1.66-1.81 (2H, m), 1.92 (1H, d, J=14Hz), 1.87-2.01 (3H, m), 2.01 (3H, s), 2.11 (1H, dd, J=13Hz, 5.0Hz), 2.16 (1H, d, J=14Hz), 2.19-2.23 (2H, m), 2.24-2.30 (1H, m), 2.37 (1H, d, J=20Hz), 2.41-2.47 (1H, m), 2.65 (1H, dd, J=13Hz, 5.6Hz), 2.80-2.85 (1H, m), 3.24 (1H, t, J=6.4Hz), 3.26 (1H, br s), 3.29 (1H, d, J=6.4Hz), 3.67 (1H, d, J=20Hz), 4.56 (1H, d, J=5.7Hz), 5.92(1H, s).

Preparation Example 29-1

The compound which was prepared according to the Preparation Example 29 and its physical property value are shown.
Present compound A-1: $^1$H-NMR ($CDCl_3$) $\delta$: 0.92 (3H, d, J=6.5Hz), 1.00 (6H, s), 1.10 (1H, t, J=13Hz), 1.19 (3H, s), 1.44-1.51 (2H, m), 1.69-1.80 (2H, m), 1.85-2.02 (2H, m), 1.92 (1H, d, J=14Hz), 2.02 (3H, s), 2.10 (1H, dd, J=13Hz, 5.1Hz), 2.18 (1H, d, J=14Hz), 2.23-2.31 (3H, m), 2.38 (1H, d, J=20Hz), 2.52-2.60 (1H, m), 2.79 (1H, dd, J=14Hz, 5.9Hz), 3.06-3.08 (1H, m), 3.23 (1H, s), 3.21-3.26 (1H, m), 3.29 (1H, d, J=6.1Hz), 3.70 (1H, d, J=20Hz), 3.91 (1H, dd, J=11Hz, 4.5Hz), 3.99 (1H, dd, J=11Hz, 6.4Hz), 4.55-4.57 (1H, m), 5.93 (1H, s).

Next, the formulation examples of the present compounds and the compounds of the present invention are shown below. In the formulation examples, the "parts" represents "part by weight" unless otherwise specified. The present compound S represents the present compounds A-1 to A-23, B-1, B-2, C-1 to C-44, D-1, D-2, E-1 to E-5, F-1 to F-5, G-1 and G-2, as well as the compounds AA-1 to AA-3, BB-1, EE-1 to EE-7, and GG-1 to GG-3 of the present invention.

Formulation Example 1

Thirty five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

Formulation Example 2

Fifty (50) parts of any one of the present compound S, 3 parts of calcium lignin sulfonate, 2 parts of sodium lauryl sulfate, and 45 parts of silica are well mixed-grinding to obtain a formulation.

Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the

mixture is well kneaded and is then granulated with a granulator and dried to obtain a formulation.

Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Formulation Example 6

Zero point one (0.1) parts of any one of the present compound S and 39.9 parts of kerosene are dissolved while mixing, and the mixture is placed in an aerosol container, and 60 parts of liquefied petroleum gas (mixture of propane, butane and isobutane; saturated vapor pressure: 0.47 MPa (25°C)) is filled in the container to obtain a formulation.

Formulation Example 7

Zero point two (0.2) parts of any one of the present compound S, 50 parts of pyrethrum extract dreg powder, 30 parts of Machilus thunbergii powder, and 19.8 parts of wood powder are mixed, and an appropriate amount of water is added thereto, and the mixture is well kneaded, and is extructed with an extruder into a plate-like sheet, and the resulting sheet is made a spiral-like form thereof with a punching machine to obtain a formulation.

Next, Test Examples were conducted to show an efficacy of the present compound and the compound of the present invention on controlling a harmful arthropod or a harmful nematode. The following test examples were conducted at 25°C.

Also, the "untreated group" represents a group where the similar treatment procedure to that of the treated group except not using the test compound is done.

Test Method 1

The test compounds are made to a formulation according to a similar method to that described in the Formulation Example 5, and thereto is added water containing 0.03 v/v % of Shindain (registered trademark) to prepare a diluted solution containing a prescribed concentration of the test compound.

Cabbage (*Brassicae oleracea*) seedling (on the developmental stage of the third to fourth true leaf) is planted in a cup, and the diluted solutions are sprayed into the seedling at a ratio of 20 mL/seedling. Thereafter, ten (10) cotton worm (*Spodoptera litura*) at the third instar larval stage are released. After 6 days, the number of the surviving insects is counted, and the mortality of insects is calculated by the following equation.

Mortality (%) = (1 - Number of surviving insects/10) $\times$ 100

Test Example 1-1

The test was conducted by making the prescribed concentration 200 ppm and using the below-mentioned Present compounds or the compounds of the present invention as a test compound according to the test method 1. As a result of the test, the below-mentioned Present compounds and the compounds of the present invention showed 80 % or greater as the mortality of insects.

Present compounds: A-1, A-18, A-19, B-1, and B-2
Compounds of the present invention: AA-1, AA-2, AA-3, BB-1, EE-2, EE-3, EE-5, and GG-3

Test Method 2

Test compounds are made to a formulation according to a similar method to that described in the Formulation Example 5, and thereto is added water containing 0.03 v/v % of Shindain (registered trademark) to prepare a diluted solution containing a prescribed concentration of the test compound.

Cabbage (*Brassicae oleracea*) seedling (on the developmental stage of the third to fourth true leaf) is planted in a cup, and the diluted solutions are sprayed into the seedling at a ratio of 20 mL/seedling. Thereafter, ten (10) cotton worm (*Spodoptera litura*) at the third instar larval stage are released. After 6 days, the number of the surviving insects is counted, and the mortality of insects is calculated by the following equation.

Mortality (%) = (1 - Number of surviving insects/10) × 100

Test Example 2-1

The test was conducted by making the prescribed concentration 200 ppm and using the below-mentioned Present compounds or the compounds of the present invention as a test compound according to the test method 2. As a result of the test, the below-mentioned Present compounds and the compounds of the present invention showed 80 % or greater as the mortality of insects.

Present compounds: A-1, A-18, A-19, B-1, B-2, and E-1
Compounds of the present invention: AA-1, AA-2, AA-3, BB-1, EE-2, EE-4, EE-5, and EE-6

Test Method 3

Test compounds are made to a formulation according to a similar method to that described in the Formulation Example 5, and thereto is added water containing 0.03 v/v% of Shindain (registered trademark) to prepare a diluted solution containing a prescribed concentration of the test compound.
Cucumber (*cucumber sativus*) seedling (on the developmental stage of the second true leaf) is planted in a cup, and approximately 30 cotton aphids (*Aphis gossypii*) (all stages of life) are released onto the seedling. After one day, the diluted solutions are sprayed into the seedling at a ratio of 10 mL/seedling. After additional 5 days, the number of the surviving insects is examined and the controlling value is calculated by the following equation.

Cb: Number of the test insects in untreated group;
Cai: Number of the surviving insects at the time of the examination in untreated group;
Tb: Number of the test insects in treated group;
Tai: Number of the surviving insects at the time of the examination in treated group;

Test Example 3-1

The test was conducted by making the prescribed concentration 200 ppm and using the below-mentioned Present compounds or the compounds of the present invention as a test compound according to the test method 3. As a result of the test, the below-mentioned Present compounds and the compounds of the present invention showed 90 % or greater as the mortality of insects.

Present compounds: A-1, A-18, A-19, B-1, B-2, and E-1
Compounds of the present invention: AA-1, AA-2, AA-3, BB-1, EE-1, EE-2, EE-3, EE-5, and GG-3

Test Method 4

Test compounds are made to a formulation according to a similar method to that described in the Formulation Example 5, and thereto is added water containing 0.03 v/v% of Shindain (registered trademark) to prepare a diluted solution containing a prescribed concentration of the test compound.
Rice (*Oryza sativa*) seedling (on the developmental stage of the second true leaf) is planted in a cup, and the diluted solutions are sprayed into the seedling at a ratio of 10 mL/seedling. Thereafter, twenty (20) of 3rd instar larvae of brown planthoppers (*Nilaparvata lugens*) are released onto the rice leaves. After 6 days, the number of the surviving insects is examined and the mortality of insects is calculated by the following equation.

Mortality (%) = (1 - Number of surviving insects/20) × 100

Test Example 4-1

The test was conducted by making the prescribed concentration 200 ppm and using the below-mentioned Present compounds or the compounds of the present invention as a test compound according to the test method 4. As a result of the test, the below-mentioned Present compounds and the compounds of the present invention showed 90 % or greater as the mortality of insects.

Present compounds: A-1, A-18, A-19, B-2, and E-1
Compounds of the present invention: AA-1, AA-2, AA-3, EE-2, EE-3, EE-4, EE-5, GG-1, and GG-3

Test Method 5

Test compounds are made to a formulation according to a similar method to that described in the Formulation Example 5, and thereto is added water containing 0.03 v/v% of Shindain (registered trademark) to prepare a diluted solution containing a prescribed concentration of the test compound.

Cucumber (*cucumber sativus*) seedling (on the developmental stage of the second true leaf) is planted in a cup, and the diluted solutions are sprayed into the seedling at a ratio of 10 mL/seedling. Thereafter, the second true leaf is cut, and the cut leaf was placed in the cup, and approximately twenty (20) of larvae of western flower thrips (Frankliniella occidentalis) larvae are released onto the leaf. After 6 days, the number of the surviving insects is examined and the mortality of insects is calculated by the following equation.

Mortality (%) = (1 - Number of surviving insects/20) × 100

Test Example 5-1

The test was conducted by making the prescribed concentration 200 ppm and using the below-mentioned Present compounds or the compounds of the present invention as a test compound according to the test method 5. As a result of the test, the below-mentioned Present compounds and the compounds of the present invention showed 90 % or greater as the mortality of insects.

Present compounds: A-18, A-19, B-1, and E-1
Compounds of the present invention: AA-1, AA-2, BB-1, EE-4, EE-1, EE-2, EE-3, EE-5, EE-6, and GG-3

Test Method 6

Test compounds are made to a formulation according to a similar method to that described in the Formulation Example 5, and thereto is added water containing 0.03 v/v% of Shindain (registered trademark) to prepare a diluted solution containing a prescribed concentration of the test compound.

An adult of silverleaf whiteflies (Bemisia tabaci) are released on tomato (Lycopersicon esculentum) seedling that is planted in the container, and then spawn for about 24 hours. The seedling are stored for 8 days, and the larvae of silverleaf whiteflies are hatched from the laid eggs. The diluted solutions are sprayed into the seedling in a ratio of 10 mL/seedling. After 7 days, the number of the surviving insects is examined, and the controlling value is calculated by the following equation.

$$\text{Controlling value (\%)} = \{1-(Cb \times Tai)/(Cai \times Tb)\} \times 100$$

wherein the symbols in the formula represent the following descriptions.

Cb: Number of the insects shortly before the treatment in untreated group;
Cai: Number of the surviving insects at the time of the investigation in untreated group;
Tb: Number of the insects shortly before the treatment in treated group;
Tai: Number of the surviving insects at the time of the investigation in treated group;

Test Example 6-1

The test was conducted by making the prescribed concentration 200 ppm and using the below-mentioned Present compounds or the compounds of the present invention as a test compound according to the test method 6. As a result of the test, the below-mentioned Present compounds and the compounds of the present invention showed 90 % or greater as the mortality of insects.

Present compounds: A-1, and E-1
Compounds of the present invention: AA-1, AA-2, AA-3, EE-1, and EE-2

Test Method 7

Test compounds 60 mg are dissolved in acetone 600 μL containing Tween (Registered trade mark) by 5 %, and thereto is added water to prepare a diluted solution containing a prescribed concentration of the test compound.

The diluted solution 20 mL are soil which is polluted with southern root-knot nematode (Meloidogyne incognita) 400 mL are mixed, and a root of healthy tomato seedlings (on the developmental stage of third to fourth true leaf) is transplanted in the soil. After the transplanting, the diluted solution 40 mL is irrigated onto soli of tomato seedlings. After 10 days, the roots are pull out from the soil, washed and the damage degree of the root (root-knot degree) is examined according to a root-knot index by Zeck (Zeck, W.M. (1971): Pflanzenschutz-Nachrichten. Bayer AG, 24, 141-144).

[Root-knot index by Zeck]

0: No root-knots is recognized.
1: By careful observation, several small root-knots can be recognized.
2: Several small root-knots can be easily confirmed similarly to 1.
3: Many small root-knots are found, and some of which are fused. A function of the root is not almost lost.
4: Many small root-knots are found, and some large ones are found. Most of the root are functional.
5: Twenty-five (25) % of the root were remarkably covered with root-knots, and the roots were not functional.
6: Fifty (50) % of the root were remarkably covered with root-knots, and the roots were not functional.
7: Seventy-five (75) % of the root were remarkably covered with root-knots, and the regenerative ability of the roots is lost.
8: No healthy roots is found, and the uptake of nutrients by the plant is inhibited. The foliage is still green.
9: The root system that is completely covered with root-knots is rotting. Plants are dying.
10: Plants and roots are died.

Test Example 7

The test was conducted by making the prescribed concentration 1,000 ppm and using the compound AA-1 of the present invention as a test compound according to the test method 7. As a result of the test, the root-knot index of the treated group of the compound AA-1 of the present invention was "1". On the other hand, the root-knot index in untreated group was "4". Accordingly, the compound AA-1 of the present invention reduced a formation of root-knot.

Test Method 8

The test compounds are dissolved with acetone containing a surfactant, and the solution was then poured into a container of 20 mL contents, and the solution was coating uniformly in the inner face of the container such that the test compound is made 10 mg/$m^2$, and the surfactant is made 40 mg/$m^2$, and the container is dried.
Five (5) of female adult of northern house mosquitos (Culex pipiens pipiens) are placed in the contained, and the contained is put the lid. After the passing of the prescribed times, the state of the northern house mosquitos are examined, and the mortality is calculated by the following equation.

Mortality (%) = (Number of dead insects/Number of surviving insects) $\times$ 100

Test Example 8

The test results that were conducted according to Test Method 8 are shown below. When the prescribed time was made one (1) day and the below-mentioned present compounds or the compounds of the present invention were used as a test compound, as a result of the test, the below-mentioned Present compounds and the compounds of the present invention showed 80 % or greater as the mortality of insects.

Present compounds: A-19, B-1, B-2, and E-1
Compounds of the present invention: AA-1, AA-2, AA-3, BB-1, EE-1, EE-2, EE-3, EE-4, EE-5, EE-6, GG-1, GG-2, and GG-3

Industrial Applicability

The present compounds and the compounds of the present invention show an excellent control effect on harmful arthropods or harmful nematodes

## Claims

1. A method for controlling a harmful arthropod or a harmful nematode, which comprises applying an effective amount of a compound represented by formula (I):

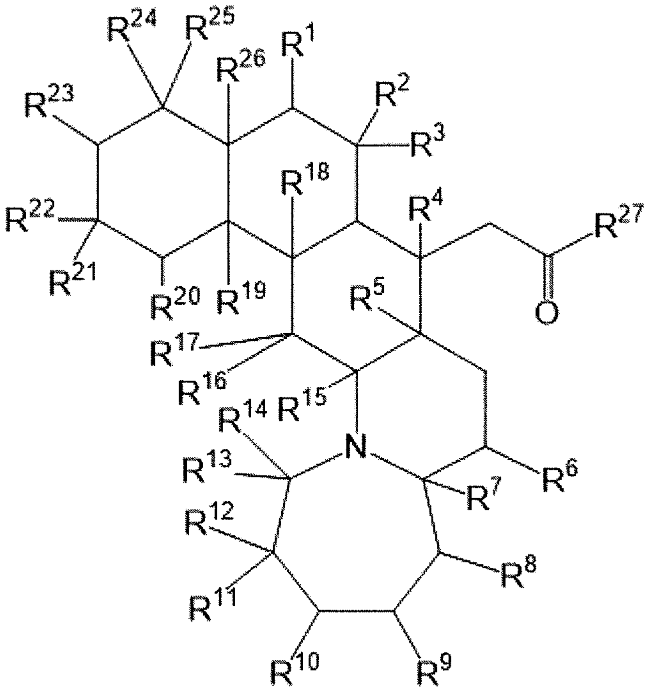

(I)

[wherein

$R^1$ represents a hydrogen atom, a C1-C3 alkyl group, or $CH_2OR^{A1}$,
$R^2$ represents $OR^{A2}$,
$R^3$ represents a hydrogen atom,
$R^4$ represents a methyl group or $CH_2OR^{A3}$,
$R^5$ represents a methyl group or $CH_2OR^{A4}$,
$R^6$ represents a hydrogen atom or $OR^{A5}$,
$R^7$ represents a hydrogen atom,
$R^8$ represents a hydrogen atom, a halogen atom, a methoxy group, or $OR^{A6}$,
$R^9$ represents a methyl group or $CH_2OR^{A7}$,
$R^{10}$ represents a hydrogen atom or $OR^{A8}$,
$R^{11}$ represents $OR^{A9}$,
$R^{12}$ represents a hydrogen atom or $OR^{A10}$,
$R^{13}$ represents a hydrogen atom, a dichloromethyl group, a hydroxy(methoxy)methyl group, a hydroxy(ethoxy) methyl group, or $OR^{A11}$,
$R^{14}$ represents a hydrogen atom,
$R^{15}$ represents a hydrogen atom or $OR^{A12}$,
$R^{16}$ represents a hydrogen atom, a halogen atom, or $OR^{A13}$,
$R^{17}$ represents a hydrogen atom,
$R^{18}$ represents a methyl group, or $CH_2OR^{A14}$,
$R^{19}$ represents a hydrogen atom,
$R^{20}$ represents a hydrogen atom or $OR^{A15}$,
$R^{21}$ represents a methyl group, $CH_2OR^{A16}$, or $CH_2NHCH_2C(O)OCH_3$,
$R^{22}$ represents a hydrogen atom or $OR^{A17}$,
$R^{23}$ represents a hydrogen atom or $OR^{A18}$,
$R^{24}$ represents a hydrogen atom, a C1-C3 alkoxy group, or $OR^{A19}$,
$R^{25}$ represents a hydrogen atom,
$R^{26}$ represents a hydrogen atom or $OR^{A20}$,

$R^{27}$ represents a C1-C3 alkoxy group or a hydroxy group,

$R^{A1}$, $R^{A2}$, $R^{A3}$, $R^{A4}$, $R^{A5}$, $R^{A6}$, $R^{A7}$, $R^{A8}$, $R^{A9}$, $R^{A10}$, $R^{A11}$, $R^{A12}$, $R^{A13}$, $R^{A14}$, $R^{A15}$, $R^{A16}$, $R^{A17}$, $R^{A18}$, $R^{A19}$, and $R^{A20}$ are identical to or different from each other, and each represents a hydrogen atom, a (C1-C6 alkyl)carbonyl group, or a (C3-C6 cycloalkyl)carbonyl group,

$R^1$ and $R^{27}$ may combine with each other to form $\#^1$-$CH_2$-O-, and $\#^1$ represents a binding position of a carbon atom to which $R^1$ is attached,

$R^2$ and $R^3$ may combine with each other to form an oxo group,

$R^3$ and $R^{18}$ may combine with each other to form $\#^3$-O-$CHR^{B1}$-, and $\#^3$ represents a binding position of a carbon atom to which $R^3$ is attached,

$R^4$ and $R^{27}$ may combine with each other to form $\#^4$-$CH_2$-O-, and $\#^4$ represents a binding position of a carbon atom to which $R^4$ is attached,

$R^6$ and $R^{27}$ may combine with each other to form a bond,

$R^7$ and $R^{11}$ may combine with each other to form -O-,

$R^{13}$ and $R^{14}$ may combine with each other to form an oxo group,

$R^{15}$ and $R^{16}$ may combine together with carbon atoms to which they are attached to form a double bond,

$R^{15}$ and $R^{18}$ may combine with each other to form $\#^{15}$-O-$CHR^{B2}$-, and $\#^{15}$ represents a binding position of a carbon atom to which $R^{15}$ is attached,

$R^{15}$ and $R^{27}$ may combine with each other to form -O-,

$R^{16}$ and $R^{17}$ may combine with each other to form an oxo group,

$R^{18}$ and $R^{22}$ may combine with each other to form $\#^{18}$-$CH_2$-O-, and $\#^{18}$ represents a binding position of a carbon atom to which $R^{18}$ is attached,

$R^{18}$ and $R^{23}$ may combine with each other to form -$CH_2$-, $R^{19}$, $R^{20}$, $R^{22}$, $R^{23}$, $R^{25}$, and $R^{26}$ may combine together with carbon atoms to which they are attached to form a benzene ring,

$R^{21}$ and $R^{22}$ may combine with each other to form =$CH_2$, $R^{22}$ and $R^{23}$ may combine together with carbon atoms to which they are attached to form a double bond,

$R^{24}$ and $R^{25}$ may combine with each other to form an oxo group,

$R^{B1}$ and $R^{B2}$ are identical to or different from each other, and each represents a hydrogen atom, a C1-C3 alkoxy group, or a hydroxy group,

with the proviso that zoanthamine, norzoanthamine, zoanthaminone, and norzoanthaminone are excluded.]

or an N-oxide thereof or a salt thereof to a harmful arthropod or a harmful nematode or a habitat for harmful arthropod or harmful nematode.

**2.** The method according to claim 1 wherein in the formula (I), a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of a to g;

a: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{21}$ and $R^{22}$ may combine with each other to form =$CH_2$, $R^{23}$ represents a hydrogen atom, or $OR^{A18}$, $R^{18}$ and $R^{23}$ may combine with each other to form -$CH_2$-, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, and $R^{26}$ represents a hydrogen atom or $OR^{A20}$;

b: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ and $R^{27}$ combine with each other to form -O-, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, $CH_2OR^{A16}$, or $CH_2NHCH_2C(O)$ $OCH_3$, and $R^{24}$ represents a C1-C3 alkoxy group, or $OR^{A19}$;

c: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^3$ and $R^{18}$ may combine with each other to form $\#^3$-O-$CHR^{B1}$-, $R^4$ and $R^{27}$ combine with each other to form $\#^4$-$CH_2$-O-, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, or $CH_2OR^{A14}$, $R^{15}$ and $R^{18}$ may combine with each other to form $\#^{15}$-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{18}$ and $R^{22}$ may combine with each other to form $\#^{18}$-$CH_2$-O-, $R^{23}$ represents a hydrogen atom, or $OR^{A18}$, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

d: a combination wherein $R^1$ and $R^{27}$ combine with each other to form $\#^1$-$CH_2$-O-, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom or $OR^{A20}$;

e: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, $R^{26}$ represents a hydrogen atom or $OR^{A20}$, and $R^{27}$ represents a C1-C3 alkoxy group or a hydroxy group;

f: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ and $R^{27}$ combine with each other to form a bond, $R^7$ and $R^{11}$ combine with each other to form -O-, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ represents a hydrogen atom, a C1-C3 alkoxy group, or $OR^{A19}$, $R^{25}$ represents a hydrogen atom, $R^{24}$ and $R^{25}$ may combine with each other to form an oxo group, and $R^{26}$ represents a hydrogen atom or $OR^{A20}$; and

g: a combination wherein $R^1$ represents a hydrogen atom, a C1-C3 alkyl group, or $CH_2OR^{A1}$, $R^2$ and $R^3$ combine with each other to form an oxo group, $R^4$ represents a methyl group or $CH_2OR^{A3}$, $R^6$ represents a hydrogen atom or $OR^{A5}$, $R^7$ represents a hydrogen atom, $R^{11}$ represents $OR^{A9}$, $R^{13}$ represents a hydrogen atom, a dichloromethyl group, a hydroxy(methoxy)methyl group, a hydroxy(ethoxy)methyl group, or $OR^{A11}$, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{18}$ represents a methyl group, or $CH_2OR^{A14}$, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom or $OR^{A17}$, $R^{23}$ represents a hydrogen atom, or $OR^{A18}$, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom or $OR^{A20}$, and $R^{27}$ represents

a C1-C3 alkoxy group or a hydroxy group.

**3.** The method according to claim 1 or 2 wherein in formula (I), $R^5$ represents a methyl group, and a combination of $R^1$, $R^2$, $R^3$, $R^4$, $R^6$, $R^7$, $R^{11}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^1$ to $g^1$:

$a^1$: a combination wherein $R^1$ represents a hydrogen atom, a C1-C3 alkyl group, or $CH_2OR^{A1}$, $R^2$ represents $OR^{A2}$, $R^3$ represents a hydrogen atom, $R^2$ and $R^3$ may combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OR^{A16}$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or $OR^{A19}$, $R^{25}$ represents a hydrogen atom, $R^{24}$ and $R^{25}$ may combine with each other to form an oxo group, and $R^{26}$ represents a hydrogen atom;

$b^1$: a combination wherein $R^1$ represents a hydrogen atom or a methyl group, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{15}$ and $R^{27}$ combine with each other to form -O-, $R^{16}$ represents a hydrogen atom or a halogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a C1-C3 alkoxy group or $OR^{A19}$;

$c^1$: a combination wherein $R^1$ represents a methyl group, $R^2$ represents $OR^{A2}$, $R^3$ represents a hydrogen atom, $R^2$ and $R^3$ may combine with each other to form an oxo group, $R^3$ and $R^{18}$ may combine with each other to form $\#^3$-O-$CHR^{B1}$-, $R^4$ and $R^{27}$ combine with each other to form $\#^4$-$CH_2$-O-, $R^6$ represents a hydrogen atom or $OR^{A5}$, $R^7$ and $R^{11}$ combine with each other to form -O-, $R^{13}$ represents a hydrogen atom or $OR^{A11}$, $R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form $\#^{15}$-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom, $R^{18}$ and $R^{22}$ may combine with each other to form $\#^{18}$-$CH_2$-O-, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

$d^1$: a combination wherein $R^1$ and $R^{27}$ combine with each other to form $\#^1$-$CH_2$-O-, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^1$: a combination wherein $R^1$ represents a hydrogen atom or a C1-C3 alkyl group, $R^2$ and $R^3$ combine with each other to form an oxo group, $R^4$ represents a methyl group, $R^6$ represents a hydrogen atom, $R^7$ and $R^{11}$ combine with each other to form -O-, $R^{13}$ represents a hydrogen atom, a dichloromethyl group, a hydroxy(methoxy)methyl group, or a hydroxy(ethoxy)methyl group, $R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, $R^{26}$ represents a hydrogen atom, and $R^{27}$ represents a C1-C3 alkoxy group or a hydroxy group;

$f^1$: a combination wherein $R^1$ represents a hydrogen atom or a C1-C3 alkyl group, $R^2$ and $R^3$ combine with each other to form an oxo group, $R^4$ represents a methyl group, $R^6$ and $R^{27}$ combine with each other to form a bond, $R^7$

and $R^{11}$ combine with each other to form -O-, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, and $R^{26}$ represents a hydrogen atom; and

$g^1$: a combination wherein $R^1$ represents a hydrogen atom or a C1-C3 alkyl group, $R^2$ and $R^3$ combine with each other to form an oxo group, $R^4$ represents a methyl group, $R^6$ represents a hydrogen atom, $R^7$ represents a hydrogen atom, $R^{11}$ represents $OR^{A9}$, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a C1-C3 alkoxy group or a hydroxy group.

**4.** The method according to any one of claims 1 to 3 wherein $R^5$ represents a methyl group, $R^8$ represents a hydrogen atom, a halogen atom, a methoxy group, or a hydroxy group, $R^9$ represents a methyl group or $CH_2OH$, $R^{10}$ represents a hydrogen atom, an acetyloxy group, or a hydroxy group, and $R^{12}$ represents a hydrogen atom or a hydroxy group.

**5.** The method according to claim 1 or 2 wherein in the formula (I), $R^{A2}$ represents a hydrogen atom, $R^{A5}$ represents a hydrogen atom, $R^{A8}$ represents a hydrogen atom or an acetyl group, $R^{A9}$ represents a hydrogen atom, $R^{A11}$ represents a hydrogen atom, $R^{A12}$ represents a hydrogen atom, $R^{A13}$ represents a hydrogen atom, $R^{A15}$ represents a hydrogen atom, $R^{A16}$ represents a hydrogen atom, $R^{A17}$ represents a hydrogen atom, $R^{A18}$ represents a hydrogen atom, $R^{A19}$ represents a hydrogen atom or an acetyl group, $R^{A20}$ represents a hydrogen atom, $R^{B1}$ represents a hydrogen atom or a methoxy group, $R^{B2}$ represents a hydrogen atom or a methoxy group, and
a combination of $R^1$, $R^2$, $R^3$, $R^4$, $R^5$, $R^6$, $R^7$, $R^8$, $R^9$, $R^{10}$, $R^{11}$, $R^{12}$, $R^{13}$, $R^{14}$, $R^{15}$, $R^{16}$, $R^{17}$, $R^{18}$, $R^{19}$, $R^{20}$, $R^{21}$, $R^{22}$, $R^{23}$, $R^{24}$, $R^{25}$, $R^{26}$, and $R^{27}$ represents any combinations of $a^2$ to $g^2$:

$a^2$: a combination wherein $R^1$ represents a hydrogen atom, a C1-C3 alkyl group, or $CH_2OH$, $R^2$ represents $OR^{A2}$, $R^3$ represents a hydrogen atom, $R^2$ and $R^3$ may combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group or $CH_2OH$, $R^{10}$ represents a hydrogen atom or $OR^{A8}$, $R^{12}$ represents a hydrogen atom or a hydroxy group, $R^{13}$ represents a hydrogen atom, $R^{14}$ represents a hydrogen atom, $R^{13}$ and $R^{14}$ may combine with each other to form an oxo group, $R^{15}$ and $R^{27}$ combine with each other to form -O-, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group or $CH_2OR^{A16}$, $R^{22}$ represents a hydrogen atom or $OR^{A17}$, $R^{21}$ and $R^{22}$ may combine with each other to form $=CH_2$, $R^{23}$ represents a hydrogen atom, or $OR^{A18}$, $R^{18}$ and $R^{23}$ may combine with each other to form $-CH_2-$, $R^{22}$ and $R^{23}$ may combine together with carbon atoms to which they are attached to form a double bond, $R^{24}$ represents a hydrogen atom or a hydroxy group, $R^{25}$ represents a hydrogen atom, $R^{24}$ and $R^{25}$ may combine with each other to form an oxo group, and $R^{26}$ represents a hydrogen atom;

$b^2$: a combination wherein $R^1$ represents a hydrogen atom or a methyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ and $R^{27}$ combine with each other to form - O-, $R^{16}$ represents a hydrogen atom or a halogen atom, $R^{17}$ represents a hydrogen atom, $R^{18}$ represents a methyl group, $R^{19}$, $R^{20}$, $R^{22}$, $R^{23}$, $R^{25}$, and $R^{26}$ combine together with carbon atoms to which they are attached to form a benzene ring, $R^{21}$ represents a methyl group, and $R^{24}$ represents a C1-C3 alkoxy group or $OR^{A19}$;

$c^2$: a combination wherein $R^1$ represents a methyl group, $R^2$ represents $OR^{A2}$, $R^3$ represents a hydrogen atom, $R^2$ and $R^3$ may combine with each other to form an oxo group, $R^3$ and $R^{18}$ may combine with each other to form $\#^3$-O-$CHR^{B1}$-, $R^4$ and $R^{27}$ combine with each other to form $\#^4$-$CH_2$-O-, $R^5$ represents a methyl group, $R^6$ represents a hydrogen atom or $OR^{A5}$, $R^7$ and $R^{11}$ combine with each other to form -O-, $R^8$ represents a hydrogen atom, a halogen atom, a methoxy group, or a hydroxy group, $R^9$ represents a methyl group or $CH_2OH$, $R^{10}$ represents a hydrogen atom or a hydroxy group, $R^{12}$ represents a hydrogen atom, $R^{13}$ represents a hydrogen atom or $OR^{A11}$,

$R^{14}$ represents a hydrogen atom, $R^{15}$ represents a hydrogen atom, $R^{16}$ represents a hydrogen atom, a halogen atom, or $OR^{A13}$, $R^{15}$ and $R^{16}$ may combine together with carbon atoms to which they are attached to form a double bond, $R^{17}$ represents a hydrogen atom, $R^{16}$ and $R^{17}$ may combine with each other to form an oxo group, $R^{18}$ represents a methyl group, $R^{15}$ and $R^{18}$ may combine with each other to form $\#^{15}$-O-$CHR^{B2}$-, $R^{19}$ represents a hydrogen atom, $R^{20}$ represents a hydrogen atom or $OR^{A15}$, $R^{21}$ represents a methyl group, $CH_2OR^{A16}$, or $CH_2NHCH_2C(O)OCH_3$, $R^{22}$ represents a hydrogen atom, $R^{18}$ and $R^{22}$ may combine with each other to form $\#^{18}$-$CH_2$-O-, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, and $R^{26}$ represents a hydrogen atom or $OR^{A20}$;

$d^2$: a combination wherein $R^1$ and $R^{27}$ combine with each other to form $\#^1$-$CH_2$-O-, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom or a hydroxy group, $R^{12}$ represents a hydrogen atom, $R^{13}$ represents a hydrogen atom, $R^{14}$ represents a hydrogen atom, $R^{15}$ represents a hydrogen atom or $OR^{A12}$, $R^{16}$ represents a hydrogen atom, a halogen atom, or $OR^{A13}$, $R^{17}$ represents a hydrogen atom, $R^{16}$ and $R^{17}$ may combine with each other to form an oxo group, $R^{18}$ represents a methyl group, $R^{19}$ represents a hydrogen atom, $R^{20}$ represents a hydrogen atom, $R^{21}$ represents a methyl group, $R^{22}$ and $R^{23}$ combine together with carbon atoms to which they are attached to form a double bond, $R^{24}$ and $R^{25}$ combine with each other to form an oxo group, and $R^{26}$ represents a hydrogen atom;

$e^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ represents a hydrogen atom, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, a dichloromethyl group, a hydroxy(methoxy)methyl group, or a hydroxy(ethoxy)methyl group, $R^{14}$ represents a hydrogen atom, $R^{15}$ represents a hydrogen atom, $R^{16}$ represents a hydrogen atom or a halogen atom, $R^{15}$ and $R^{16}$ may combine together with carbon atoms to which they are attached to form a double bond, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents a hydroxy group, $R^{25}$ represents a hydrogen atom, $R^{24}$ and $R^{25}$ may combine with each other to form an oxo group, $R^{26}$ represents a hydrogen atom, and $R^{27}$ represents a C1-C3 alkoxy group or a hydroxy group;

$f^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ and $R^{27}$ combine with each other to form a bond, $R^7$ and $R^{11}$ combine with each other to form -O-, $R^8$ represents a hydrogen atom, $R^9$ represents a methyl group, $R^{10}$ represents a hydrogen atom, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom or a halogen atom, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ represents $OR^{A19}$, $R^{25}$ represents a hydrogen atom, $R^{24}$ and $R^{25}$ may combine with each other to form an oxo group, and $R^{26}$ represents a hydrogen atom; and

$g^2$: a combination wherein $R^1$ represents a hydrogen atom or a C1-C3 alkyl group, $R^2$ and $R^3$ combine with each other to form an oxo group, $R^4$ represents a methyl group, $R^5$ represents a methyl group, $R^6$ represents a hydrogen atom, $R^7$ represents a hydrogen atom, $R^8$ represents a hydrogen atom, $R^9$ represents a methyl group, $R^{10}$ represents a hydrogen atom, $R^{11}$ represents $OR^{A9}$, $R^{12}$ represents a hydrogen atom, $R^{13}$ represents a hydrogen atom, $R^{14}$ represents a hydrogen atom, $R^{15}$ represents a hydrogen atom, $R^{16}$ represents a hydrogen atom, a halogen atom, or $OR^{A13}$, $R^{17}$ represents a hydrogen atom, $R^{18}$ represents a methyl group, $R^{19}$ represents a hydrogen atom, $R^{20}$ represents a hydrogen atom, $R^{21}$ represents a methyl group, $R^{22}$ represents a hydrogen atom, $R^{23}$ represents a hydrogen atom, $R^{22}$ and $R^{23}$ may combine together with carbon atoms to which they are attached to form a double bond, $R^{24}$ and $R^{25}$ combine with each other to form an oxo group, $R^{26}$ represents a hydrogen atom, and $R^{27}$ represents a C1-C3 alkoxy group or a hydroxy group.

**6.** A composition for controlling a harmful arthropod or a harmful nematode which comprises the compound represented by formula (I) according to any one of claims 1 to 5 or an N-oxide thereof or a salt thereof.

**7.** The composition according to claim 6 which further comprises one or more ingredients selected from the group consisting of the following Group (a), Group (b), Group (c), Group (d), and Group (e):

Group (a): a group consisting of insecticidal active ingredients, miticidal active ingredients and nematicidal active ingredients;
Group (b): fungicidal active ingredients;
Group (c): plant growth regulatory ingredients;
Group (d): repellent active ingredients;
Group (e): biological control materials.

**8.** A method for controlling a harmful arthropod or a harmful nematode which comprises applying an effective amount of the composition according to claim 7 to a harmful arthropod or a harmful nematode or a habitat for the harmful arthropod or the harmful nematode.

**9.** A seed or vegetative reproductive organ carrying an effective amount of the compound according to any one of claims 1 to 5 or an N-oxide thereof or a salt thereof or an effective amount of the composition according to claim 7.

**10.** A composition for controlling a harmful arthropod or a harmful nematode which comprises the compound represented by formula (I) according to any one of claims 1 to 5 or an N-oxide thereof or a salt thereof, and an inert carrier.

**11.** A compound represented by formula (II):

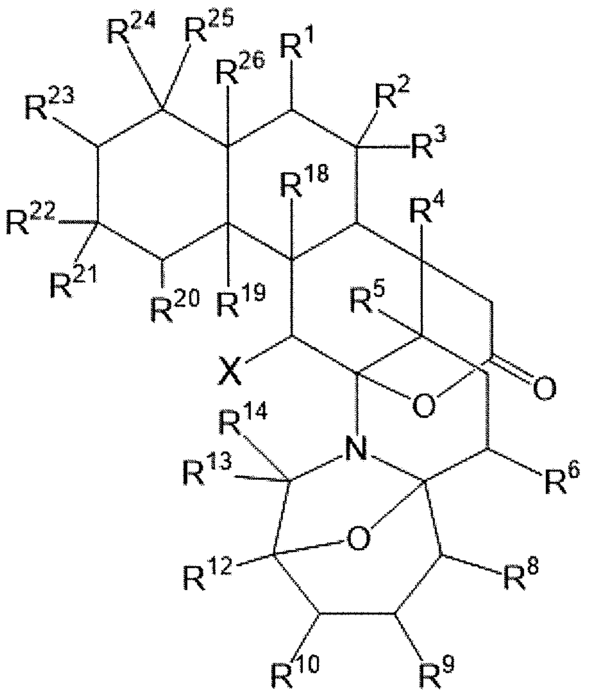

(II)

[wherein

$R^1$ represents a hydrogen atom, a C1-C3 alkyl group, or $CH_2OR^{A1}$,
$R^2$ represents $OR^{A2}$,
$R^3$ represents a hydrogen atom,
$R^4$ represents a methyl group or $CH_2OR^{A3}$,
$R^5$ represents a methyl group or $CH_2OR^{A4}$,
$R^6$ represents a hydrogen atom or $OR^{A5}$,
$R^8$ represents a hydrogen atom, a halogen atom, a methoxy group, or $OR^{A6}$,
$R^9$ represents a methyl group or $CH_2OR^{A7}$,
$R^{10}$ represents a hydrogen atom or $OR^{A8}$,
$R^{12}$ represents a hydrogen atom or $OR^{A10}$,
$R^{13}$ represents a hydrogen atom,
$R^{14}$ represents a hydrogen atom,
X represents a halogen atom,

$R^{18}$ represents a methyl group, or $CH_2OR^{A14}$,
$R^{19}$ represents a hydrogen atom,
$R^{20}$ represents a hydrogen atom or $OR^{A15}$,
$R^{21}$ represents a methyl group, $CH_2OR^{A16}$, or $CH_2NHCH_2C(O)OCH_3$,
$R^{22}$ represents a hydrogen atom or $OR^{A17}$,
$R^{23}$ represents a hydrogen atom or $OR^{A18}$,
$R^{24}$ represents a hydrogen atom, a C1-C3 alkoxy group, or $OR^{A19}$,
$R^{25}$ represents a hydrogen atom,
$R^{26}$ represents a hydrogen atom or $OR^{A20}$,
$R^{A1}$, $R^{A2}$, $R^{A3}$, $R^{A4}$, $R^{A5}$, $R^{A6}$, $R^{A7}$, $R^{A8}$, $R^{A10}$, $R^{A14}$, $R^{A15}$, $R^{A16}$, $R^{A17}$, $R^{A18}$, $R^{A19}$, and $R^{A20}$ are identical to or different from each other and each represents a hydrogen atom, a (C1-C6 alkyl)carbonyl group, or a (C3-C6 cycloalkyl)carbonyl group,
$R^2$ and $R^3$ may combine with each other to form an oxo group,
$R^3$ and $R^{18}$ may combine with each other to form $\#^3$-O-$CHR^{B1}$-, $\#^3$ represents a binding position of a carbon atom to which $R^3$ is attached,
$R^{13}$ and $R^{14}$ may combine with each other to form an oxo group,
$R^{18}$ and $R^{22}$ may combine with each other to form $\#^{18}$ -$CH_2$-O-, $\#^{18}$ represents a binding position of a carbon atom to which $R^{18}$ is attached,
$R^{18}$ and $R^{23}$ may combine with each other to form -$CH_2$-,
$R^{19}$, $R^{20}$, $R^{22}$, $R^{23}$, $R^{25}$, and $R^{26}$ may combine together with carbon atoms to which they are attached to form a benzene ring,
$R^{21}$ and $R^{22}$ may combine with each other to form =$CH_2$,
$R^{22}$ and $R^{23}$ may combine together with carbon atoms to which they are attached to form a double bond,
$R^{24}$ and $R^{25}$ may combine with each other to form an oxo group, and
$R^{B1}$ represents a hydrogen atom, or a C1-C3 alkoxy group]
, or an N-oxide thereof or a salt thereof.

**12.** The compound according to claim 11 wherein in the formula (II), wherein
$R^2$ and $R^3$ combine with each other to form an oxo group, $R^4$ represents a methyl group, $R^5$ represents a methyl group, $R^6$ represents a hydrogen atom, $R^8$ represents a hydrogen atom, $R^9$ represents a methyl group, $R^{10}$ represents a hydrogen atom, $R^{12}$ represents a hydrogen atom, $R^{18}$ represents a methyl group, $R^{20}$ represents a hydrogen atom, $R^{21}$ represents a methyl group, $R^{22}$ represents a hydrogen atom or $OR^{A17}$, $R^{21}$ and $R^{22}$ may combine with each other to form =$CH_2$, $R^{23}$ represents a hydrogen atom or $OR^{A18}$, $R^{18}$ and $R^{23}$ may combine with each other to form -$CH_2$-, $R^{22}$ and $R^{23}$ may combine together with carbon atoms to which they are attached to form a double bond, $R^{24}$ represents a hydrogen atom or $OR^{A19}$, $R^{25}$ represents a hydrogen atom, $R^{24}$ and $R^{25}$ may combine with each other to form an oxo group, and $R^{26}$ represents a hydrogen atom.

**13.** A compound represented by formula (AA1), formula (AA2), formula (AA3), formula (BB1), formula (EE1), formula (EE3), formula (EE4), formula (EE5), formula (EE6), formula (EE7), formula (GG1), formula (GG2), or formula (GG3), or an N-oxide thereof or a salt thereof, or a compound represented by formula (EE2).

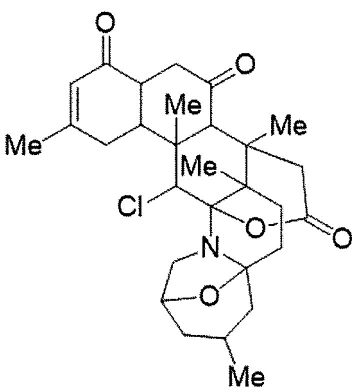
(AA1)

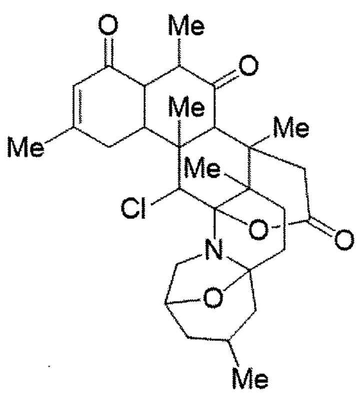
(AA2)

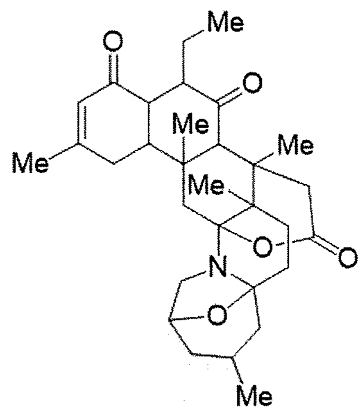
(AA3)

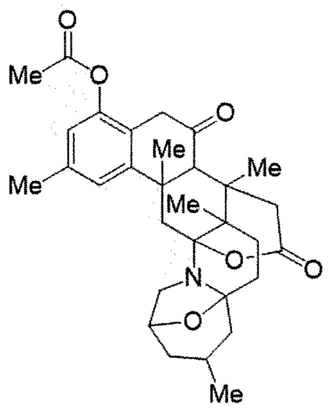

(BB1)

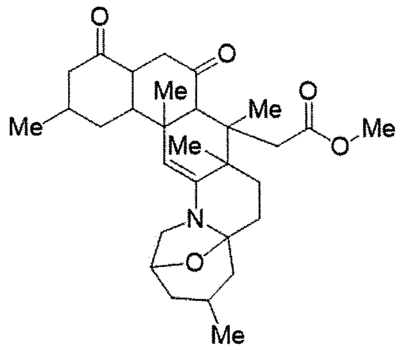

(EE1)

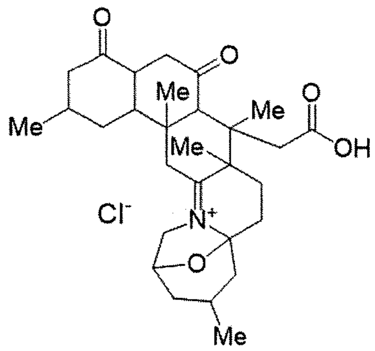

(EE2)

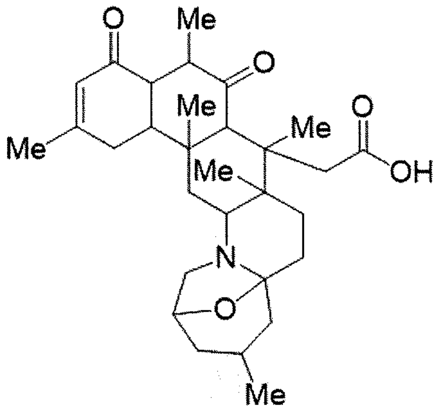

(EE3)

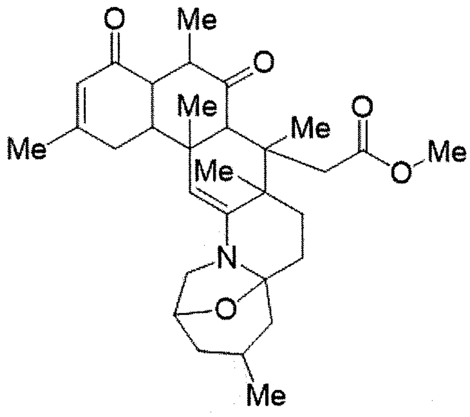

(EE4)

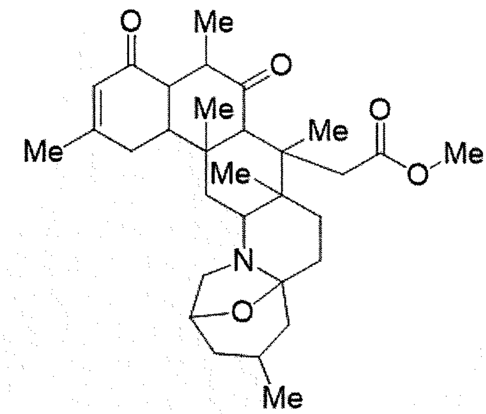

(EE5)

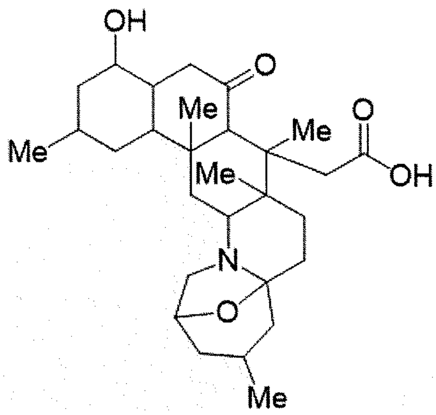

(EE6)

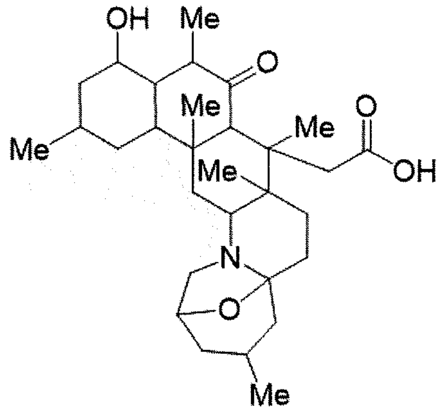

(EE7)

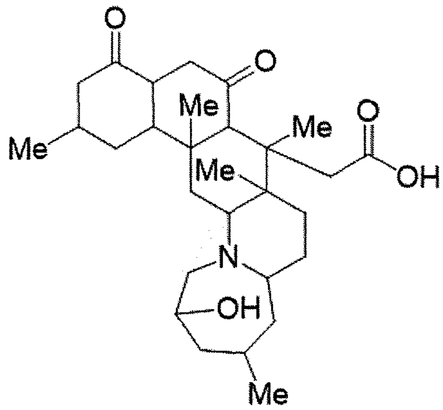

(GG1)

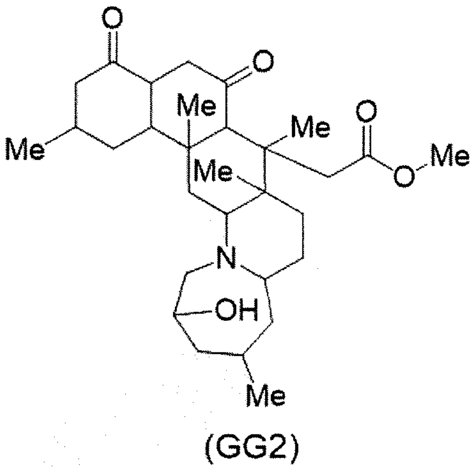

(GG2)

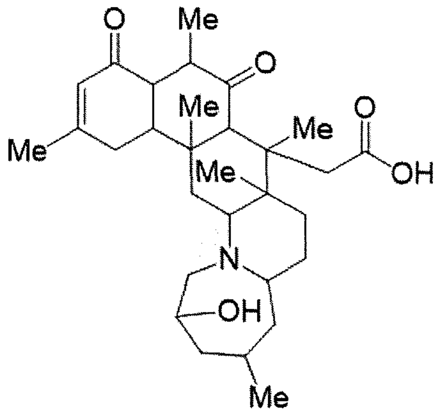

(GG3)

**14.** The compound according to claim 13 which is represented by formula (AA1), formula (AA2), formula (AA3), formula (BB1), formula (EE1), formula (EE3), formula (EE4), formula (EE5), formula (EES), formula (GG1), or formula (GG3), or an N-oxide thereof or a salt thereof, or a compound represented by formula (EE2).

**15.** A composition for controlling a harmful arthropod or a harmful nematode which comprises the compound according to any one of claims 11 to 14, or an N-oxide thereof or a salt thereof.

**16.** The composition according to claim 15 which further comprises one or more ingredients selected from the group consisting of the following Group (a), Group (b), Group (c), Group (d), and Group (e):

Group (a): a group consisting of insecticidal active ingredients, miticidal active ingredients and nematicidal active ingredients;
Group (b): fungicidal active ingredients;
Group (c): plant growth regulatory ingredients;
Group (d): repellent ingredients;
Group (e): biological control materials.

**17.** A method for controlling a harmful arthropod or a harmful nematode which comprises applying an effective amount of the composition according to claim 16 to a harmful arthropod or a harmful nematode or a habitat for the harmful arthropod or the harmful nematode.

**18.** A seed or vegetative reproductive organ carrying an effective amount of the compound according to any one of claims 11 to 14 or an N-oxide thereof or a salt thereof or an effective amount of the composition according to claim 16.

**19.** A composition for controlling a harmful arthropod or a harmful nematode which comprises the compound according to any one of claims 11 to 14, or the N-oxide thereof or the salt thereof, and an inert carrier.

## INTERNATIONAL SEARCH REPORT

International application No.
**PCT/JP2023/007767**

**A. CLASSIFICATION OF SUBJECT MATTER**

***A01N 43/90***(2006.01)i; ***A01P 5/00***(2006.01)i; ***A01P 7/04***(2006.01)i; ***C07D 471/04***(2006.01)i; ***C07D 498/18***(2006.01)i; ***C07D 498/22***(2006.01)i
FI: A01N43/90 102; A01P7/04; A01P5/00; C07D498/18; C07D471/04 121; C07D498/22 CSP

According to International Patent Classification (IPC) or to both national classification and IPC

**B. FIELDS SEARCHED**

Minimum documentation searched (classification system followed by classification symbols)

A01N43/90; A01P5/00; A01P7/04; C07D471/04; C07D498/18; C07D498/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 10-53589 A (SAGAMI CHEM RES CENTER) 24 February 1998 (1998-02-24) | 1-19 |
| A | JP 10-101678 A (SAGAMI CHEM RES CENTER) 21 April 1998 (1998-04-21) | 1-19 |
| A | JP 6-199867 A (SAGAMI CHEM RES CENTER) 19 July 1994 (1994-07-19) | 1-19 |
| A | KURAMOTO, M. et al. Structure-activity relationship of norzoanthamine exhibiting significant inhibition of osteoporosis. BULLETIN OF THE CHEMICAL SOCIETY OF JAPAN. 1998, vol. 71, no. 4, pp. 771-779<br>in particular, fig. 1, 13, 16, 17 | 1-19 |
| P, A | WO 2022/186146 A1 (SUMITOMO CHEMICAL CO) 09 September 2022 (2022-09-09) | 1-19 |
| P, A | JP 2022-153590 A (SUMITOMO CHEMICAL CO) 12 October 2022 (2022-10-12) | 1-19 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 March 2023** | **18 April 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/007767**

| Patent document cited in search report | Publication date (day/month/year) | Patent family member(s) | Publication date (day/month/year) |
|---|---|---|---|
| JP 10-53589 A | 24 February 1998 | WO 1997/035581 A1 | |
| JP 10-101678 A | 21 April 1998 | (Family: none) | |
| JP 6-199867 A | 19 July 1994 | (Family: none) | |
| WO 2022/186146 A1 | 09 September 2022 | (Family: none) | |
| JP 2022-153590 A | 12 October 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022032312 A
- JP 2022133984 A
- JP H10101678 A
- US 201600360974 A1
- JP H1053589 A
- US 20160036094 A1


### Non-patent literature cited in the description


- The Pesticide Manual. British Crop Protection Council, 2018
- *Bulletin of the Chemical Society of Japan*, 1998, vol. 71, 771-779
- *Heterocyclic Communications*, 1995, vol. 1 (2-3), 207-214
- *Heterocyclic Communications*, 1995, vol. 1 (2-3), 207
- *Journal of Organic Chemistry*, 1985, vol. 50 (20), 3757
- *Heterocycles*, 1989, vol. 28 (1), 103
- *Tetrahedron*, 1998, vol. 54 (27), 7891
- *Tetrahedron*, 1999, vol. 55 (17), 5539
- *Marine Drugs*, 2014, vol. 12 (10), 5188
- *Tetrahedron Letters*, 2014, vol. 55 (39), 5369
- *Tetrahedron*, 2015, vol. 71 (45), 8601
- *Journal of Natural Products*, 2019, vol. 82 (10), 2790
- *Journal of Natural Products*, 2016, vol. 79 (10), 2674
- *Marine Drugs*, 2018, vol. 16 (7), 242
- *Tetrahedron Letters*, 2008, vol. 49 (14), 2189
- *J. Org. Chem.*, 2020, vol. 85, 12553
- *Bioorganic Chemistry*, 2021, vol. 109, 104700
- *Bulletin of the Chemical Society of Japan*, 1998, vol. 71 (4), 771
- *Heterocyclic Communications*, 1998, vol. 4 (6), 575
- Angewandte Chemie. 2009, vol. 48, 8905
- *Chemistry - An Asian Journal*, 2011, vol. 6 (3), 922
- *Tetrahedron Letters*, 1997, vol. 38 (32), 5683
- *62nd Symposium on the Chemistry of Natural Products*, 2020, 2-8
- *Pure and Applied Chemistry*, 2007, vol. 79, 651
- Manual on development and use of FAO and WHO Specifications for pesticides. *FAO Plant Production and Protection Papers-271-276, prepared by the FAO/WHO Joint Meeting on Pesticide Specifications*, 2016
- Angewandte Chemie. 2009, vol. 48, 8905-8
- *Chemistry - An Asian Journal*, 2011, vol. 6 (3), 922-931
- *Bulletin of the Chemical Society of Japan*, 1998, vol. 71 (4), 771-779
- *Heterocyclic Communications*, 1998, vol. 4 (6), 575-580
- *Tetrahedron Letters*, 1997, vol. 38 (32), 5683-5686
- **ZECK, W.M.** *Pflanzenschutz-Nachrichten. Bayer AG*, 1971, vol. 24, 141-144